# FASCICULE DE BREVET EUROPEEN

(11) **EP 2 271 653 B1**
(45) Date de publication et mention de la délivrance du brevet: **07.12.2011**
(21) Numéro de dépôt: 09745933.3
(22) Date de dépôt: 15.04.2009
(51) Int. Cl.: C07H 1/00, C07H 21/00

(54) **NOUVEAU PROCEDE DE PREPARATION D'OLIGONUCLEOTIDES COMPORTANT UNE EXTREMITE 5'-PHOSPHATE MONOESTER OU 5'-THIOPHOSPHATE MONOESTER**
NEUARTIGES VERFAHREN ZUR HERSTELLUNG VON OLIGONUKLEOTIDEN MIT EINEM 5'-PHOSPHAT-MONOESTER- ODER EINEM 5'-THIOPHOSPHAT-MONOESTER-ENDE
NOVEL METHOD FOR PREPARING OLIGONUCLEOTIDES COMPRISING A 5' -PHOSPHATE MONOESTER OR 5' -THIOPHOSPHATE MONOESTER END

(30) Priorité: 16.04.2008 FR 0802097
(43) Date de publication de la demande: 12.01.2011
(73) Titulaire: Université Joseph Fourier, 38041 Grenoble Cedex 09 (FR)
(72) Inventeur: DEFRANCQ, Eric, F-38830 Saint Pierre D'allevard (FR); LARTIA, Remi M., F-38610 Gieres (FR)
(74) Mandataire: Grosset-Fournier, Chantal Catherine
(86) Numéro de dépôt international: PCT/FR2009/050689
(87) Numéro de publication internationale: WO 2009/138614

(56) Documents cités:
- COOKE ET AL: "Solid-phase synthesis of terminal oligonucleotide-phosphoramidate conjugates" TETRAHEDRON LETTERS, ELSEVIER, AMSTERDAM, vol. 47, no. 5, 30 janvier 2006 (2006-01-30), pages 719-722, XP005222417 ISSN: 0040-4039 cité dans la demande

## Description

La présente invention concerne la préparation d'oligonucléotides comportant une extrémité 5'-phosphate monoester ou 5'-thiophosphate monoester.

Les oligonucléotides comportant une extrémité 5'-phosphate sont des composés utilisés entre autres, en biologie moléculaire dans le cadre de la ligation enzymatique ou de l'étude des mécanismes de réparation des lésions des acides nucléiques, et en thérapeutique comme adjuvants pour les préparation vaccinales à l'aide d'oligonucléotides CpG ou pour l'inhibition de l'expression génétique par les siARN.

Les oligonucléotides 5'-phosphate sont en général obtenus par synthèse chimique par introduction d'un groupement phosphate en position 5' au moyen de réactifs de phosphorylation appartenant à la famille des phosphoramidites décrits dans les références ci-après (Solid phase 5'-phosphorylation of oligonucleotides, Connolly B.A., Tetrahedron Lett., 1987, 28, 463-466 ; Chemical 5'-phosphorylation of oligonucleotides valuable in automated DNA synthesis, Uhlmann E., Engel J., Tetrahedron Lett., 1986, 27, 1023-1026 ; A chemical 5'-phosphorylation of oligonucleotides that can be monitored by trityl cation release, Horn T., Urdea M.S., Tetrahedron Lett., 1986, 27, 4705-4708 ; Synthesis and utility of a DNA phosphorylating agent based on 2-(triphenylsilyl)ethanol, Celebuski J. E., Chan C., J. Org. Chem., 1992, 57, 5535-5538 ; A new approach for chemical phosphorylation of oligonucleotides at the 5'-terminus, Guzaev A., Salo H., Azhayev A, Lönnberg H., Tetrahedron, 1995, 51, 9375-9384 ; A novel reagent for the chemical phosphorylation of oligonucleotides, Leuck M., Vagle K. E., Roach J. S., Wolter A., Tetrahedron Lett., 2004, 45, 321-324 ; New reagent for the preparation of oligonucleotides involving a 5'-thiophosphate or a 5' phosphate group; , Lartia R., Asseline U., Tetrahedron Lett., 2004, 45, 5949-5952 ; An efficient reagent for the phosphorylation of deoxyribonucleotides, DNA oligonucleotides, and their thermolytic analogues, Ausin C., Grajkowski A., Cieslak J., Beaucage S. L., Org Lett., 2005, 7, 4201-4204 ; A versatile reagent for the synthesis of 5'-phosphorylated, 5'-thiophosphorylated or 5'-phosphoramidate conjugated oligonucleotides, Meyer A., Bouillon C., Vidal S., Vasseur J.-J., Morvan F., Tetrahedron Lett., 2006, 47, 8867-8871 ; Solid-phase synthesis of terminal oligonucleotide-phosphoramidate conjugates ; Cooke L. A., Frauendorf C., Gilea M. A., Holmes S. C., Vyle J. S., Tetrahedron Lett., 2006, 47, 719-722.

Cependant, tous ces différents réactifs de type phosphoramidite présentent les inconvénients inhérents à cette famille chimique, à savoir :
- une fabrication longue et fastidieuse,
- un prix élevé lorsque le réactif est disponible commercialement,
- une sensibilité élevée vis-à-vis de l'eau et des oxydants tels que l'oxygène atmosphérique, entraînant la nécessité de manipuler le réactif sous atmosphère inerte,
- une durée de conservation du réactif courte, tout particulièrement lorsque le conditionnement a été ouvert,
- une présentation sous forme d'huile, ce qui rend la manipulation délicate notamment pour la pesée de petites quantités.

Un des aspects de l'invention concerne un procédé permettant d'obtenir des oligonucléotides comportant une extrémité 5'-phosphate monoester ou 5'-thiophosphate monoester, sans avoir recours à des réactifs de type phosphoramidite et des inconvénients associés à ceux-ci.

Un autre aspect de l'invention est de fournir un procédé simple de synthèse d'oligonucléotides comportant une extrémité 5'-phosphate monoester ou 5'-thiophosphate monoester.

Encore un autre aspect de l'invention permet la fourniture d'un procédé de synthèse d'oligonucléotides comportant une molécule d'intérêt telle que des sondes fluorescentes, des peptides, des sucres, des agents intercalants...en position 5'.

La présente invention concerne l'utilisation d'au moins un oxydant notamment choisi parmi :
- les dérivés de l'iode hypervalent, notamment l'acide 2-iodoxybenzoique (IBX) et le Dess-Martin periodinane (DMP), et,
- le réactif de Swern,
pour la synthèse d'oligonucléotides, modifiés ou non, notamment d'oligoribonucléotides, d'oligodésoxyribonucléotides, les dits oligonucléotides comportant une extrémité 5'-phosphate monoester ou 5'-thiophosphate monoester.

Par l'expression « dérivés de l'iode hypervalent », il faut comprendre des composés iodés dans lesquels l'atome d'iode contient plus de huit électrons dans la couche de valence exigée par la règle de l'octet. Les dérivés de l'iode hypervalent peuvent être des dérivés de l'iode (III) hypervalent dénommés iodanes, ou des dérivés de l'iode (V) hypervalent dénommés periodinanes, et permettent l'oxydation d'alcools primaires en aldéhydes et d'alcools secondaires en cétones.

II existe de nombreux dérivés de l'iode (III) hypervalent tels que, sans être limités à ceux-ci le PIFA (Bis (trifluoroacétoxy) iodobenzène, le PIDA (diacétoxy) iodobenzène,
le réactif de Koser : 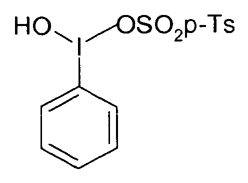 ,
les dérivés d'IBA (iodosyl benzoic acid) : 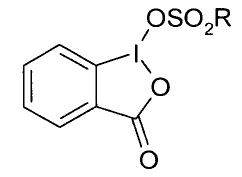 avec R= p-Ts ou Ms.

Des composés préférés de dérivés de l'iode (V) hypervalent, sans être limités à ceux-ci, sont l'IBX : 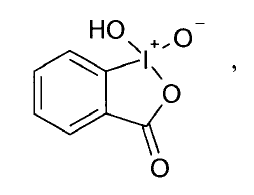 et le réactif de Dess Martin (DMP) :

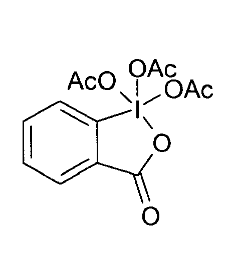

D'autres oxydants permettant d'oxyder un alcool primaire en aldéhyde peuvent être utilisés, notamment, mais sans être limité à celui-ci, le réactif de Swern (Mancuso, A. J.; Huang, S. L.; Swern, D. J. Org. Chem., 1978, 43, 2480).

Les oligonucléotides sont de courtes séquences de nucléotides (quelques dizaines de bases) qui peuvent être soit de l'ADN (oligodésoxyribonucléotides) ou de l'ARN (oligoribonucléotides).

Un nucléotide est formé par l'une des bases puriques ou bases pyrimidiques, liée à un résidu sucre (le 2-désoxyribose pour l'ADN et le ribose pour l'ARN) par une liaison *N*-glycosidique.

L'enchaînement des nucléosides est assuré par des liens phosphodiester qui unissent le carbone C-5. d'une unité osidique au carbone C-3. de la suivante.

Par l'expression « oligonucléotides modifiés », il faut donc comprendre des oligonucléotides dans lesquels les groupes phosphates, la base et/ou le sucre ont été modifiés, c'est-à-dire n'ayant pas la même structure que lorsqu'ils sont d'origine naturelle, par exemple un sucre possédant une anomérie α au lieu de β, ou une base ou un sucre sur lesquels une substitution chimique a été effectuée, sans toutefois être limité par ces exemples.

Dans un mode de réalisation préféré, l'invention concerne l'utilisation d'un oligonucléotide modifié ou non, avec un oxydant tel que défini ci-dessus, et :
- comportant une extrémité 5'-OH, et,
- contenant (n+1) nucléotides,
   pour obtenir respectivement un oligonucléotide modifié ou non,
- comportant une extrémité 5'-phosphate monoester ou 5'-thiophosphate monoester, et,
- contenant n nucléotides.

La réaction est schématisée ci-dessous :

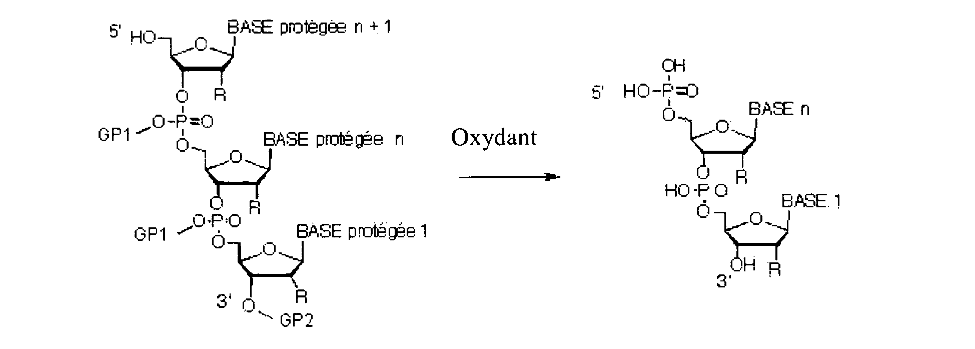

GP1 représente un groupe protecteur du phosphate et GP2 un groupe protecteur de la fonction alcool.

La réaction consiste donc à oxyder l'alcool en 5', du nucléotide n + 1, en aldéhyde, qui subit ensuite une β-élimination qui conduit à l'élimination de la base nucléique n+1 pour obtenir l'oligonucléotide comportant n nucléotides.

Selon un autre mode de réalisation, l'utilisation d'un oligonucléotide modifié ou non comportant une extrémité 5'-OH, avec un oxydant, tels que définis ci-dessus est effectuée dans le cadre d'une synthèse sur support.

L'expression « synthèse sur support » signifie que la synthèse est effectué en phase solide sur support constitué d'un polymère synthétique, notamment, mais sans être limité à ceux-ci, un « controlled pore glass (CPG) », c'est-à-dire une matrice de verre à pores de taille contrôlée, ou des billes de polystyrène.

Le support est situé au niveau de l'alcool en 3' du nucléotide 1 (GP 2 ci-dessus).

L'oxydation a lieu à l'issue de la synthèse sur support de l'oligonucléotide (ODN) protégé. L'ODN obtenu est soumis à un premier traitement basique doux, à l'aide par exemple, mais sans être limité à celles-ci, d'une base telle que le carbonate de potassium ou la méthylamine, la triéthylamine, la diisopropyl amine et les amines tertiaires en général pour promouvoir la β-élimination, suivi d'un traitement à l'ammoniaque concentrée.

Selon un mode de réalisation préféré, l'invention comprend l'utilisation d'un oligonucléotide modifié ou non comportant une extrémité 5'-OH, avec un oxydant, tels que définis ci-dessus, pour effectuer la synthèse d'un composé de formule (I) suivante :
dans laquelle : 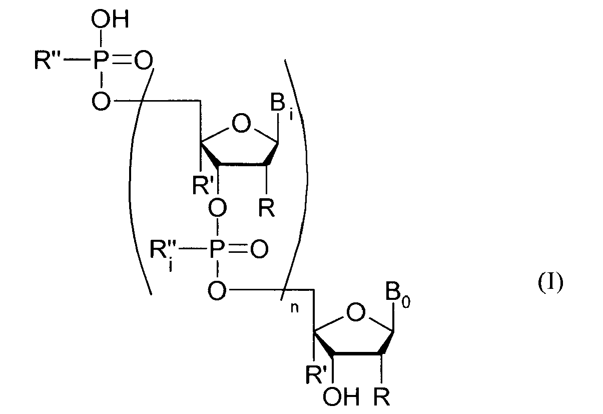
* n est un nombre entier compris de 1 à 100, notamment de 1 à 50, et en particulier de 5 à 40,
   et i est un nombre entier de 1 à n,
* B₀ et Bᵢ représentent indépendamment les uns des autres une base azotée, notamment :
   - un dérivé de purine, notamment l'adénine, la guanine, la 8-hydroxy guanine, la 8-hydroxy-adénine, 8-amino-guanine, 8-méthoxy-guanine, 8-bromo-guanine, 8-bromo-adénine, l'inosine, ou,
   - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine, mais sans être limité à celles-ci.
* R représente :
   - l'hydrogène, ou,
   - un groupement hydroxyle, ou
   - un groupement -O-alkyle comportant 1 à 4 atomes de carbones linéaire ou ramifié, ou
   - un groupement -O-(CH₂)ₘ-NH₂, dans lequel m représente un nombre entier compris de 2 à 10, ou
   - un atome d'oxygène,
* R' représente :
   - l'hydrogène, ou,
   - un groupe -(CH₂)ₚ- dans lequel p varie de 1 à 4,
   tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther, et,
* R" et Rᵢ" représentent indépendamment un groupement hydroxyle (OH) ou mercapto (SH).

Les oligonucléotides ainsi synthétisés sont des oligonucléotides modifiés ou non modifiés, d'ADN ou d'ARN, et comportant une extrémité 5'-phosphate monoester ou 5'-thiophosphate monoester.

Selon un mode de réalisation encore plus préféré, l'invention comprend l'utilisation d'un oligonucléotide modifié ou non comportant une extrémité 5'-OH, avec un oxydant, tels que définis ci-dessus, pour effectuer la synthèse d'un composé de formule (II) suivante : 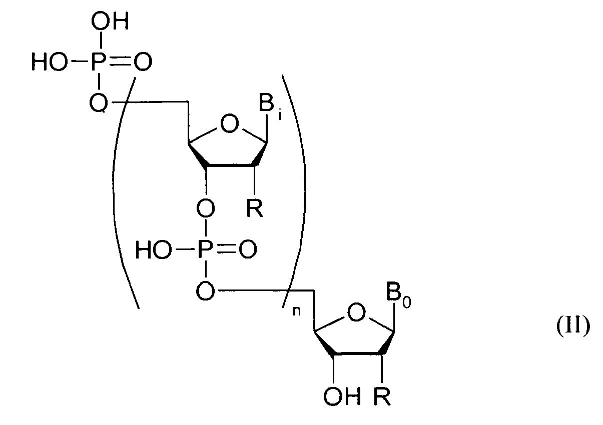 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la thymine, la cytosine, ou l'uracile,
* R représente l'hydrogène ou un groupement hydroxyle.

Les oligonucléotides ainsi synthétisés sont des oligonucléotides non modifiés d'ADN ou d'ARN et comportant une extrémité 5'-phosphate monoester.

Selon un mode de réalisation encore plus préféré, l'invention comprend l'utilisation d'un oligonucléotide modifié ou non comportant une extrémité 5'-OH, avec un oxydant, tels que définis ci-dessus, pour effectuer la synthèse d'un composé de formule (III) suivante : 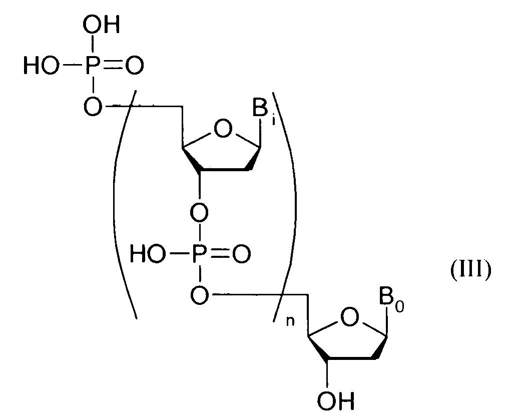 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la thymine, ou la cytosine.

Les oligonucléotides ainsi synthétisés sont des oligonucléotides non modifiés d'ADN et comportant une extrémité 5'-phosphate monoester.

Dans un mode de réalisation préféré, l'invention comprend l'utilisation d'un oligonucléotide modifié ou non comportant une extrémité 5'-OH, avec un oxydant, tels que définis ci-dessus, pour effectuer la synthèse d'un composé de formule (IV) suivante : 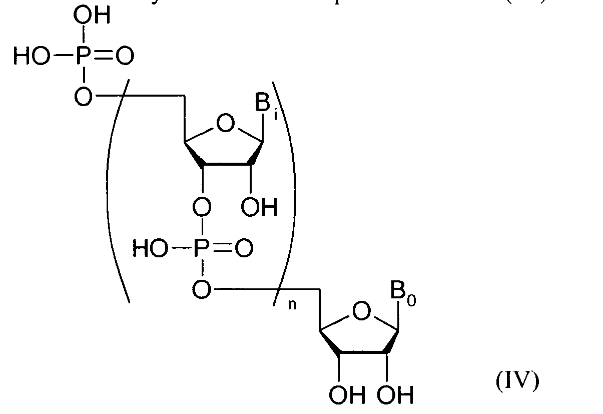 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la cytosine, ou l'uracile.

Les oligonucléotides ainsi synthétisés sont des oligonucléotides non modifiés d'ARN, et comportant une extrémité 5'-phosphate monoester.

Dans un mode de réalisation encore plus préféré, l'invention comprend l'utilisation d'un oligonucléotide non modifié comportant une extrémité 5'-SH, avec un oxydant, tels que définis ci-dessus, pour effectuer la synthèse d'un composé de formule (V') suivante : 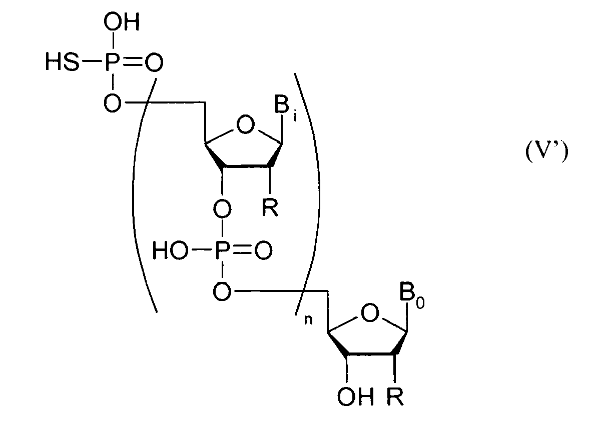 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la thymine, la cytosine, ou l'uracile,
* R représente l'hydrogène ou un groupement hydroxyle.

Les oligonucléotides ainsi synthétisés sont des oligonucléotides non modifiés d'ADN ou d'ARN et comportant une extrémité 5'-thiophosphate monoester.

Dans un mode de réalisation encore plus préféré, l'invention comprend l'utilisation d'un oligonucléotide modifié ou non comportant une extrémité 5'-OH, avec un oxydant, tels que définis ci-dessus, pour effectuer la synthèse d'un composé de formule (V) suivante : 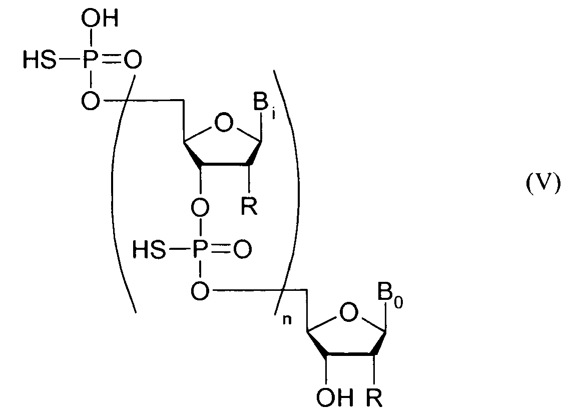 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres une base azotée, notamment :
   - un dérivé de purine, notamment l'adénine, la guanine, la 8-hydroxy guanine, l'inosine, ou,
   - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente l'hydrogène ou un groupement hydroxyle.

Les oligonucléotides ainsi synthétisés sont des oligonucléotides modifiés (de type phosphorothioate), d'ADN ou d'ARN, et comportant une extrémité 5'-thiophosphate monoester.

Selon un autre mode de réalisation, l'invention comprend l'utilisation d'un oligonucléotide modifié ou non comportant une extrémité 5'-OH, avec un oxydant, tels que définis ci-dessus, pour effectuer la synthèse d'un composé de formule (VI) suivante : 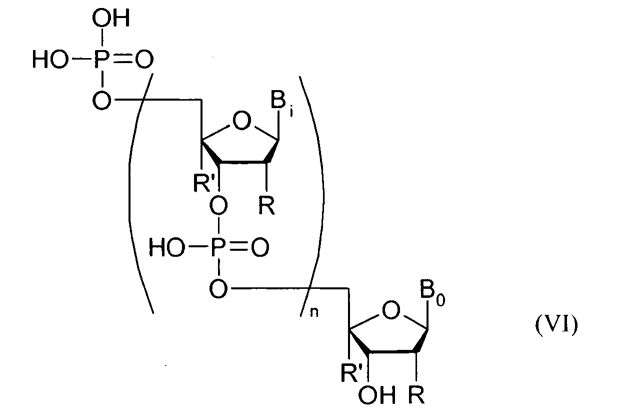 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres une base azotée, notamment :
   - un dérivé de purine, notamment l'adénine, la guanine, la 8-hydroxy guanine, l'inosine, ou,
   - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente :
   - un groupement -O-alkyle comportant 1 à 4 atomes de carbones linéaire ou ramifié, ou,
   - un groupement -O-(CH₂)ₘ-NH₂, dans lequel m représente un nombre entier compris de 2 à 10, ou,
   - ou un atome d'oxygène,
* R' représente :
   - l'hydrogène, ou,
   - un groupe -(CH₂)ₚ- dans lequel p varie de 1 à 4,
      tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther.

Les oligonucléotides ainsi synthétisés sont des oligonucléotides modifiés d'ARN, et comportant une extrémité 5'-phosphate monoester.

Selon un mode de réalisation encore plus préféré, l'oxydant utilisé pour l'oxydation d'un oligonucléotide modifié ou non modifié comportant une extrémité 5'-OH tels que définis ci-dessus, est l'IBX.

L'IBX peut être préparé mais il est également possible d'utiliser un mélange commercial stabilisé contenant 40% d'IBX et de l'acide benzoique.

Dans un autre aspect, l'invention concerne un procédé de préparation d'un composé de formule (I) suivante : 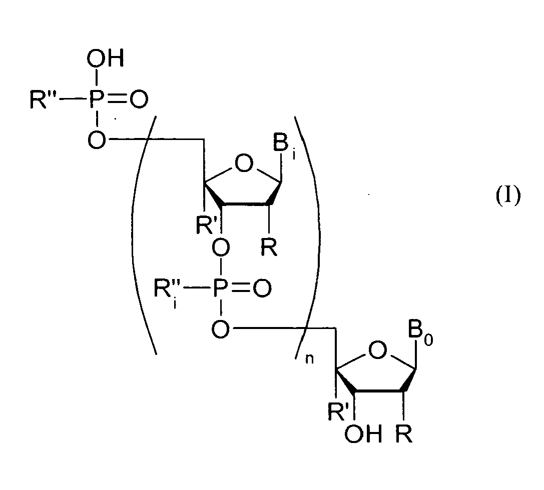 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres une base azotée, notamment :
   - un dérivé de purine, notamment l'adénine, la guanine, la 8-hydroxy guanine, l'inosine, ou,
   - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente :
   - l'hydrogène, ou,
   - un groupement hydroxyle, ou
   - un groupement -O-alkyle comportant 1 à 4 atomes de carbones linéaire ou ramifié, ou
   - un groupement -O-(CH₂)ₘ-NH₂, dans lequel m représente un nombre entier compris de 2 à 10, ou
   - un atome d'oxygène,
* R' représente :
   - l'hydrogène, ou,
   - un groupe -(CH₂)ₚ- dans lequel p varie de 1 à 4,
      tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther, et,
* R" et Rᵢ" représentent indépendamment un groupement hydroxyle ou thiol, comprenant l'étape de :
   réaction d'un oxydant choisi parmi les dérivés de l'iode hypervalent, notamment l'acide 2-iodoxybenzoique (IBX) et le Dess-Martin periodinane (DMP), et le réactif de Swern,
   sur un composé de formule (XI) suivante : 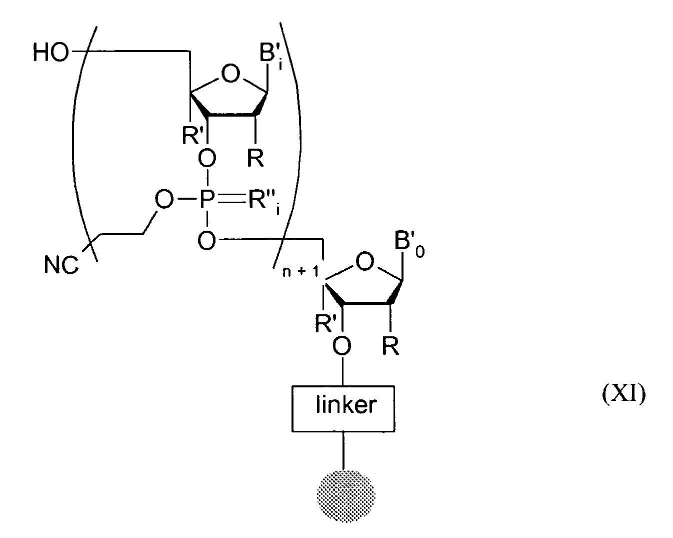 dans laquelle :
      * n est un nombre entier compris de 1 à 100, notamment de 1 à 50, et en particulier de 5 à 40,
      * i est un nombre entier compris de 1 à n+1,
      * B'₀ et B'ᵢ représentent indépendamment les uns des autres une base azotée, notamment :
         - un dérivé de purine, notamment l'adénine, la guanine, la 8-hydroxy guanine, l'inosine, ou,
         - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
            la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
            * R représente :
         - l'hydrogène, ou,
         - un groupement hydroxyle protégé par un groupe protecteur, notamment choisi parmi les groupes acétyle (Ac), silyle tels que tertbutyldiméthylsilyl (TBDMS), triisopropylsilyloxyméthyl (TOM)
         - un groupement -O-alkyle comportant 1 à 4 atomes de carbones linéaire ou ramifié, ou
         - un groupement -O-(CH₂)ₘ-NH₂, dans lequel m représente un nombre entier compris de 2 à 10, et dans lequel la fonction amine est protégée par un groupe protecteur, notamment choisi parmi les carbamates tels que le groupe fluorénylméthyloxycarbonyle (Fmoc), les amides tel que le trifluoroacétamide (CO-CF3), et le phtalimide,
            ou
         - un atome d'oxygène,
            * R' représente :
         - l'hydrogène ou,
         - un groupe -(CH₂)ₚ- dans lequel p varie de 1 à 4,
            sauf dans le cas de la dernière base qui subit la β-élimination, où R' = H uniquement.
            tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther, et, * Rᵢ" représentent indépendamment un atome d'oxygène ou de soufre,
            * linker représente un linker notamment choisi parmi :
         - un groupe -CO-Z-CO-, dans laquelle Z représente un groupe alkyle de 1 à 2 carbones ou un groupe -CH₂-O-Ph-O-CH₂-,
         - un groupe -CO-CO-
         - un groupe de formule (A) ou (B) suivante : 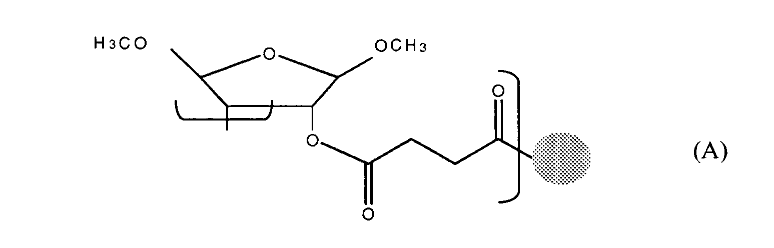 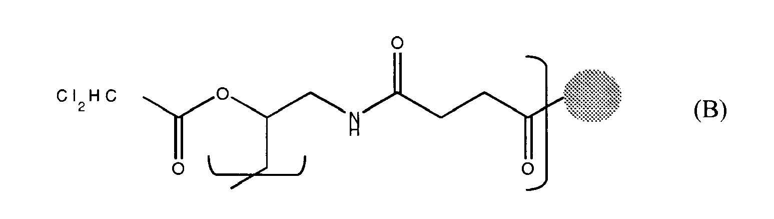
* 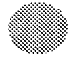 représente un support solide, notamment choisi parmi CPG (Controlled Pore Glass avec longue chaîne aminoalkyle (Icca) de différentes porosités) et les résines dérivées du polystyrène,
   suivie d'une réaction en milieu basique, notamment avec de l'hydroxyde d'ammonium, de l'hydroxyde de sodium, de la méthylamine, du carbonate de potassium, ou un mélange de ces bases,
   pour obtenir le dit composé de formule (I).

Les groupes protecteurs des bases puriques ou pyrimidiques sont bien connus de l'homme du métier et les groupes cités ci-dessus le sont à titre d'exemple sans limitation aucune.

Le groupe protecteur (cyano-éthyle) du lien phophodiester indiqué ici ne l'est qu'à titre d'exemple. D'autres groupes protecteurs de ce lien bien connus de l'homme du métier peuvent également être utilisés.

Le terme « linker » signifie que l'oligonucléotide n'est pas relié directement au support solide mais est séparé de celui-ci par un bras espaceur tel que défini ci-dessus.

Le linker est lié d'une part au support par sa partie NH, et d'autre part au sucre du nucléotide.

Les CPG sont des matrices de verres possédant de nombreux pores de taille homogène.

L'expression « de différentes porosités » signifie que les matrices de verre CPG peuvent être obtenues avec différentes tailles de pores et par conséquent une porosité différente. La taille des pores conditionne la taille des oligonucléotides qui peuvent être synthétisés.

La réaction d'oxydation est effectuée dans un solvant tel que le DMSO à une température comprise de 20°C à 60°C, préférentiellement 40°C, pendant un temps compris de 15 min à 4 h, préférentiellement de 15 min à 2h, en particulier 30 min.

Le traitement en milieu basique permet d'effectuer la déprotection de l'oligonucléotide de son support et de cliver les groupes protecteurs encore présents.

Il est préférentiellement effectué dans une solution d'ammoniaque à 28%, à une température comprise de 40°C à 60°C, préférentiellement 55°C, pendant un temps compris de 14h à 20h, en particulier 16 heures.

Dans un mode de réalisation préféré, le procédé de préparation défini ci-dessus permet la préparation d'un composé de formule (II) suivante : 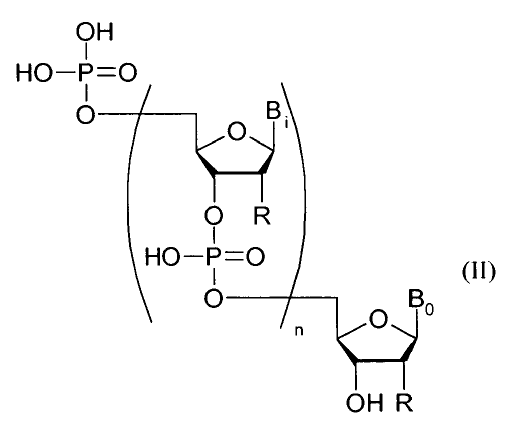 dans laquelle :
* n est un nombre entier compris de 1 à 100, notamment de 1 à 50, et en particulier de 5 à 40
* i est un nombre entier compris de 1 à n,
* B₀ et Bᵢ représentent indépendamment les uns des autres l'adénine, la guanine, la thymine, la cytosine, ou l'uracile,
* R représente l'hydrogène ou un groupement hydroxyle
   comprenant l'étape de :
   réaction d'un oxydant choisi parmi les dérivés de l'iode hypervalent, notamment l'acide 2-iodoxybenzoique (IBX) et le Dess-Martin periodinane (DMP), et le réactif de Swern,
   sur un composé de formule (XII) suivante : 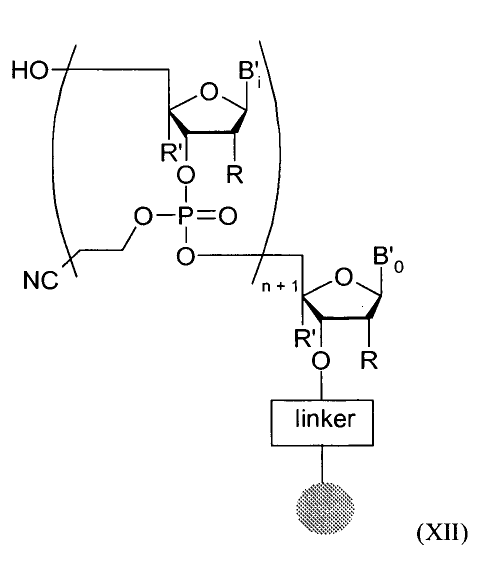
   dans laquelle :
* n est un nombre entier compris de 1 à 100, notamment de 1 à 50, et en particulier de 5 à 40,
* i est un nombre entier compris de 1 à n+1,
* B'₀ et B'ᵢ représentent indépendamment les uns des autres une base azotée choisie parmi l'adénine, la guanine, la thymine, la cytosine, et l'uracile,
   la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* R représente l'hydrogène ou un groupement hydroxyle protégé par un groupe protecteur, notamment choisi parmi les groupes acétyle (Ac), silyle tels que tertbutyldiméthylsilyl (TBDMS) et triisopropylsilyloxyméthyl (TOM).
* R' représente l'hydrogène
* linker représente un linker notamment choisi parmi :
   - un groupe -CO-Z-CO-, dans laquelle Z représente un groupe alkyle de 1 à 2 carbones ou un groupe -CH₂-O-Ph-O-CH₂-,
   - un groupe -CO-CO-,
   - un groupe de formule (A) ou (B) suivante : 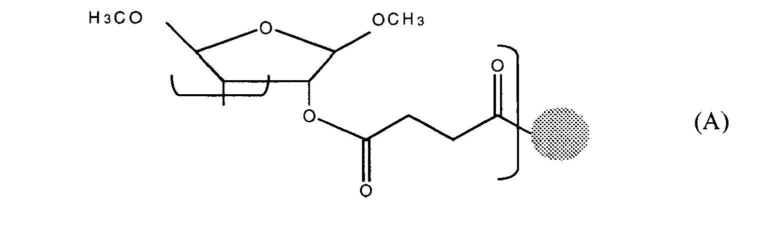 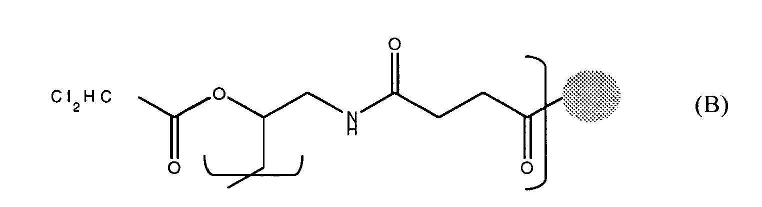
* représente un support solide notamment choisi parmi CPG (Controlled Pore Glass) avec longue chaîne aminoalkyle (Icca) de différentes porosités et les résines dérivées du polystyrène,
   suivie d'une réaction en milieu basique, notamment avec de l'hydroxyde d'ammonium, de l'hydroxyde de sodium, de la méthylamine, du carbonate de potassium, ou un mélange de ces bases,
   pour obtenir le dit composé de formule (II).

Dans ce mode de réalisation, des oligonucléotides non modifiés d'ADN ou d'ARN, et comportant une extrémité 5'-phosphate monoester sont préparés.

Le linker est lié d'une part au support par sa partie NH, et d'autre part au sucre du nucléotide.

Dans un mode de réalisation encore plus préféré, le procédé de préparation défini ci-dessus permet la préparation d'un composé de formule (III) suivante : 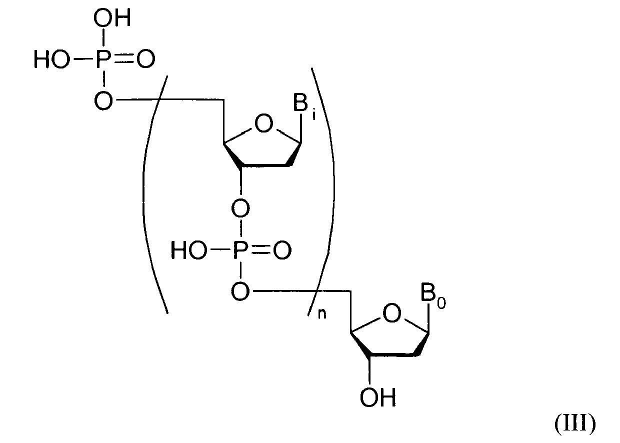 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la thymine, ou la cytosine,
   comprenant l'étape de :
   réaction d'un oxydant choisi parmi les dérivés de l'iode hypervalent, notamment l'acide 2-iodoxybenzoique (IBX) et le Dess-Martin periodinane (DMP), et le réactif de Swern,
   sur un composé de formule (XIII) suivante : 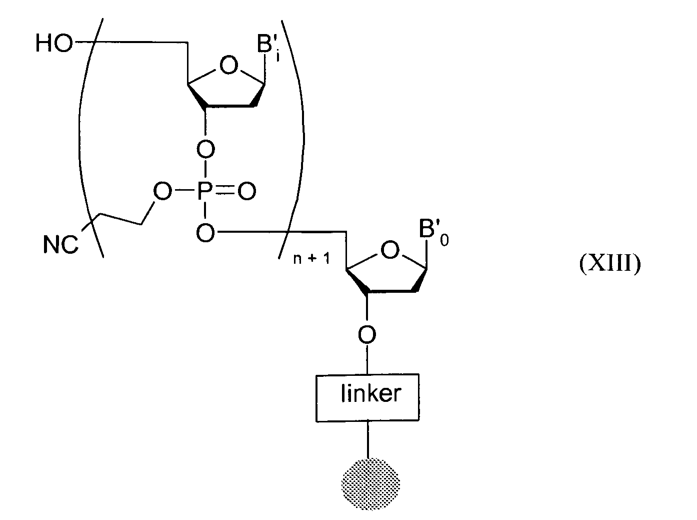
   dans laquelle :
* n est un nombre entier compris de 1 à 100, notamment de 1 à 50, et en particulier de 5 à 40,
* i est un nombre entier compris de 1 à n+1,
* B'₀ et B'ᵢ représentent indépendamment les uns des autres une base azotée choisie parmi l'adénine, la guanine, la thymine, et la cytosine,
   la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* linker représente un linker notamment choisi parmi :
   - un groupe -CO-Z-CO-, dans laquelle Z représente un groupe alkyle de 1 à 2 carbones ou un groupe -CH₂-O-Ph-O-CH₂-,
   - un groupe -CO-CO-,
   - un groupe de formule (A) ou (B) suivante : 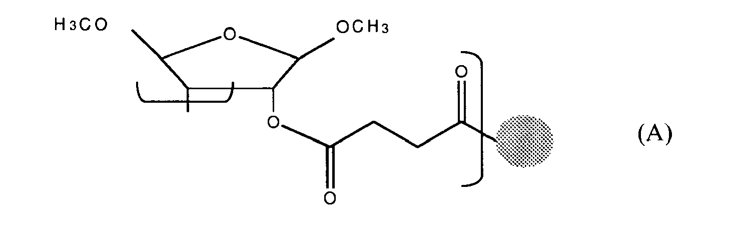 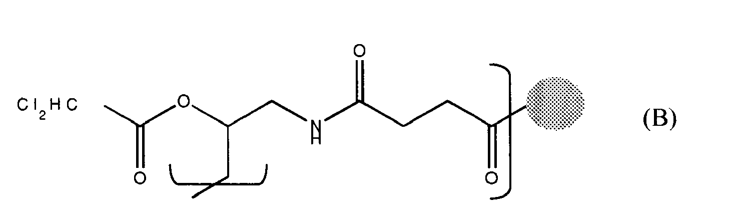
* 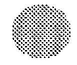 représente un support solide notamment choisi parmi CPG (Controlled Pore Glass) avec longue chaîne aminoalkyle (Icca) de différentes porosités et les résines dérivées du polystyrène,
   suivie d'une réaction en milieu basique, notamment avec de l'hydroxyde d'ammonium, de l'hydroxyde de sodium, de la méthylamine, du carbonate de potassium, ou un mélange de ces bases,
   pour obtenir le dit composé de formule (III).

Dans ce mode de réalisation, des oligonucléotides non modifiés d'ADN, et comportant une extrémité 5'-phosphate monoester sont préparés.

Selon un autre mode de réalisation, le procédé de préparation défini ci-dessus permet la préparation d'un composé de formule (IV) suivante : 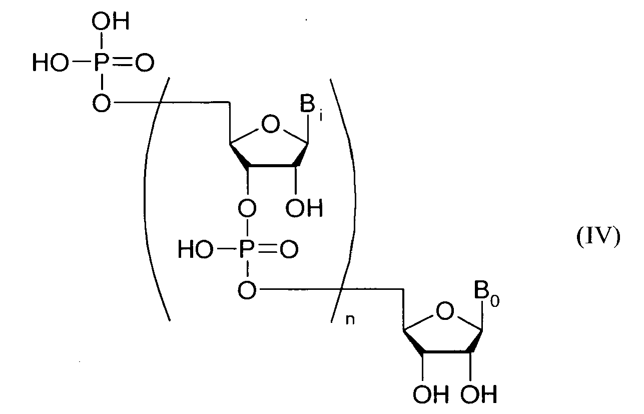 dans laquelle :
* n est un nombre entier compris de 1 à 100, notamment de 1 à 50, et en particulier de 5 à 40
* i est un nombre entier compris de 1 à n,
* B₀ et Bᵢ représentent indépendamment les uns des autres l'adénine, la guanine, la cytosine, ou l'uracile,
   comprenant l'étape de :
   réaction d'un oxydant choisi parmi les dérivés de l'iode hypervalent, notamment l'acide 2-iodoxybenzoique (IBX) et le Dess-Martin periodinane (DMP), et le réactif de Swern
   sur un composé de formule (XIV) suivante : 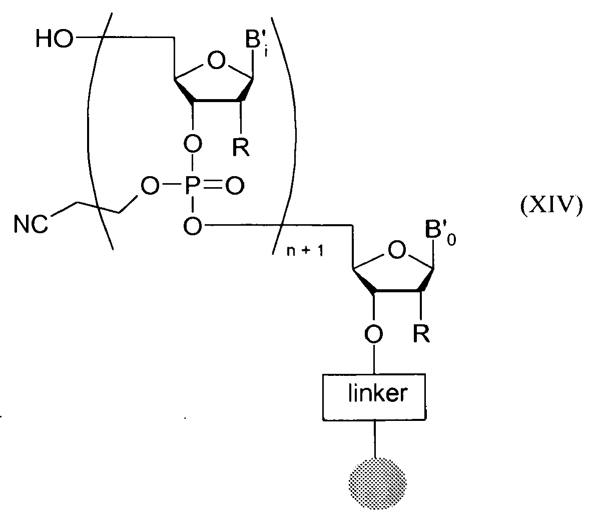
   dans laquelle :
* n est un nombre entier compris de 1 à 100, notamment de 1 à 50, et en particulier de 5 à 40
* i est un nombre entier compris de 1 à n+1,
* B'₀ et B'ᵢ représentent indépendamment les uns des autres une base azotée choisie parmi l'adénine, la guanine, la cytosine, et l'uracile,
   la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* linker représente un linker notamment choisi parmi :
   - un groupe -CO-Z-CO-, dans laquelle Z représente un groupe alkyle de 1 à 2 carbones ou un groupe -CH₂-O-Ph-O-CH₂-,
   - un groupe -CO-CO-,
   - un groupe de formule (A) ou (B) suivante : 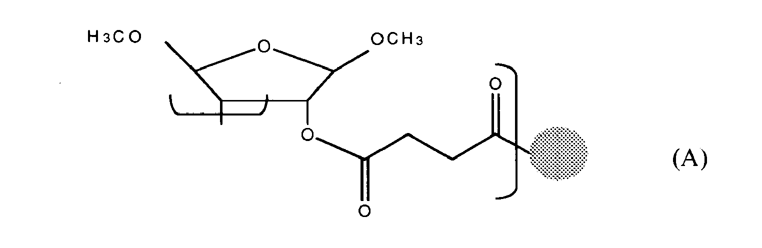 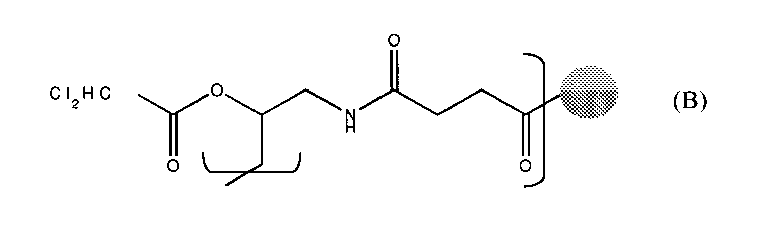
* 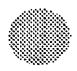 représente un support solide notamment choisi parmi CPG (Controlled Pore Glass) avec longue chaîne aminoalkyle (Icca) de différentes porosités et les résines dérivées du polystyrène,
   suivie d'une réaction en milieu basique, notamment avec de l'hydroxyde d'ammonium, de l'hydroxyde de sodium, de la méthylamine, du carbonate de potassium, ou un mélange de ces bases,
   pour obtenir le dit composé de formule (IV).

Dans ce mode de réalisation, des oligonucléotides non modifiés d'ARN, et comportant une extrémité 5'-phosphate monoester sont préparés.

Selon un autre mode de réalisation, le procédé de préparation défini ci-dessus permet la préparation d'un composé de formule (V') suivante:
dans laquelle : 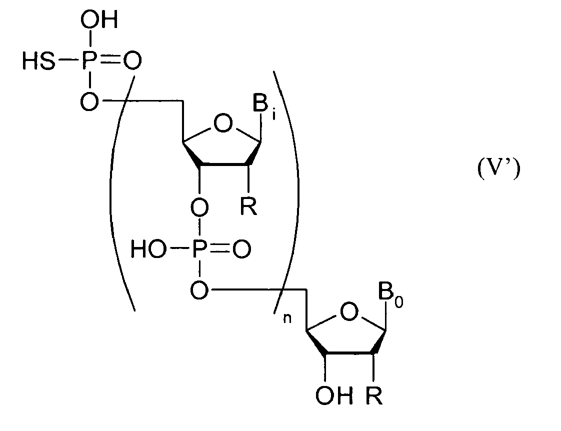
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la thymine, la cytosine, ou l'uracile,
* R représente l'hydrogène ou un groupement hydroxyle,
   comprenant l'étape de :
   réaction d'un oxydant choisi parmi les dérivés de l'iode hypervalent, notamment l'acide 2-iodoxybenzoique (IBX) et le Dess-Martin periodinane (DMP), et le réactif de Swern,
   sur un composé de formule (XXI) suivante : 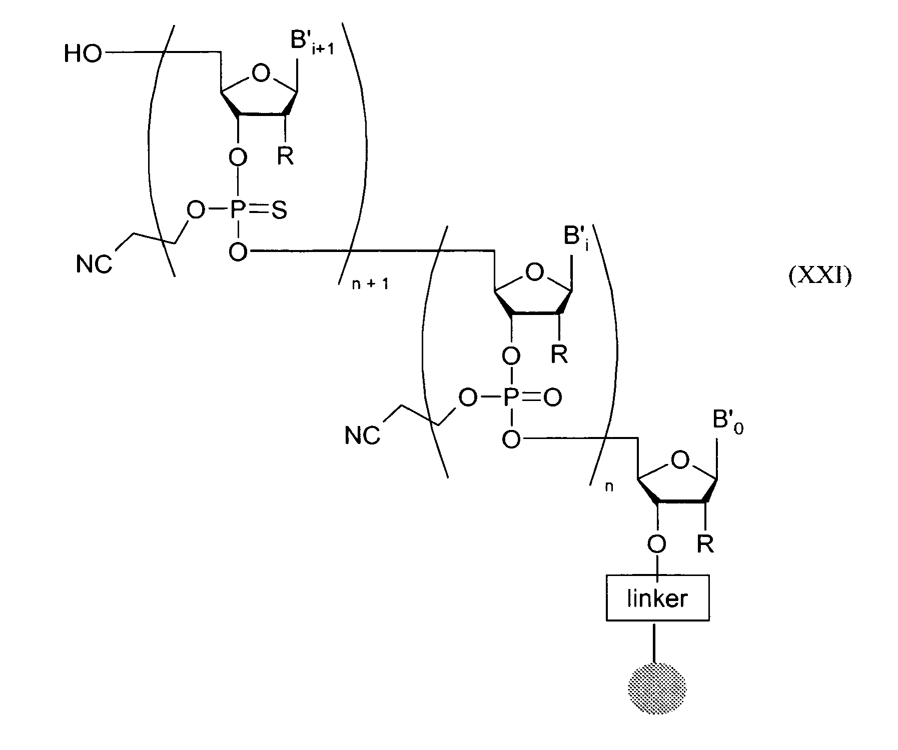
   * n est un nombre entier compris de 1 à 100, notamment de 1 à 50, et en particulier de 5 à 40
   * i est un nombre entier compris de 1 à n+1,
   * B'₀, B'ᵢ et B'ᵢ₊₁ représentent indépendamment les uns des autres une base azotée choisie parmi l'adénine, la guanine, la cytosine, et l'uracile,
      la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
   * R représente un groupement hydroxyle protégé par un groupe protecteur, notamment choisi parmi les groupes acétyle (Ac), silyle tels que tertbutyldiméthylsilyl (TBDMS) et triisopropylsilyloxyméthyl (TOM).
   * linker représente un linker notamment choisi parmi :
      - un groupe -CO-Z-CO-, dans laquelle Z représente un groupe alkyle de 1 à 2 carbones ou un groupe -CH₂-O-Ph-O-CH₂-,
      - un groupe -CO-CO-,
      - un groupe de formule (A) ou (B) suivante : 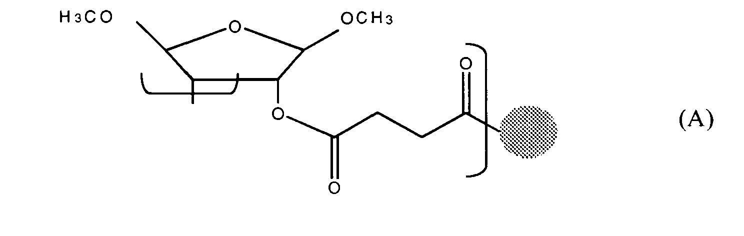 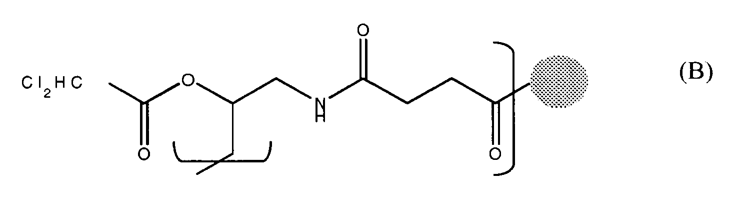
* 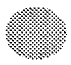 représente un support solide notamment choisi parmi CPG (Controlled Pore Glass) avec longue chaîne aminoalkyle (Icca) de différentes porosités et les résines dérivées du polystyrène,
   suivie d'une réaction en milieu basique, notamment avec de l'hydroxyde d'ammonium, de l'hydroxyde de sodium, de la méthylamine, du carbonate de potassium, ou un mélange de ces bases,
   pour obtenir le dit composé de formule (V').

Les oligonucléotides ainsi synthétisés sont des oligonucléotides non modifiés d'ADN ou d'ARN et comportant une extrémité 5'-thiophosphate monoester.

Selon un autre mode de réalisation encore plus préféré, le procédé de préparation défini ci-dessus permet la préparation d'un composé de formule (V) suivante :
dans laquelle : 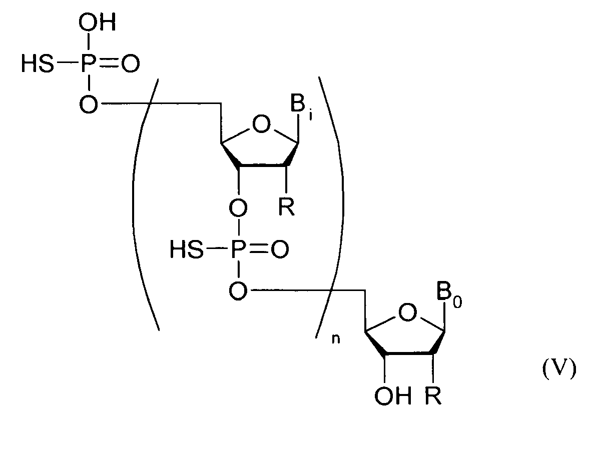
   * n est un nombre entier compris de 1 à 100, notamment de 1 à 50, et en particulier de 5 à 40
   * i est un nombre entier compris de 1 à n,
   * B₀ et Bᵢ représentent indépendamment les uns des autres une base azotée, notamment :
      - un dérivé de purine, notamment l'adénine, la guanine, la 8-hydroxy guanine, l'inosine, ou,
      - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
   * R représente l'hydrogène ou un groupement hydroxyle,
      comprenant l'étape de :
      réaction d'un oxydant choisi parmi les dérivés de l'iode hypervalent, notamment l'acide 2-iodoxybenzoique (IBX) et le Dess-Martin periodinane (DMP), et le réactif de Swern,
      sur un composé de formule (XV) suivante : 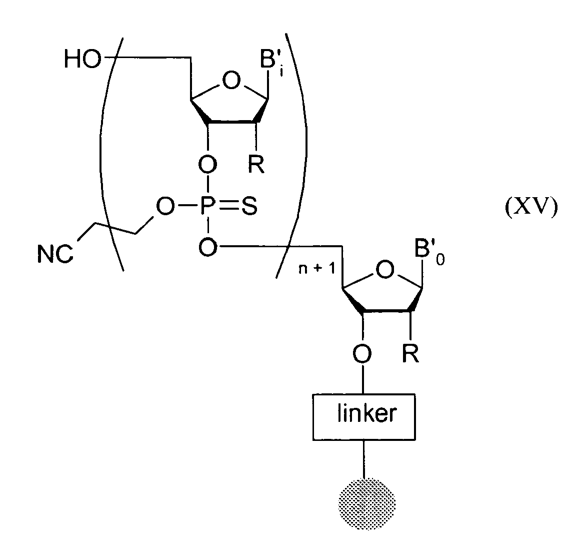 dans laquelle :
      * n est un nombre entier compris de 1 à 100, notamment de 1 à 50, et en particulier de 5 à 40
      * i est un nombre entier compris de 1 à n+1,
      * B'₀ et B'ᵢ représentent indépendamment les uns des autres une base azotée, notamment :
         - un dérivé de purine, notamment l'adénine, la guanine, la 8-hydroxy guanine, l'inosine, ou,
         - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
            la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
      * R représente l'hydrogène ou un groupement hydroxyle protégé par un groupe protecteur, notamment choisi parmi les groupes acétyle (Ac), silyle tels que tertbutyldiméthylsilyl (TBDMS) et triisopropylsilyloxyméthyl (TOM).
      * linker notamment choisi parmi :
         - un groupe -CO-Z-CO-, dans laquelle Z représente un groupe alkyle de 1 à 2 carbones ou un groupe -CH₂-O-Ph-O-CH₂-,
         - un groupe -CO-CO-,
         - un groupe de formule (A) ou (B) suivante : 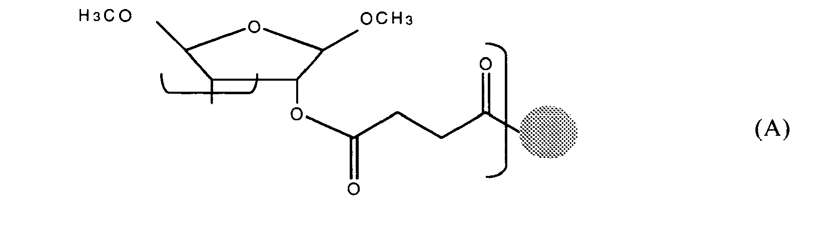 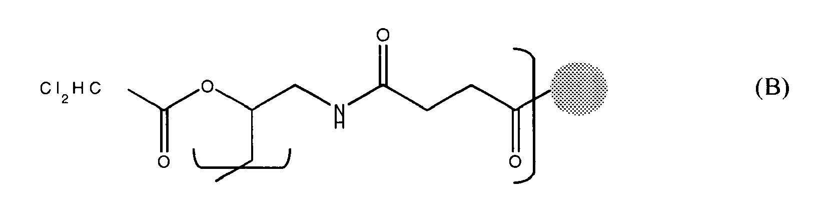
* 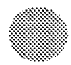 représente un support solide notamment choisi parmi CPG (Controlled Pore Glass) avec longue chaîne aminoalkyle (lcca) de différentes porosités et les résines dérivées du polystyrène,
   suivie d'une réaction en milieu basique, notamment avec de l'hydroxyde d'ammonium, de l'hydroxyde de sodium, de la méthylamine, du carbonate de potassium, ou un mélange de ces bases,
   pour obtenir le dit composé de formule (V).

Dans ce mode de réalisation, des oligonucléotides modifiés d'ADN et d'ARN (de type phosphorothioate), et comportant une extrémité 5'-thiophosphate monoester sont préparés.

Selon un autre mode de réalisation encore plus préféré, le procédé de préparation défini ci-dessus permet la préparation d'un composé de formule (VI) suivante : 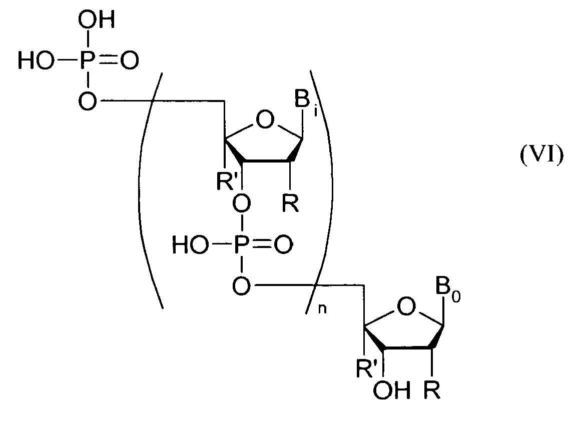 dans laquelle :
* n est un nombre entier compris de 1 à 100, notamment de 1 à 50, et en particulier de 5 à 40
* i est un nombre entier compris de 1 à n,
* B₀ et Bᵢ représentent indépendamment les uns des autres une base azotée, notamment :
   - un dérivé de purine, notamment l'adénine, la guanine, la 8-hydroxy guanine, l'inosine, ou,
   - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente :
   - un groupement -O-alkyle comportant 1 à 4 atomes de carbones linéaire ou ramifié, ou,
   - un groupement -O-(CH₂)ₘ-NH₂, dans lequel m représente un nombre entier compris de 2 à 10, ou,
   - ou un atome d'oxygène,
* R' représente :
   - l'hydrogène, ou,
   - un groupe -(CH₂)ₚ- dans lequel p varie de 1 à 4,
      tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther, et,
      comprenant l'étape de :
      réaction d'un oxydant choisi parmi les dérivés de l'iode hypervalent, notamment l'acide 2-iodoxybenzoique (IBX) et le Dess-Martin periodinane (DMP), et le réactif de Swern,
      sur un composé de formule (XVI) suivante : 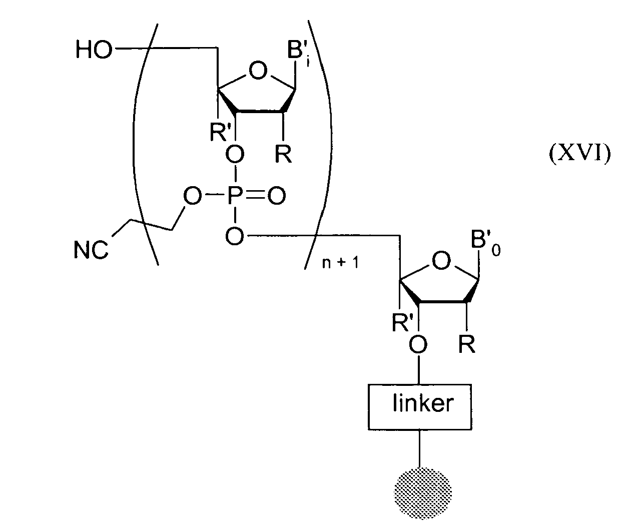
      dans laquelle :
* n est un nombre entier compris de 1 à 100, notamment de 1 à 50, et en particulier de 5 à 40,
* i est un nombre entier compris de 1 à n,
* B'₀ et B'ᵢ représentent indépendamment les uns des autres une base azotée, notamment :
   - un dérivé de purine, notamment l'adénine, la guanine, la 8-hydroxy guanine, l'inosine, ou,
   - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
      la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* R représente :
   - un groupement -O-alkyle comportant 1 à 4 atomes de carbones linéaire ou ramifié, ou
   - un groupement -O-(CH₂)ₘ-NH₂, et dans lequel la fonction amine est protégée par un groupe protecteur, notamment choisi parmi les carbamates tels que le groupe fluorénylméthyloxycarbonyle (Fmoc), les amides tel que le trifluoroacétamide (CO-CF3), et le phtalimide,
      dans lequel m représente un nombre entier compris de 2 à 10, ou
   - un atome d'oxygène,
* R' représente :
   - l'hydrogène, ou,
   - un groupe -(CH₂)ₚ- dans lequel p varie de 1 à 4,
      tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther, sauf dans le cas de la dernière base qui subit la β-élimination, où R' représente l'hydrogène uniquement.
      et,
* Rᵢ" représentent indépendamment un groupement hydroxyle ou thiol,
* linker notamment choisi parmi :
   - un groupe -CO-Z-CO-, dans laquelle Z représente un groupe alkyle de 1 à 2 carbones ou un groupe -CH₂-O-Ph-O-CH₂-,
   - un groupe -CO-CO-,
   - un groupe de formule (A) ou (B) suivante : 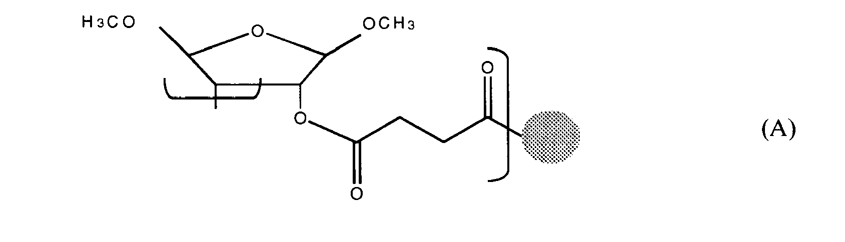 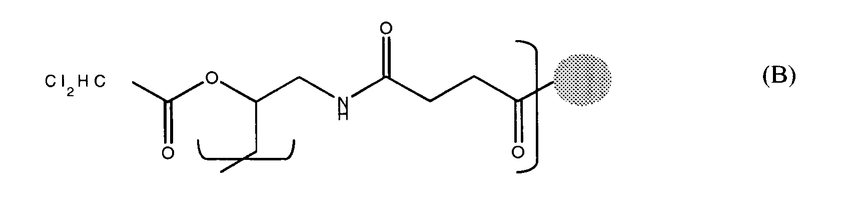
* 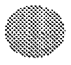 représente un support solide notamment choisi parmi CPG (Controlled Pore Glass) avec longue chaîne aminoalkyle (Icca) de différentes porosités et les résines dérivées du polystyrène,
   suivie d'une réaction en milieu basique, notamment avec de l'hydroxyde d'ammonium, de l'hydroxyde de sodium, de la méthylamine, du carbonate de potassium, ou un mélange de ces bases,
   pour obtenir le dit composé de formule (VI).

Dans ce mode de réalisation, des oligonucléotides modifiés d'ARN, et comportant une extrémité 5'-phosphate monoester sont préparés.

Selon un autre aspect, l'invention concerne un procédé de préparation d'un composé de formule (XX) suivante : 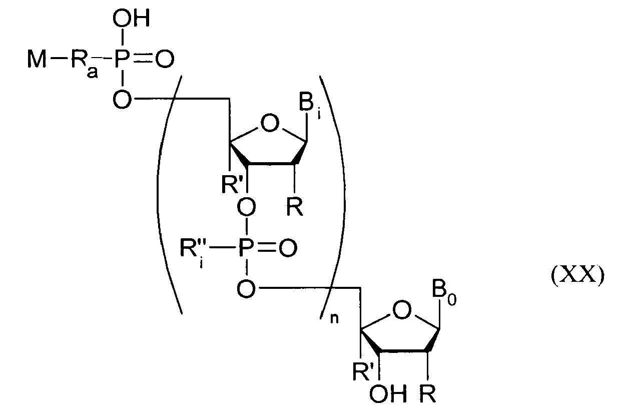 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres une base azotée, notamment :
   - un dérivé de purine, notamment l'adénine, la guanine, la 8-hydroxy guanine, l'inosine, ou,
   - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente :
   - l'hydrogène, ou,
   - un groupement hydroxyle, ou
   - un groupement -O-alkyle comportant 1 à 4 atomes de carbones linéaire ou ramifié, ou
   - un atome d'oxygène,
      * Rᵢ'' représentent indépendamment un groupement hydroxyle ou thiol,
      * Ra représente un atome de soufre ou d'oxygène,
      * M représente une sonde fluorescente, un peptide, un oligonucléotide ou un agent intercalant,
         comprenant l'étape de :
         réaction d'un oxydant choisi parmi les dérivés de l'iode hypervalent, notamment l'acide 2-iodoxybenzoique (IBX) et le Dess-Martin periodinane (DMP), et le réactif de Swern,
         sur un composé de formule (XI) suivante : 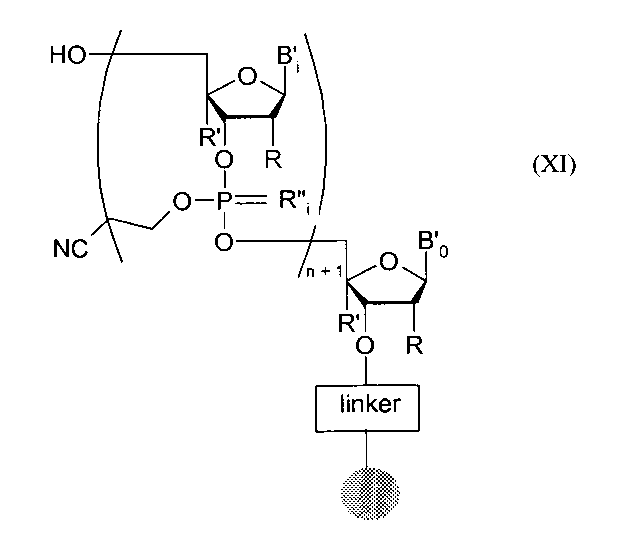
      dans laquelle :
      * n est un nombre entier compris de 1 à 100, notamment de 1 à 50, et en particulier de 5 à 40,
      * i est un nombre entier compris de 1 à n+1,
      * B'₀ et B'ᵢ représentent indépendamment les uns des autres une base azotée, notamment :
         - un dérivé de purine, notamment l'adénine, la guanine, la 8-hydroxy guanine, l'inosine, ou,
         - un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
            la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
            * R représente :
         - l'hydrogène, ou,
         - un groupement hydroxyle, protégé par un groupe protecteur, notamment choisi parmi les groupes acétyle (Ac), silyle tels que tertbutyldiméthylsilyl (TBDMS) et triisopropylsilyloxyméthyl (TOM) ou
         - un groupement -O-alkyle comportant 1 à 4 atomes de carbones linéaire ou ramifié, ou
         - un atome d'oxygène,
      * R' représente :
         - l'hydrogène, ou,
         - un groupe -(CH₂)ₚ- dans lequel p varie de 1 à 4,
            tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther,
            sauf dans le cas de la dernière base qui subit la β-élimination, où R' représente l'hydrogène uniquement.
         * Rᵢ'' représentent indépendamment un atome d'oxygène ou de soufre,
         * linker représente un linker notamment choisi parmi :
            - un groupe -CO-Z-CO-, dans laquelle Z représente un groupe alkyle de 1 à 2 carbones ou un groupe -CH₂-O-Ph-O-CH₂-,
            - un groupe -CO-CO-,
            - un groupe de formule (A) ou (B) suivante : 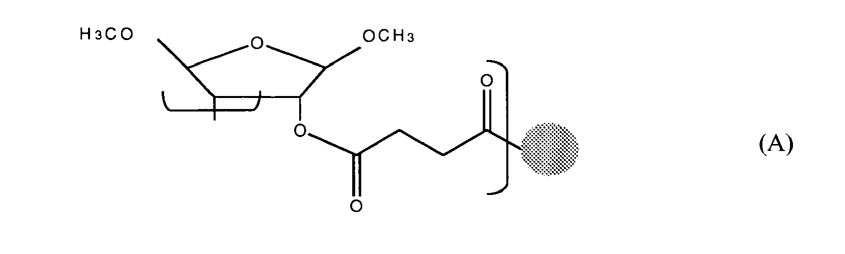 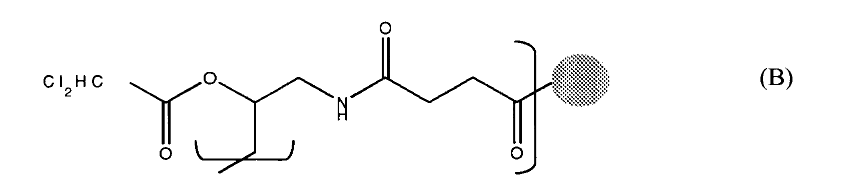
      * 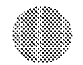 représente un support solide, notamment choisi parmi CPG (Controlled Pore Glass) avec longue chaîne aminoalkyle (Icca) de différentes porosités et les résines dérivées du polystyrène,
         suivie d'une réaction avec une molécule M-GP, dans laquelle M représente une sonde fluorescente, un peptide, un oligonucléotide ou un agent intercalant, et GP représente un groupe partant, notamment choisi parmi le chlore, le brome, l'iode, les groupes tosylate, mésylate, lorsque Ra est un soufre,
         ou avec une molécule M-OH ou M-NH2 après activation du phosphate en 5' lorsque Ra est un oxygène.
         et d'une réaction en milieu basique, notamment avec de l'hydroxyde d'ammonium, de l'hydroxyde de sodium, de la méthylamine, du carbonate de potassium, ou un mélange de ces bases,
         les dites réactions avec une molécule M-GP, M-OH ou M-NH₂ et en milieu basique pouvant être effectuées successivement ou en une seule étape « one-pot », pour obtenir le dit composé de formule (XX).

Par « sonde fluorescente », on entend des molécules possédant des propriétés de luminescence tels que la cyanine, la fluorescéine, le Texas Red sans être limité à ceux-ci.

Par « oligonucléotide », on entend un oligonucléotide synthétisé par un procédé tel que ci-dessus ou par un procédé bien connu par l'homme du métier et permettant la synthèse d'oligonucléotides plus longs, notamment par synthèse convergente par fragments.

Par agent intercalant, on entend des molécules de type poly-aromatique ayant des propriétés d'intercalation dans l'ADN tels que l'acridine, la quinacridine, sans être limité à celles-ci.

Le traitement en milieu basique comme ci-dessus permet d'effectuer la déprotection de l'oligonucléotide de son support et de cliver les groupes protecteurs encore présents.

Selon un mode de réalisation préféré, l'oxydant utilisés dans les procédés ci-dessus est l'IBX.

### PARTIE EXPERIMENTALE

### 1) Schéma général de synthèse des oligonucléotides comportant une liaison 5'-phosphate ou 5'-thiophosphate monoester

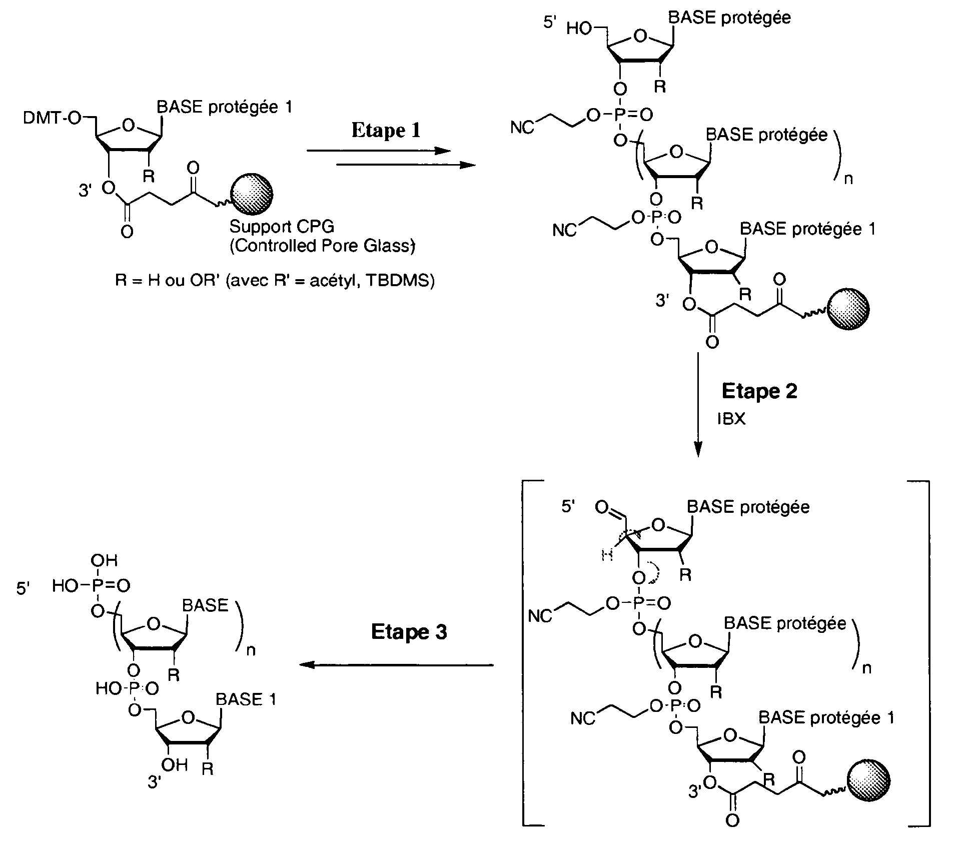

L'étape 1 correspond au cycle complet de synthèse d'oligonucléotide par voie phosphoramidite et consiste à la déprotection (détritylation) à l'acide trichloroacétique ou dichloroacétique, suivie du couplage avec la base suivante (n+1) sous forme de phosphoramidite, de l'oxydation du phosphore III en phosphore P(V) par une solution aqueuse d'iode, du "capping" (protection des alcools en 5' n'ayant pas réagit) par acétylation.
L'étape **2** correspond à l'étape d'oxydation par le dérivé de l'iode hypervalent.
L'étape **3** correspond à la déprotection par l'ammoniaque.

Sur ce schéma sont représentée les molécules dans lesquelles R' = H.

Ce même schéma est également valable dans le cas ou R' = un groupe -(CH₂)ₚ- dans lequel p varie de 1 à 4, à la condition que pour la dernière base, R' = H.

La formation du 5'-phosphate est vraisemblablement due à un mécanisme de β-élimination par arrachement du proton acide en α de l'aldéhyde généré par l'oxydation par IBX.

Une fois l'élimination effectuée, il est alors possible de faire réagir sur l'intermédiaire non isolé une molécule M-PG pour obtenir les composés définis ci-dessus.

### 2) Protocole pour oligonucléotides conventionnels (en série ADN ou ARN) :

L'oligonucléotide est préparé par la voie classique de synthèse sur support solide (voie aux phosphoramidites). En fin de synthèse, l'oligonucléotide est attaché par son extrémité 3' sur le support solide sous forme complètement protégée. Ainsi, les groupes amines exocycliques des nucléobases sont protégés sous forme de benzoyle (pour dA et dC) ou isobutyryle (pour dG) ou autres groupes protecteurs commerciaux, le phosphodiester internucléosidique sous forme de cyanoéthyle et l'alcool en 3' sous forme d'ester avec le support.

En série ARN, la fonction alcool en 2' est protégée sous forme de TBDMS ou acétyl ou autres groupes protecteurs décrits pour cette protection. Ce support est alors mis dans une solution d'IBX dans le DMSO (solution commerciale à 100 mM) et chauffé à 60° pendant 16 heures. Cette étape a pour but d'oxyder la fonction alcool primaire en 5' en aldéhyde.

Le support solide est ensuite récupéré et mis dans une solution d'ammoniaque à 28% et chauffé à 55°C pendant 16 heures. Ce traitement est communément utilisé en synthèse conventionnelle des oligonucléotides et correspond à l'étape de coupure de l'oligonucléotide du support et de déprotection des amines des nucléobases et du lien phosphodiester. L'oligonucléotide est ensuite purifié selon les méthodes classiques (chromatographie liquide haute performance ou électrophorèse sur gel).

### 3) Protocole pour oligonucléotides modifiés :

Le protocole de synthèse est le même que celui décrit auparavant pour les oligonucléotides naturels. La modification est incorporée selon le protocole décrit par le constructeur.

Par exemple, des liens phosphorothioates peuvent remplacer les liens phosphodiesters en utilisant le réactif de Beaucage lors de l'étape d'oxydation, la fonction OH en 2' peut-être sous forme alcoxy ou attaché via un méthylène à la position 4' (LNA).

## Revendications

1. Utilisation d'au moins un oxydant notamment choisi parmi :
- les dérivés de l'iode hypervalent, notamment l'acide 2-iodoxybenzoique (IBX) et le Dess-Martin periodinane (DMP), et,
- le réactif de Swern,
avec un oligonucléotide modifié ou non, comportant une extrémité 5'-OH, pour la synthèse d'oligonucléotides, modifiés ou non, notamment d'oligoribonucléotides, d'oligodésoxyribonucléotides, les dits oligonucléotides comportant une extrémité 5'-phosphate monoester ou 5'-thiophosphate monoester.

2. Utilisation selon la revendication 1, d'un oligonucléotide modifié ou non,
- comportant une extrémité 5'-OH, et,
- contenant (n+1) nucléotides,
pour obtenir respectivement un oligonucléotide modifié ou non,
- comportant une extrémité 5'-phosphate monoester ou 5'-thiophosphate monoester, et,
- contenant n nucléotides.

3. Utilisation selon l'une quelconque des revendications 1 ou 2, dans laquelle la synthèse est une synthèse sur support.

4. Utilisation selon l'une quelconque des revendications 1 à 3, pour la synthèse d'un composé de formule (I) suivante : 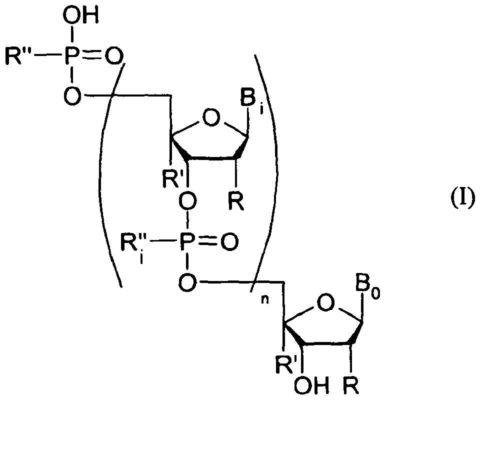 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres une base azotée, notamment :
- un dérivé de purine, notamment l'adénine, la guanine, la 8-hydroxy guanine, la 8-hydroxy-adénine, 8-amino-guanine, 8-méthoxy-guanine, 8-bromo-guanine, 8-bromo-adénine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente :
- l'hydrogène, ou,
- un groupement hydroxyle, ou
- un groupement -O-alkyle comportant 1 à 4 atomes de carbones linéaire ou ramifié, ou
- un groupement -O-(CH₂)ₘ-NH₂, dans lequel m représente un nombre entier compris de 2 à 10, ou
- un atome d'oxygène,
* R' représente :
- l'hydrogène, ou,
- un groupe -(CH₂)ₚ- dans lequel p varie de 1 à 4,
tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther, et,
* R" et Rᵢ" représentent indépendamment un groupement hydroxyle (OH) ou mercapto (SH).

5. Utilisation selon la revendication 4, pour la synthèse d'un composé de formule (II)
suivante; 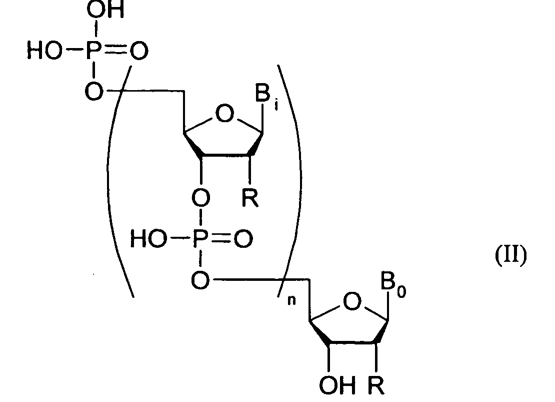 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la thymine, la cytosine, ou l'uracile,
* R représente l'hydrogène ou un groupement hydroxyle.

6. Utilisation selon l'une quelconque des revendications 4 et 5, pour la synthèse d'un composé de formule (III) suivante : 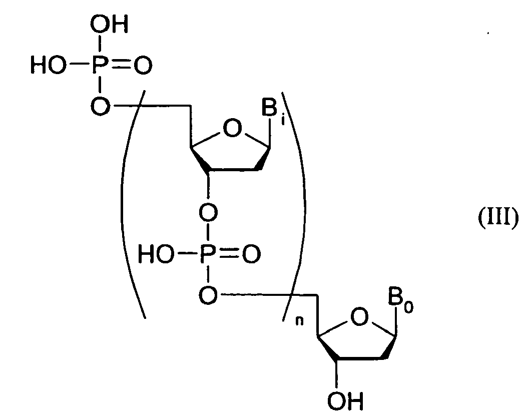 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la thymine, ou la cytosine.

7. Utilisation selon l'une quelconque des revendications 4 à 5, pour la synthèse d'un composé de formule (IV) suivante : 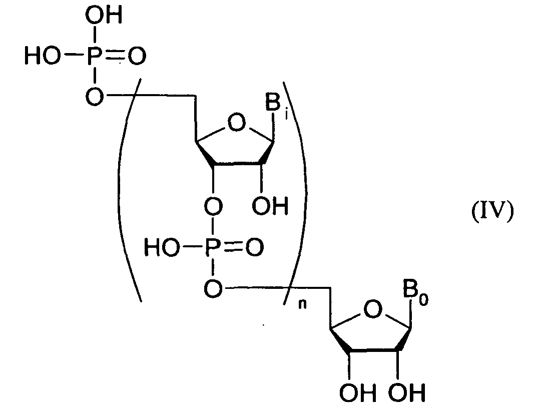 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la cytosine, ou l'uracile.

8. Utilisation selon la revendication 4, pour la synthèse d'un composé de formule (V') suivante : 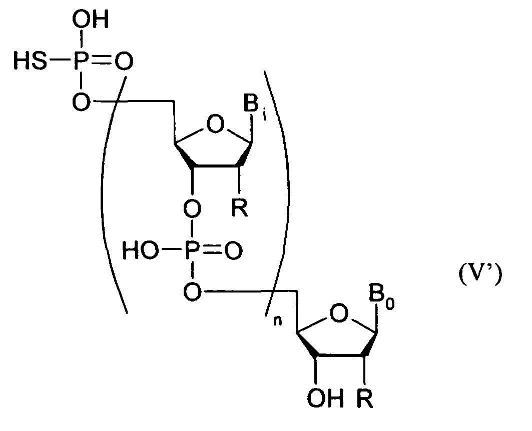 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la cytosine, ou l'uracile.
* R représente l'hydrogène ou un groupement hydroxyle.

9. Utilisation selon la revendication 4, pour la synthèse d'un composé de formule (V) suivante : 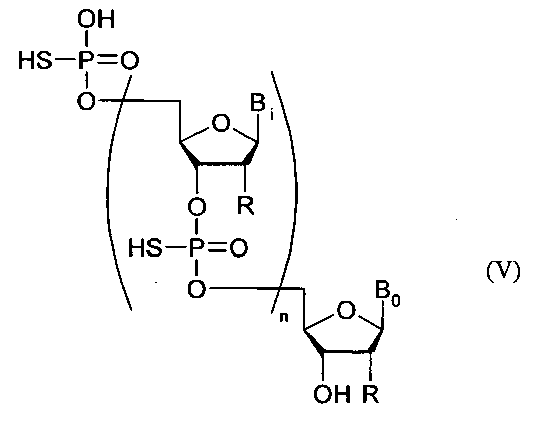 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente l'hydrogène ou un groupement hydroxyle.

10. Utilisation selon la revendication 4, pour la synthèse d'un composé de formule (VI) suivante : 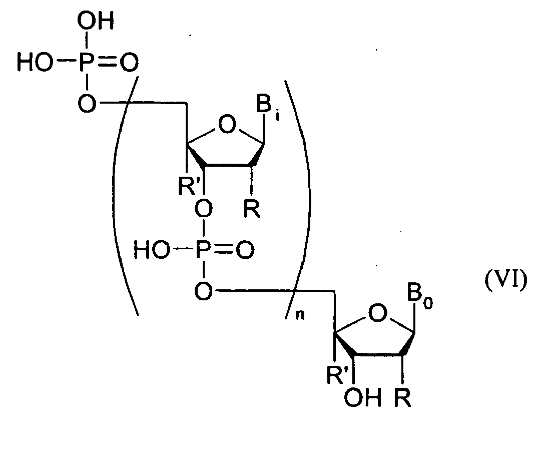 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente :
- un groupement -O-alkyle comportant 1 à 4 atomes de carbones linéaire ou ramifié, ou,
- un groupement -O-(CH₂)ₘ-NH₂, dans lequel m représente un nombre entier compris de 2 à 10, ou,
- ou un atome d'oxygène,
* R' représente :
- l'hydrogène, ou,
- un groupe -(CH₂)ₚ- dans lequel p varie de 1 à 4,
tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther.

11. Utilisation selon l'une quelconque des revendications 1 à 10, dans laquelle l'oxydant est l'IBX.

12. Procédé de préparation d'un composé, tel que défini dans la revendication 1, de formule (I) suivante : 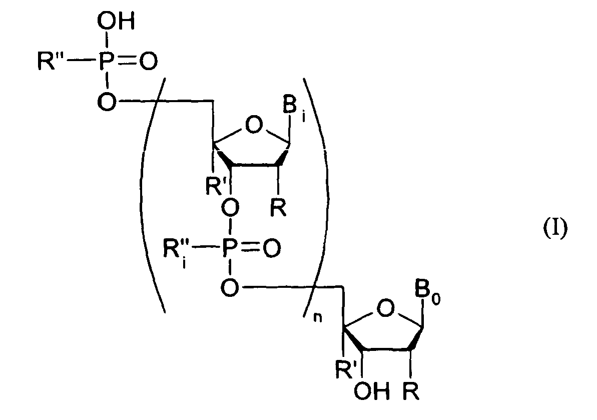 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente :
- l'hydrogène, ou,
- un groupement hydroxyle, ou
- un groupement -O-alkyle comportant 1 à 4 atomes de carbones linéaire ou ramifié, ou
- un groupement -O-(CH₂)ₘ-NH₂, dans lequel m représente un nombre entier compris de 2 à 10, ou
- un atome d'oxygène,
* R' représente :
- l'hydrogène, ou,
- un groupe -(CH₂)ₚ- dans lequel p varie de 1 à 4,
tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther, et,
* R" et Rᵢ'' représentent indépendamment un groupement hydroxyle ou thiol, comprenant l'étape de :
réaction d'un oxydant choisi parmi les dérivés de l'iode hypervalent, notamment l'acide 2-iodoxybenzoique (IBX) et le Dess-Martin periodinane (DMP), et le réactif de Swern,
sur un composé de formule (XI) suivante : 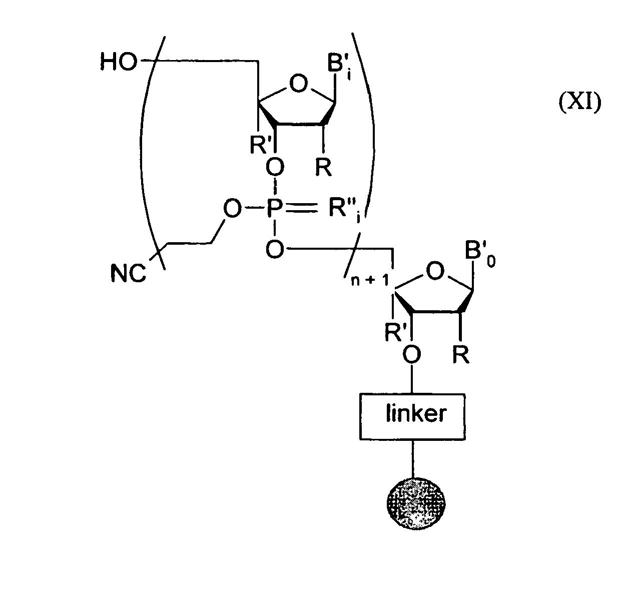 dans laquelle :
* n est un nombre entier compris de 1 à 100, notamment de 1 à 50, et en particulier de 5 à 40,
* i est un nombre entier compris de 1 à n+1,
* B'₀ et B'ᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* R représente :
- l'hydrogène, ou,
- un groupement hydroxyle protégé par un groupe protecteur, notamment choisi parmi les groupes acétyle (Ac), silyle tels que tertbutyldiméthylsilyl (TBDMS), triisopropylsilyloxyméthyl (TOM),
- un groupement -O-alkyle comportant 1 à 4 atomes de carbones linéaire ou ramifié, ou
- un groupement -O-(CH₂)ₘ-NH₂, dans lequel m représente un nombre entier compris de 2 à 10, et dans lequel la fonction amine est protégée par un groupe protecteur, notamment choisi parmi les carbamates tels que le groupe fluorénylméthyloxycarbonyle (Fmoc), les amides tel que le trifluoroacétamide (CO-CF3), et le phtalimide,
ou
- un atome d'oxygène,
* R' représente :
- l'hydrogène ou,
- un groupe -(CH₂)ₚ- dans lequel p varie de 1 à 4,
sauf dans le cas de la dernière base, où R' représente l'hydrogène.
tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther, et,
* Rᵢ'' représentent indépendamment un atome d'oxygène ou de soufre,
* linker représente un linker notamment choisi parmi :
- un groupe -CO-Z-CO-, dans laquelle Z représente un groupe alkyle de 1 à 2 carbones ou un groupe -CH₂-O-Ph-O-CH₂-,
- un groupe -CO-CO-,
- un groupe de formule (A) ou (B) suivante : 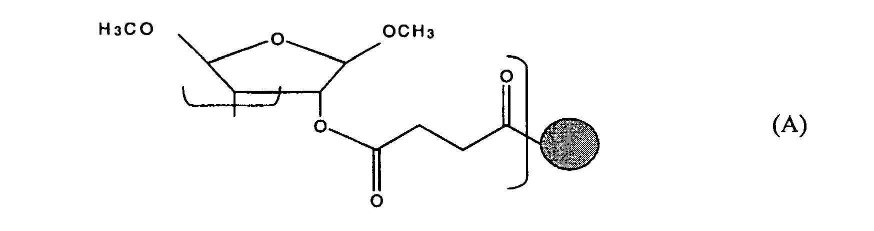 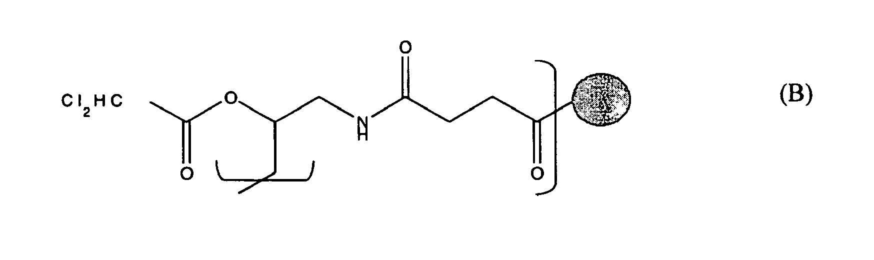
* 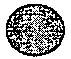 représente un support solide, notamment choisi parmi CPG (Controlled Pore Glass) avec longue chaîne aminoalkyle (Icca) de différentes porosités et les résines dérivées du polystyrène,
suivie d'une réaction en milieu basique, notamment avec de l'hydroxyde d'ammonium, de l'hydroxyde de sodium, de la méthylamine, du carbonate de potassium, ou un mélange de ces bases,
pour obtenir le dit composé de formule (I).

13. Procédé de préparation selon la revendication 12, d'un composé de formule (II) suivante : 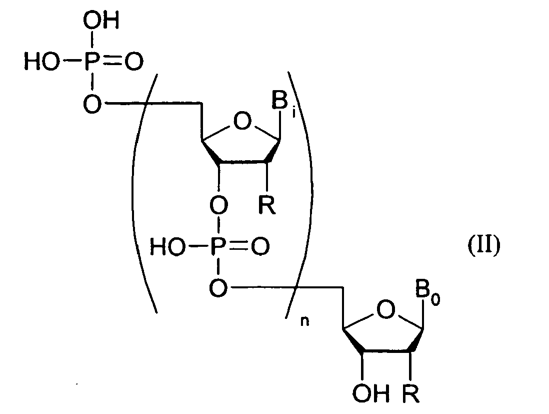 dans laquelle :
* n est un nombre entier compris de 1 à 100, notamment de 1 à 50, et en particulier de 5 à 40
* i est un nombre entier compris de 1 à n,
* B₀ et Bᵢ représentent indépendamment les uns des autres l'adénine, la guanine, la thymine, la cytosine, ou l'uracile,
* R représente l'hydrogène ou un groupement hydroxyle,
comprenant l'étape de :
réaction d'un oxydant choisi parmi les dérivés de l'iode hypervalent, notamment l'acide 2-iodoxybenzoique (IBX) et le Dess-Martin periodinane (DMP), et le réactif de Swern,
sur un composé de formule (XII) suivante : 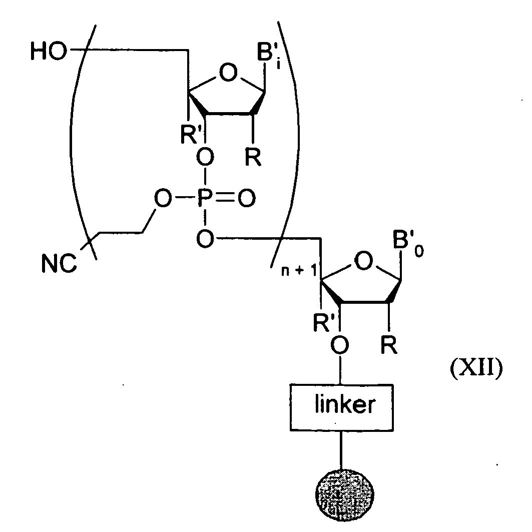
dans laquelle :
* n est un nombre entier compris de 1 à 100, notamment de 1 à 50, et en particulier de 5 à 40,
* i est un nombre entier compris de 1 à n+1,
* B'₀ et B'ᵢ représentent indépendamment les uns des autres une base azotée choisie parmi l'adénine, la guanine, la thymine, la cytosine, et l'uracile,
la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* R représente l'hydrogène ou un groupement hydroxyle,
* R' représente l'hydrogène
* linker représente un linker notamment choisi parmi :
- un groupe -CO-Z-CO-, dans laquelle Z représente un groupe alkyle de 1 à 2 carbones ou un groupe -CH₂-O-Ph-O-CH₂-,
- un groupe -CO-CO-,
- un groupe de formule (A) ou (B) suivante : 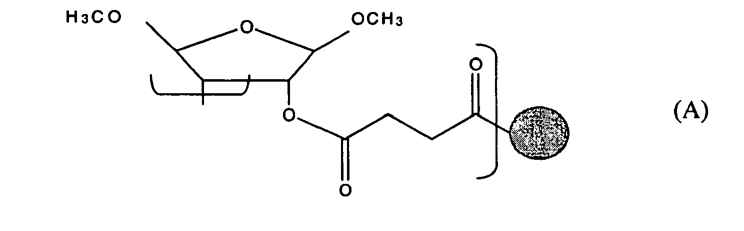 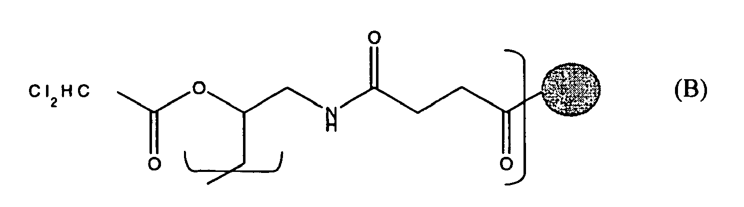
* 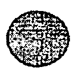 représente un support solide notamment choisi parmi CPG (Controlled Pore Glass) avec longue chaîne aminoalkyle (lcca) de différentes porosités et les résines dérivées du polystyrène,
suivie d'une réaction en milieu basique, notamment avec de l'hydroxyde d'ammonium, de l'hydroxyde de sodium, de la méthylamine, du carbonate de potassium, ou un mélange de ces bases,
pour obtenir le dit composé de formule (II).

14. Procédé de préparation selon l'une quelconque des revendications 12 et 13, d'un composé de formule (III) suivante :
dans laquelle : 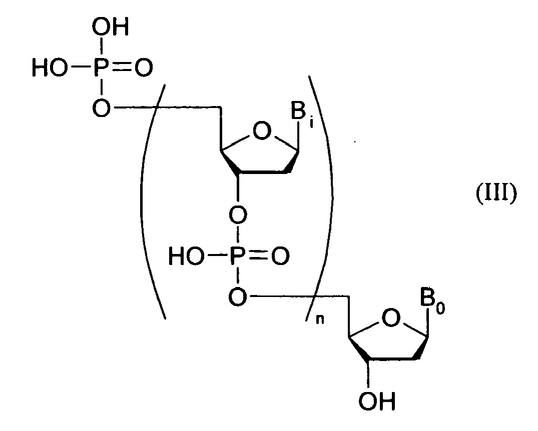
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres l'adénine, la guanine, la thymine, ou la cytosine,
comprenant l'étape de :
réaction d'un oxydant choisi parmi les dérivés de l'iode hypervalent, notamment l'acide 2-iodoxybenzoique (IBX) et le Dess-Martin periodinane (DMP), et le réactif de Swern,
sur un composé de formule (XIII) suivante : 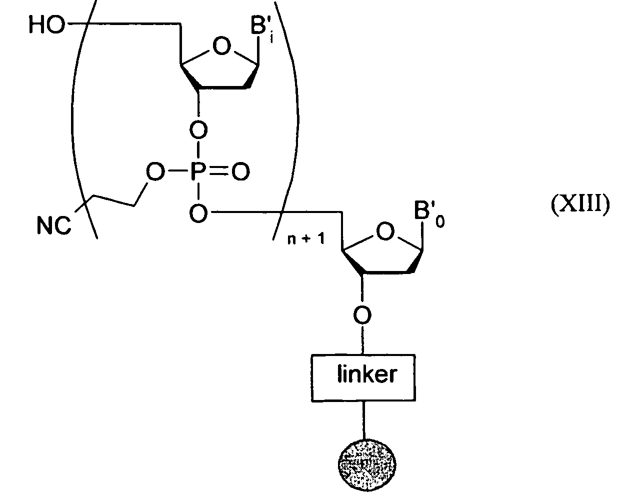
dans laquelle :
* n est un nombre entier compris de 1 à 100, notamment de 1 à 50, et en particulier de 5 à 40,
* i est un nombre entier compris de 1 à n+1,
* B'₀ et B'ᵢ représentent indépendamment les uns des autres une base azotée choisie parmi l'adénine, la guanine, la thymine, et la cytosine,
la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* linker représente un linker notamment choisi parmi :
- un groupe -CO-Z-CO-, dans laquelle Z représente un groupe alkyle de 1 à 2 carbones ou un groupe -CH₂-O-Ph-O-CH₂-,
- un groupe -CO-CO-,
- un groupe de formule (A) ou (B) suivante : 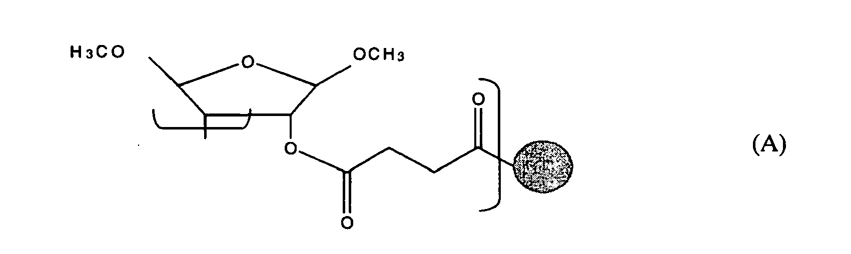 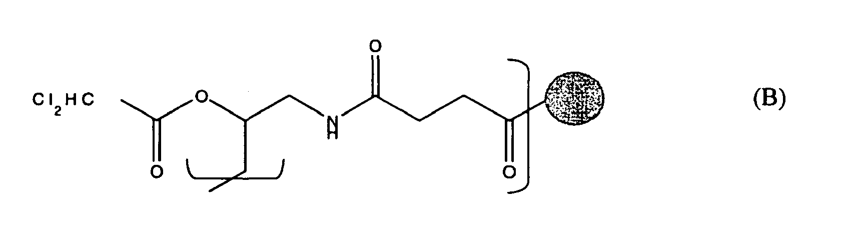
* représente un support solide notamment choisi parmi CPG (Controlled Pore Glass) avec longue chaîne aminoalkyle (1cca) de différentes porosités et les résines dérivées du polystyrène,
suivie d'une réaction en milieu basique, notamment avec de l'hydroxyde d'ammonium, de l'hydroxyde de sodium, de la méthylamine, du carbonate de potassium, ou un mélange de ces bases,
pour obtenir le dit composé de formule (III).

15. Procédé de préparation selon l'une quelconque des revendications 12 et 13, d'un composé de formule (IV) suivante :
dans laquelle : 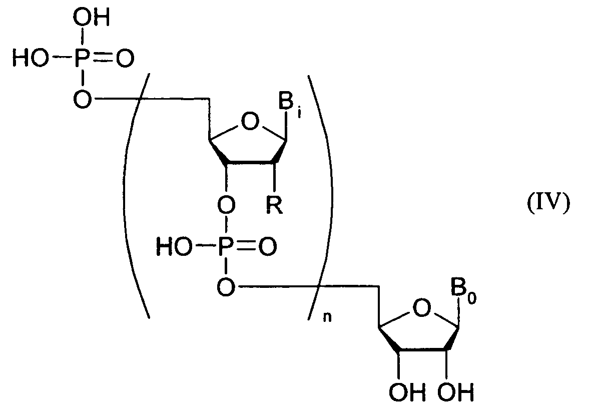
* n est un nombre entier compris de 1 à 100, notamment de 1 à 50, et en particulier de 5 à 40
* i est un nombre entier compris de 1 à n,
* B₀ et B₁ représentent indépendamment les uns des autres l'adénine, la guanine, la cytosine, ou l'uracile,
comprenant l'étape de :
réaction d'un oxydant choisi parmi les dérivés de l'iode hypervalent, notamment l'acide 2-iodoxybenzoique (IBX) et le Dess-Martin periodinane (DMP), et le réactif de Swern,
sur un composé de formule (XIV) suivante : 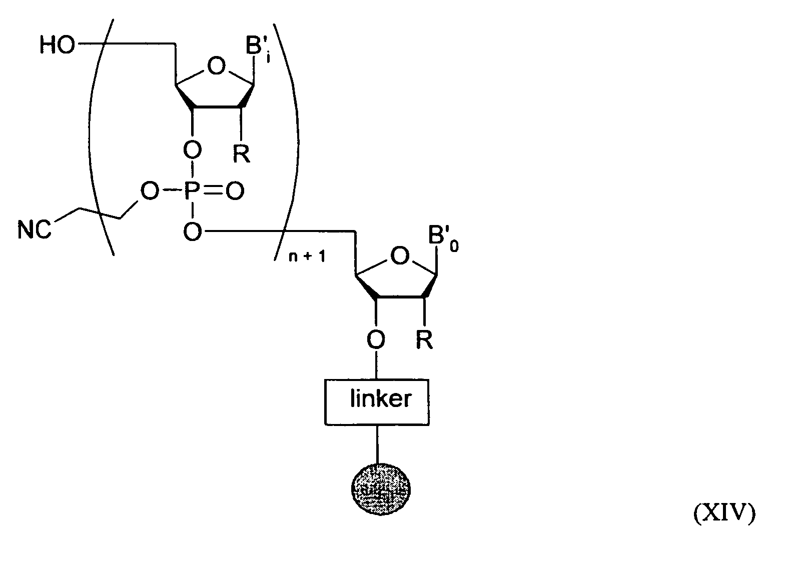 dans laquelle :
* n est un nombre entier compris de 1 à 100, notamment de 1 à 50, et en particulier de 5 à 40
* i est un nombre entier compris de 1 à n+1,
* B'₀ et B'ᵢ représentent indépendamment les uns des autres une base azotée choisie parmi l'adénine, la guanine, la cytosine, et l'uracile,
la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* linker représente un linker notamment choisi parmi :
- un groupe -CO-Z-CO-, dans laquelle Z représente un groupe alkyle de 1 à 2 carbones ou un groupe -CH₂-O-Ph-O-CH₂-,
- un groupe -CO-CO-,
- un groupe de formule (A) ou (B) suivante : 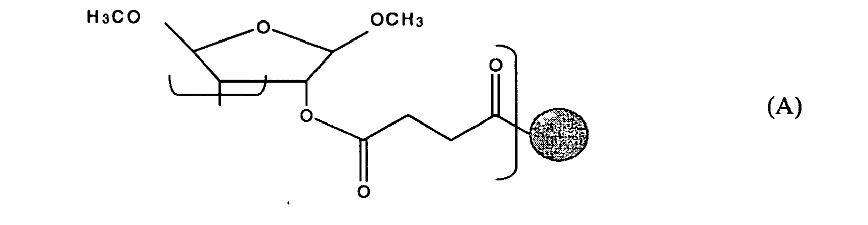 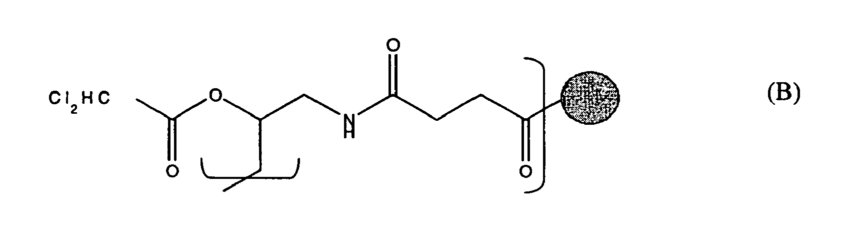
* représente un support solide notamment choisi parmi CPG (Controlled Pore Glass) avec longue chaîne aminoalkyle (Icca) de différentes porosités et les résines dérivées du polystyrène,
suivie d'une réaction en milieu basique, notamment avec de l'hydroxyde d'ammonium, de l'hydroxyde de sodium, de la méthylamine, du carbonate de potassium, ou un mélange de ces bases,
pour obtenir le dit composé de formule (IV).

16. Procédé de préparation selon la revendication 12, d'un composé de formule (V') suivante: 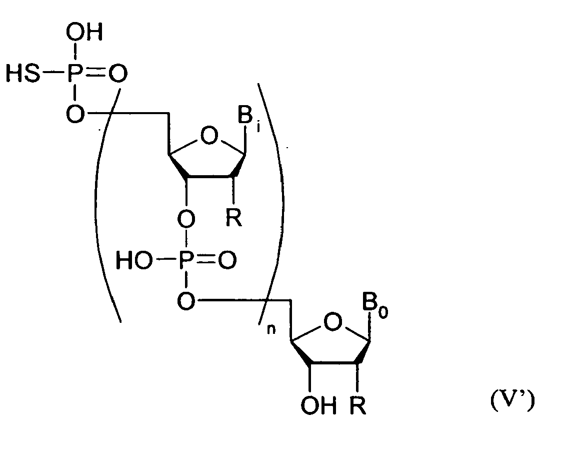 dans laquelle :
* n est un nombre entier compris de 1 à 100, notamment de 1 à 50, et en particulier de 5 à 40,
* i est un nombre entier compris de 1 à n,
* B₀ et B₁ représentent indépendamment les uns des autres l'adénine, la guanine, la thymine, la cytosine, ou l'uracile,
* R représente l'hydrogène ou un groupement hydroxyle,
comprenant l'étape de :
réaction d'un oxydant choisi parmi les dérivés de l'iode hypervalent, notamment l'acide 2-iodoxybenzoique (IBX) et le Dess-Martin periodinane (DMP), et le réactif de Swern,
sur un composé de formule (XXI) suivante : 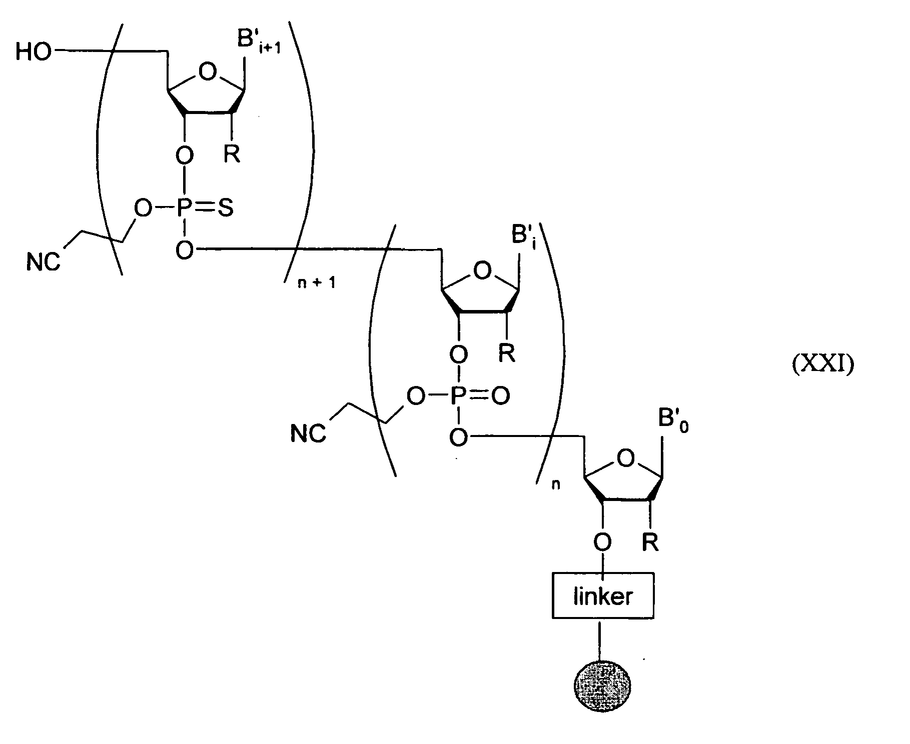
* n est un nombre entier compris de 1 à 100, notamment de 1 à 50, et en particulier de 5 à 40
* i est un nombre entier compris de 1 à n+1,
* B'₀, B'ᵢ et B'ᵢ₊₁ représentent indépendamment les uns des autres une base azotée choisie parmi l'adénine, la guanine, la cytosine, et l'uracile,
la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* R représente un groupement hydroxyle protégé par un groupe protecteur, notamment choisi parmi les groupes acétyle (Ac), silyle tels que tertbutyldiméthylsilyl (TBDMS) et triisopropylsilyloxyméthyl (TOM).
* linker représente un linker notamment choisi parmi :
- un groupe -CO-Z-CO-, dans laquelle Z représente un groupe alkyle de 1 à 2 carbones ou un groupe -CH₂-O-Ph-O-CH₂-,
- un groupe -CO-CO-,
- un groupe de formule (A) ou (B) suivante : 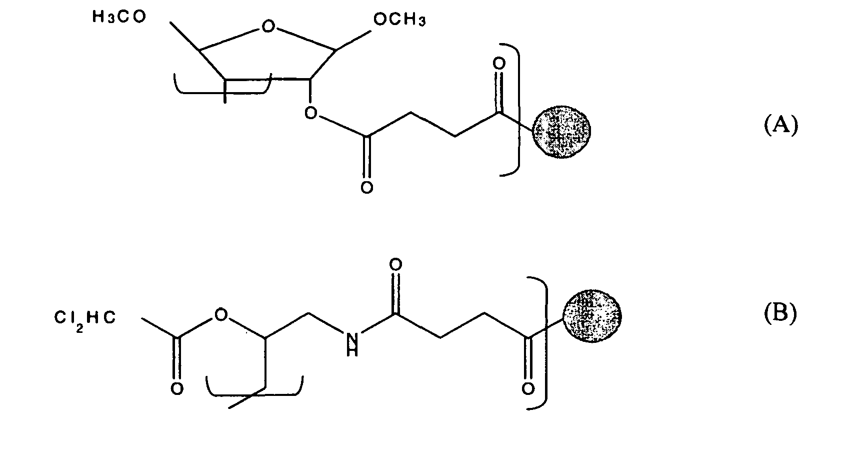
* représente un support solide notamment choisi parmi CPG (Controlled Pore Glass) avec longue chaîne aminoalkyle (lcca) de différentes porosités et les résines dérivées du polystyrène,
suivie d'une réaction en milieu basique, notamment avec de l'hydroxyde d'ammonium, de l'hydroxyde de sodium, de la méthylamine, du carbonate de potassium, ou un mélange de ces bases,
pour obtenir le dit composé de formule (V').

17. Procédé de préparation selon la revendication 12, d'un composé de formule (V) suivante : 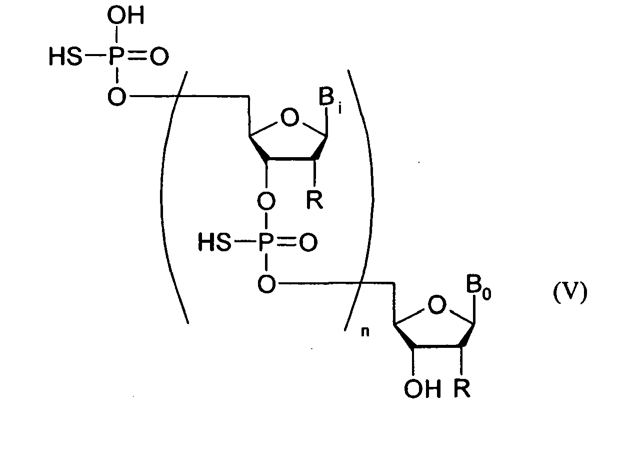 dans laquelle :
* n est un nombre entier compris de 1 à 100, notamment de 1 à 50, et en particulier de 5 à 40
* i est un nombre entier compris de 1 à n,
* B₀ et Bᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente l'hydrogène ou un groupement hydroxyle,
comprenant l'étape de :
réaction d'un oxydant choisi parmi les dérivés de l'iode hypervalent, notamment l'acide 2-iodoxybenzoique (IBX) et le Dess-Martin periodinane (DMP), et le réactif de Swern,
sur un composé de formule (XV) suivante : 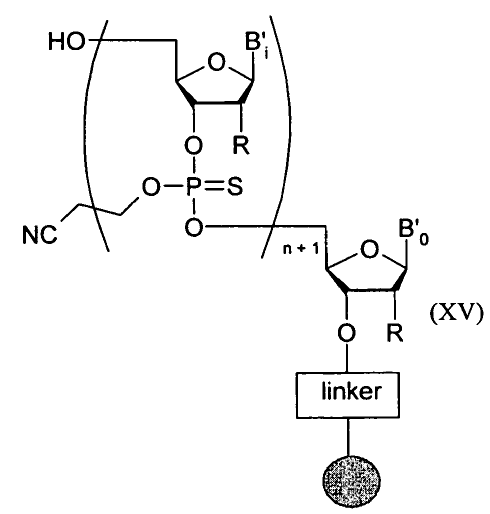
dans laquelle :
* n est un nombre entier compris de 1 à 100, notamment de 1 à 50, et en particulier de 5 à 40
* i est un nombre entier compris de 1 à n+1,
* B'₀ et B'ᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* R représente l'hydrogène ou un groupement hydroxyle,
* linker 1 notamment choisi parmi :
- un groupe -CO-Z-CO-, dans laquelle Z représente un groupe alkyle de 1 à 2 carbones ou un groupe -CH₂-O-Ph-O-CH₂-,
- un groupe -CO-CO-,
- un groupe de formule (A) ou (B) suivante : 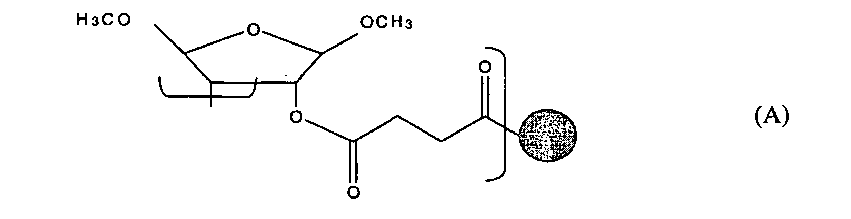 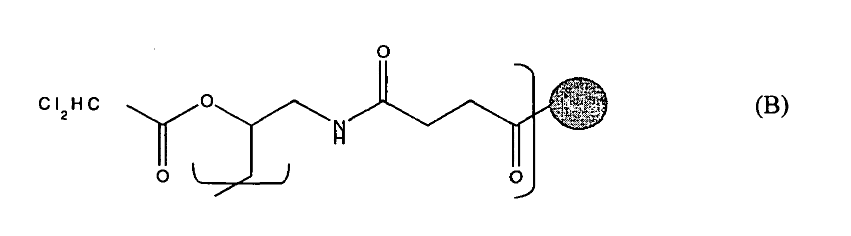
* représente un support solide notamment choisi parmi CPG (Controlled Pore Glass) avec longue chaîne aminoalkyle (Icca) de différentes porosités et les résines dérivées du polystyrène,
suivie d'une réaction en milieu basique, notamment avec de l'hydroxyde d'ammonium, de l'hydroxyde de sodium, de la méthylamine, du carbonate de potassium, ou un mélange de ces bases,
pour obtenir le dit composé de formule (V).

18. Procédé de préparation selon la revendication 12, d'un composé de formule (VI) suivante : 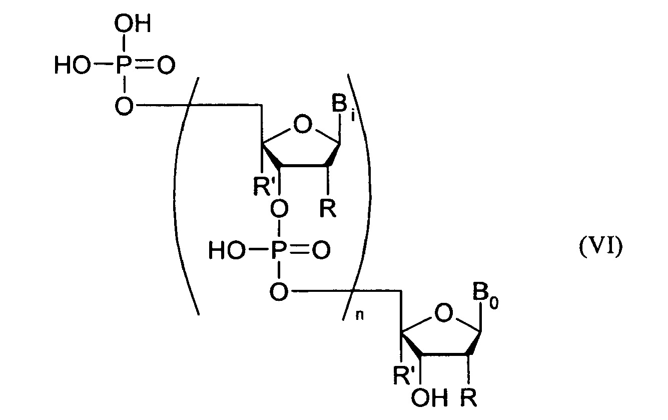 dans laquelle:
* n est un nombre entier compris de 1 à 100, notamment de 1 à 50, et en particulier de 5 à 40
* i est un nombre entier compris de 1 à n,
* B₀ et Bᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente :
- un groupement -O-alkyle comportant 1 à 4 atomes de carbones linéaire ou ramifié, ou,
- un groupement -O-(CH₂)ₘ-NH₂, dans lequel m représente un nombre entier compris de 2 à 10, ou,
- ou un atome d'oxygène,
* R' représente :
- l'hydrogène, ou,
- un groupe -(CH₂)p- dans lequel p varie de 1 à 4,
tels que lorsque R représente un atome d'oxygène, R' représente un groupe - (CH₂)p-, et lorsque R' représente un groupe -(CH₂)p-, R représente un atome d'oxygène, les groupes R et R' étant pontés et formant alors un cycle éther, et, comprenant l'étape de :
réaction d'un oxydant choisi parmi les dérivés de l'iode hypervalent, notamment l'acide 2-iodoxybenzoique (IBX) et le Dess-Martin periodinane (DMP), et le réactif de Swern,
sur un composé de formule (XVI) suivant : 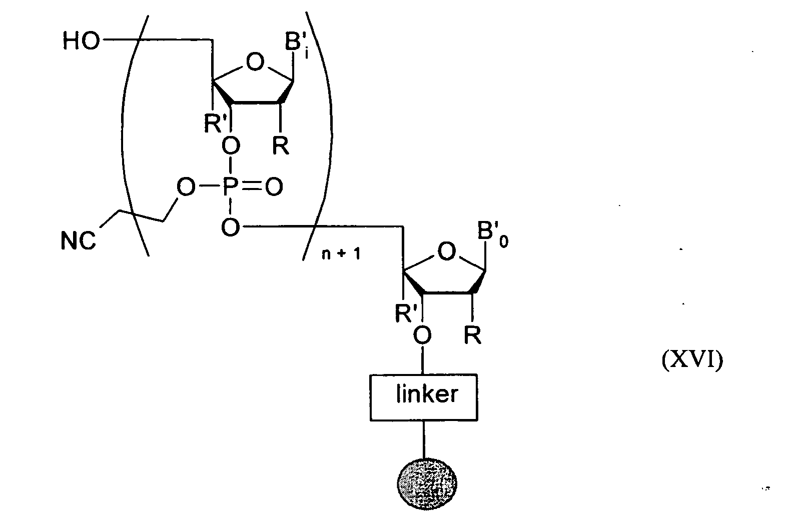 dans laquelle :
* n est un nombre entier compris de 1 à 100, notamment de 1 à 50, et en particulier de 5 à 40,
* i est un nombre entier compris de 1 à n,
* B'₀ et B'ᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* R représente:
- un groupement -O-alkyle comportant 1 à 4 atomes de carbones linéaire ou ramifié, ou
- un groupement -O-(CH_{Z})ₘ-NH₂, dans lequel m représente un nombre entier compris de 2 à 10, ou
- un atome d'oxygène,
* R' représente :
- l'hydrogène, ou,
- un groupe -(CH₂)p- dans lequel p varie de 1 à 4,
tels que lorsque R représente un atome d'oxygène, R' représente un groupe - (CH₂)p-, et lorsque R' représente un groupe -(CH₂)p-, R représente un atome d'oxygène, les groupes R et R' étant pontés et formant alors un cycle éther, sauf dans le cas de la dernière base, où R' représente l'hydrogène.
et,
* Rᵢ" représentent indépendamment un groupement hydroxyle ou thiol,
* linker 1 notamment choisi parmi :
- un groupe -CO-Z-CO-, dans laquelle Z représente un groupe alkyle de 1 à 2 carbones ou un groupe -CH₂-O-Ph-O-CH₂-,
- un groupe -CO-CO-,
- un groupe de formule (A) ou (B) suivante : 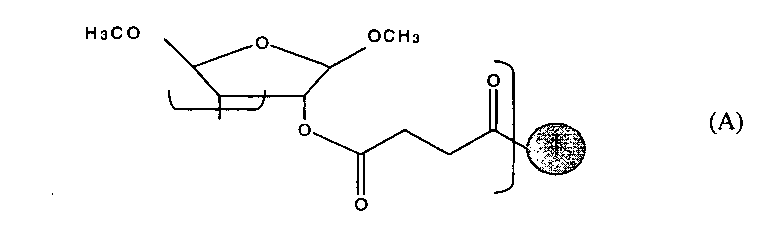 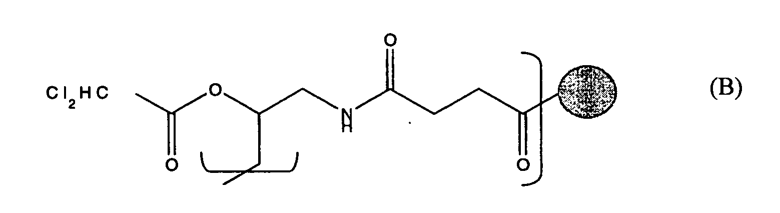
* représente un support solide notamment choisi parmi CPG (Controlled Pore Glass) avec longue chaîne aminoalkyle (Icca) de différentes porosités et les résines dérivées du polystyrène,
suivie d'une réaction en milieu basique, notamment avec de l'hydroxyde d'ammonium, de l'hydroxyde de sodium, de la méthylamine, du carbonate de potassium, ou un mélange de ces bases,
pour obtenir le dit composé de formule (VI).

19. Procédé de préparation d'un composé de formule (XX) suivante : 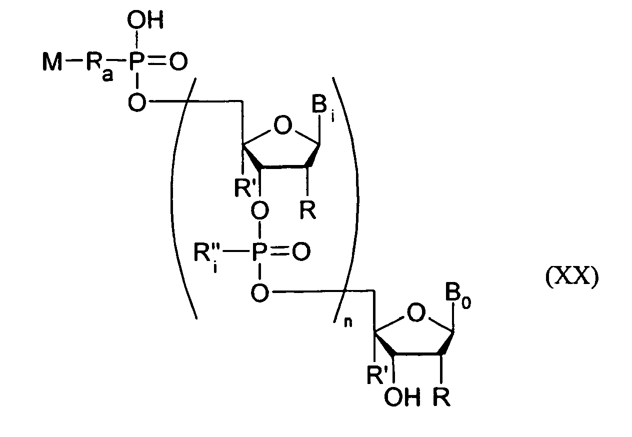 dans laquelle :
* n est un nombre entier compris de 1 à 100, notamment de 1 à 50, et en particulier de 5 à 40,
* i est un nombre entier compris de 1 à n,
* B₀ et Bᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
* R représente :
- l'hydrogène, ou,
- un groupement hydroxyle, ou
- un groupement -O-alkyle comportant 1 à 4 atomes de carbones linéaire ou ramifié, ou
- un atome d'oxygène,
* R' représente :
- l'hydrogène, ou,
- un groupe -(CH₂)p- dans lequel p varie de 1 à 4,
tels que lorsque R représente un atome d'oxygène, R' représente un groupe - (CH₂)p-, et lorsque R' représente un groupe -(CH₂)p-, R représente un atome d'oxygène, les groupes R et R' étant pontés et formant alors un cycle éther, et,
* Rᵢ" représentent indépendamment un groupement hydroxyle ou thiol,
* Ra représente un atome de soufre ou d'oxygène,
* M représente une sonde fluorescente, un peptide, un oligonucléotide ou un agent intercalant,
comprenant l'étape de :
réaction d'un oxydant choisi parmi les dérivés de l'iode hypervalent, notamment l'acide 2-iodoxybenzoique (IBX) et le Dess-Martin periodinane (DMP), et le réactif de Swern,
sur un composé de formule (XI) suivante :
dans laquelle : 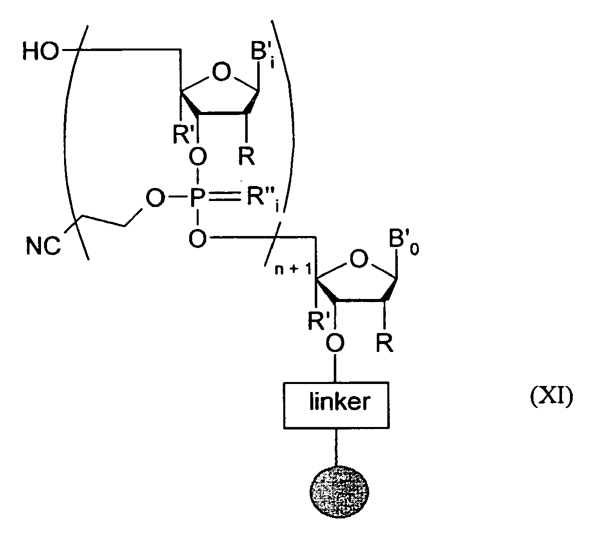
* n est un nombre entier compris de 1 à 100, notamment de 1 à 50, et en particulier de 5 à 40,
* i est un nombre entier compris de 1 à n+1,
* B'₀ et B'ᵢ représentent indépendamment les uns des autres une base azotée, notamment :
- un dérivé de purine, notamment l'adénine, la guanine, la 8-hydroxy guanine, l'inosine, ou,
- un dérivé de pyrimidine, notamment la thymine, la cytosine, l'uracile ou la 5-méthylcytosine, la 5-propynyl-uracile, la pseudo-uracile, la 4-thiothymine, la 2-thio-thymine, la 4-thio-uracile, la 5-halogéno-uracile, 5-halogéno-cytosine, la 5,6-dihydro-thymine, la 5-hydroxy-uracile, la 5-hydroxy-cytosine, la 5,6-dihydro-uracile, la 5-hydroxyméthyl-uracile, la 5,6-dihydroxy-thymine,
la dite base azotée étant protégée par au moins un groupe protecteur, notamment choisi parmi les groupes benzoyle (Bz), acétyle (Ac), isobutyryle (iBu), diméthylformamidine (dmf), phénoxyacétyl (Pac), 4-isopropyl-phénoxyacétyle (iPr-Pac),
* R représente :
- l'hydrogène, ou,
- un groupement hydroxyle, ou
- un groupement -O-alkyle comportant 1 à 4 atomes de carbones linéaire ou ramifié, ou
- un atome d'oxygène,
* R' représente :
- l'hydrogène, ou,
- un groupe -(CH₂)p- dans lequel p varie de 1 à 4,
tels que lorsque R représente un atome d'oxygène, R' représente un groupe - (CH₂)ₚ-, et lorsque R' représente un groupe -(CH₂)ₚ-, R représente un atome d'oxygène, les groupes R et R' étant pontés et formant alors un cycle éther, sauf dans le cas de la dernière base, où R' représente l'hydrogène.
et,
Rᵢ" représentent indépendamment un groupement hydroxyle ou thiol,
* linker représente un linker notamment choisi parmi :
- un groupe -CO-Z-CO-, dans laquelle Z représente un groupe alkyle de 1 à 2 carbones ou un groupe -CH₂-O-Ph-O-CH₂
- un groupe -CO-CO-,
- un groupe de formule (A) ou (B) suivante : 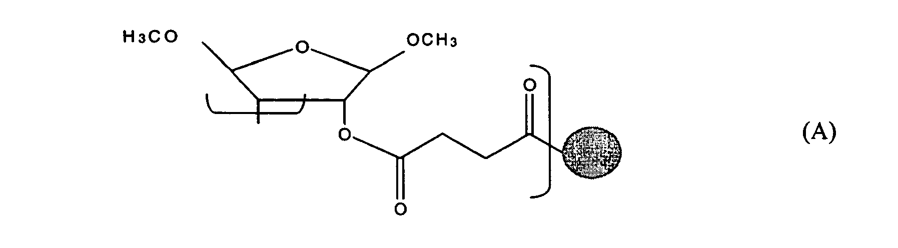 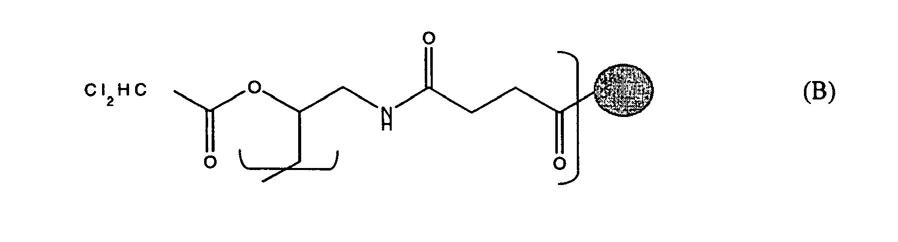
* 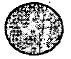 représente un support solide, notamment choisi parmi CPG (Controlled Pore Glass) avec longue chaîne aminoalkyle (Icca) de différentes porosités et les résines dérivées du polystyrène,
suivie d'une réaction avec une molécule M-GP, dans laquelle M représente une sonde fluorescente, un peptide, un oligonucléotide ou un agent intercalant, et GP représente un groupe partant, notamment choisi parmi le chlore, le brome, l'iode, les groupes tosylate, mésylate, lorsque Ra est un soufre,
ou avec une molécule M-OH ou M-NH₂ après activation du phosphate en 5' lorsque Ra est un oxygène.
et d'une réaction en milieu basique, notamment avec de l'hydroxyde d'ammonium, de l'hydroxyde de sodium, de la méthylamine, du carbonate de potassium, ou un mélange de ces bases,
les dites réactions avec une molécule M-GP, M-OH ou M-NH₂ et en milieu basique pouvant être effectuées successivement ou en une seule étape « one-pot »,
pour obtenir le dit composé de formule (XX).

20. Procédé selon l'une quelconque des revendications 12 à 19, dans lequel l'oxydant est l'IBX.

## Claims

1. Use of at least one oxidant in particular chosen from:
- the hypervalent iodine derivatives, in particular 2-iodoxybenzoic acid (IBX) and Dess-Martin periodinane (DMP), and
- Swern's reagent,
with a modified or non-modified oligonucleotide comprising a 5'-OH end, for the synthesis of modified or non-modified oligonucleotides, in particular oligoribonucleotides, oligodeoxyribonucleotides, said oligonucleotides comprising a 5'-phosphate monoester or 5'-thiophosphate monoester end.

2. Use according to claim 1, of a modified or non-modified oligonucleotide,
- comprising a 5'-OH end, and
- containing (n+1) nucleotides,
in order to obtain respectively a modified or non-modified oligonucleotide,
- comprising a 5'-phosphate monoester or 5'-thiophosphate monoester end, and
- containing n nucleotides.

3. Use according to any one of claims 1 or 2, in which the synthesis is a solid phase synthesis.

4. Use according to any one of claims 1 to 3, for the synthesis of a compound of formula (I) below: 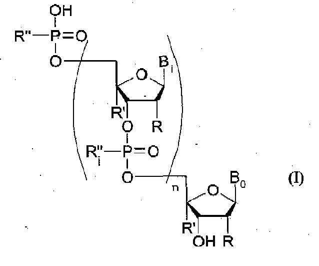 in which:
* n is an integer comprised from 1 to 100, in particular 1 to 50, and in particular 5 to 40,
* i is an integer comprised from 1 to n,
* B₀ and Bᵢ represent independently of each other a nitrogenous base, in particular:
- a purine derivative, in particular adenine, guanine, 8-hydroxy guanine, 8-hydroxy-adenine, 8-amino-guanine, 8-methoxy-guanine, 8-bromo-guanine, 8-bromo-adenine, inosine, or
- a pyrimidine derivative, in particular thymine, cytosine, uracil or 5-methylcytosine, 5-propynyl-uracil, pseudo-uracil, 4-thiothymine, 2-thiothymine, 4-thio-uracil, 5-halogeno-uracil, 5-halogeno-cytosine, 5,6-dihydro-thymine, 5-hydroxy-uracil, 5-hydroxy-cytosine, 5,6-dihydro-uracil, 5-hydroxymethyl-uracil, 5,6-dihydroxy-thymine,
* R represents:
- hydrogen, or
- a hydroxyl group, or
- a linear or branched -O-alkyl group comprising 1 to 4 carbon atoms, or
- an -O-(CH₂)ₘ-NH₂ group, in which m represents an integer comprised from 2 to 10, or
- an oxygen atom,
* R' represents:
- hydrogen, or
- a -(CH₂)ₚ- group in which p varies from 1 to 4,
such that when R represents an oxygen atom, R' represents a -(CH₂)ₚ- group, and when R' represents a -(CH₂)ₚ- group, R represents an oxygen atom, the R and R' groups being bridged and then forming an ether ring, and
* R" and Rᵢ" represent independently a hydroxyl (OH) or mercapto (SH) group.

5. Use according to claim 4, for the synthesis of a compound of formula (II) below:
in which: 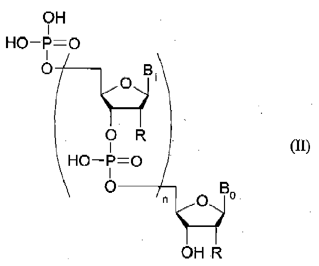
* n is an integer comprised from 1 to 100, in particular 1 to 50, and in particular 5 to 40,
* i is an integer comprised from I to n,
* B₀ and Bᵢ represent independently of each other adenine, guanine, thymine, cytosine, or uracil,
* R represents hydrogen or a hydroxyl group.

6. Use according to any one of claims 4 and 5, for the synthesis of a compound of formula (III) below: 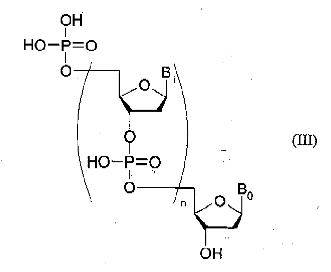 in which:
* n is an integer comprised from 1 to 100, in particular 1 to 50, and in particular 5 to 40,
* i is an integer comprised from 1 to n,
* B₀ and Bᵢ represent independently of each other adenine, guanine, thymine, or cytosine.

7. Use according to any one of claims 4 to 5, for the synthesis of a compound of formula (IV) below: 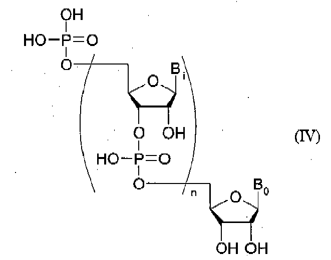 in which:
* n is an integer comprised from 1 to 100, in particular 1 to 50, and in particular 5 to 40,
* i is an integer comprised from 1 to n,
* B₀ and Bᵢ represent independently of each other adenine, guanine, cytosine, or uracil.

8. Use according to claim 4, for the synthesis of a compound of formula (V') below: 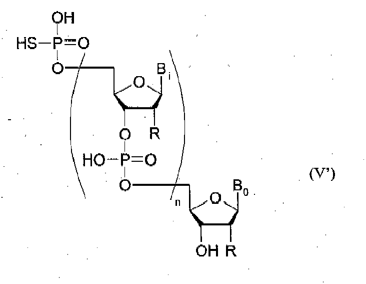 in which:
* n is an integer comprised from 1 to 100, in particular 1 to 50, and in particular 5 to 40,
* i is an integer comprised from 1 to n,
* B₀ and Bᵢ represent independently of each other adenine, guanine, cytosine, or uracil.
* R represents hydrogen or a hydroxyl group.

9. Use according to claim 4, for the synthesis of a compound of formula (V) below:
in which: 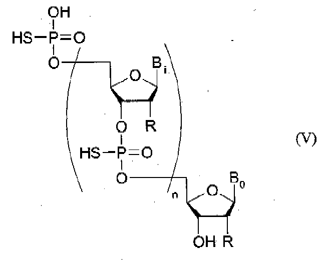
* n is an integer comprised from 1 to 100, in particular 1 to 50, and in particular 5 to 40,
* i is an integer comprised from 1 to n,
* B₀ and Bᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methylcytosine, 5-propynyl-uracil, pseudo-uracil, 4-thio-thymine, 2-thiothymine, 4-thio-uracil, 5-halogeno-uracit, 5-halogeno-cytosine, 5,6-dihydro-thymine, 5-hydroxy-uracil, 5-hydroxy-eytosine, 5,6-dihydro-uracil, 5-hydroxymethyl-uracil, 5,6-dihydroxy-thymine,
* R represents hydrogen or a hydroxyl group.

10. Use according to claim 4, for the synthesis of a compound of formula (VI) below: 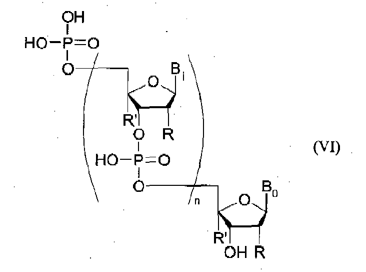 in which:
* n is an integer comprised from I to 100, in particular 1 to 50, and in particular 5 to 40,
* i is an integer comprised from 1 to n,
* B₀ and Bᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methylcytosine, 5-propynyl-uracil, pseudo-uracil, ₄-thio-thymine. 2-thiothymine, 4-thio-uracil, 5-halogeno-uracil, 5-halogeno-cytosine, 5,6-dihydro-thymine, 5-hydroxy-uracil, 5-hydroxy-cytosine, 5,6-dihydro-uracil, 5-hydroxymethyl-uracil, 5,6-dihydroxy-thymine,
* R represents:
- a linear or branched -O-alkyl group comprising I to 4 carbon atoms, or
- an -0-(CH₂)ₘ-NH₂ group, in which m represents an integer comprised from 2 to 10, or
- an oxygen atom,
* R' represents:
- hydrogen, or
- a -(CH₂)ₚ- group in which p varies from 1 to 4,
such that when R represents an oxygen atom, R' represents a -(CH₂)p- group, and when R' represents a -(CH₂)ₚ- group, R represents an oxygen atom, the R and R' groups being bridged and then forming an ether ring.

11. Use according to any one of claims 1 to 10, in which the oxidant is IBX.

12. Process for preparing a compound as defined in claim 1, of formula (I) below: 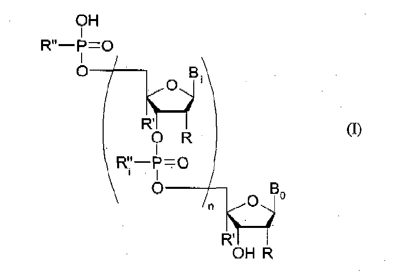 in which:
* n is an integer comprised from 1 to 100, in particular 1 to 50, and in particular 5 to 40,
* i is an integer comprised from 1 to n,
* B₀ and Bᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methylcytosine, 5-propynyt-uracil, pseudo-uracil, 4-thio-thyanine, 2-thiothymine, 4-thio-uracil, 5-halogeno-uracil, 5-halogeno-cytosine, 5,6-dihydro-thymine, 5-hydroxy-uracil, 5-hydroxy-cytosine, 5,6-dihydro-uracil, 5-hydroxymethyl-uracil, 5,6-dihydroxy-thymine,
R represents:
- hydrogen, or
- a hydroxyl group, or
- a linear or branched -O-alkyl group comprising 1 to 4 carbon atoms, or
- an -0-(CH₂)ₘ-NH₂ goup, in which m represents an integer comprised from 2 to 10, or
- an oxygen atom,
* R' represents:
- hydrogen, or
- a -(CH₂)ₚ- group in which p varies from 1 to 4,
such that when R represents an oxygen atom, R' represents a -(CH₂)ₚ- group, and when R' represents a -(CH₂)ₚ- group, R represents an oxygen atom, the R and R' groups being bridged and then forming an ether ring, and
* R" and Rᵢ" represent independently a hydroxyl or thiol goup,
comprising the stage of:
r eaction of an oxidant chosen from the hypervalent iodine derivatives, in particular 2-iodoxybenzoic acid (IBX) and Dess-Martin periodinane (DMP), and Swern's reagent, on a compound of formula (XI) below: 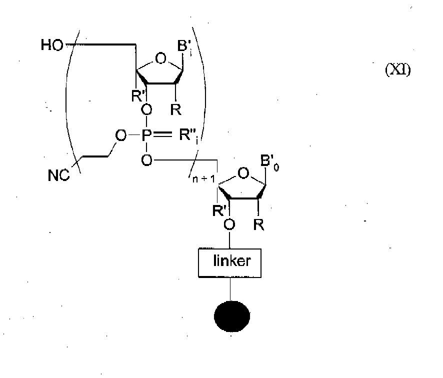
in which:
* n is an integer comprised from 1 to 100, in particular 1 to 50, and in particular 5 to 40,
* i is an integer comprised from 1 to n+1, 1,
* H'₀ and B'ᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methylcytosine, 5-propynyl-uracil, pseudo-uracil, 4-thio-thymine, 2-triothymine, 4-thio-uracil, 5-halogeno-uracil, 5-halogeno-cytosine, 5,6-dihydro-thymine, 5-hydroxy-uracil, 5-hydroxy-cytosine, 5,6-dihydro-uracil, 5-hydroxymethyl-uracil, 5,6dihydroxy-thymine,
said nitrogenous base being protected by at least one protective group, in particular chosen from the benzoyl (Bz), acetyl (Ac), isobutyryl (iBu), dimethylfotmamidine (dmf), phenoxyacetyl (Pac), 4-isopropyl-phenoxyacetyl (iPr-Pac) groups,
* R represents:
- hydrogen, or
- a hydroxyl group protected by a protective group, in particular chosen from the acetyl (Ac), silyl such as tertbutyldimethylsilyl (TBDMS), triisopropylsilyloxymethyl (TOM) groups,
- a linear or branched -O-alkyl group comprising 1 to 4 carbon atoms, or
- an -O-(CH₂)ₘ-NH₂ group, in which m represents an integer comprised from 2 to 10, and in which the amine function is protected by a protective group, in particular chosen from the carbamates such as the fluorenylmethyloxycarbonyl (Fmoc) group, the amides such as trifluoroacetamide (CO-CF3), and phtalimide,
or
- an oxygen atom,
* R' represents:
- hydrogen or,
- a -(CH₂)ₚ- group in which p varies from 1 to 4,
except in the case of the last base, where R' represents hydrogen.
such that when R represents an oxygen atom, R' represents a -(CH₂)p- group, and when R' represents a -(CH₂)ₚ- group, R represents an oxygen atom, the R and R' groups being bridged and then forming an ether ring, and
* Rᵢ" represents independently an oxygen or sulphur atom,
* linker represents a linker in particular chosen from:
- a -CO-Z-CO- group, in which Z represents an alkyl group with 1 to 2 carbon atoms or a -CH₂-O-Ph-O-CH₂- group,
- a -CO-CO- group,
- a group of formula (A) or (B) below: 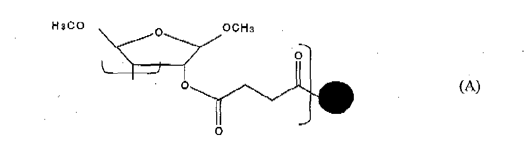 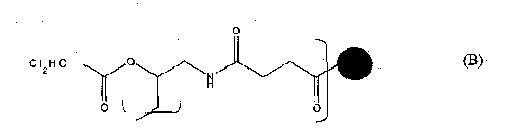
* 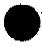 represents a solid support, in particular chosen from CPG (Controlled Pore Glass) with long chain aminoalkyl (Icaa) of different porosities and the resins derived from polystyrene,
followed by a reaction in basic medium, in particular with ammonium hydroxide, sodium hydroxide, methylamine, potassium carbonate, or a mixture of these bases,
in order to obtain said compound of formula (I).

13. Process according to claim 12, for preparing a compound of formula (II) below: 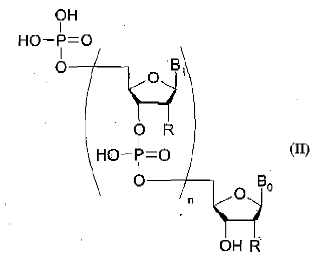 in which:
* n is an integer comprised from 1 to 100, in particular 1 to 50, and in particular 5 to 40
* i is an integer comprised from 1 to n,
* B₀ and Bᵢ represent independently of each other adenine, guanine, thymine, cytosine, or uracil,
* R represents hydrogen or a hydroxyl goup,
comprising the stage of:
r eaction of an oxidant chosen from the hypervalent iodine derivatives, in particular 2-iodoxybenzoic acid (IBX) and Dess-Martin peziodinane (DMP), and Swern's reagent, on a compound of formula (XII) below: 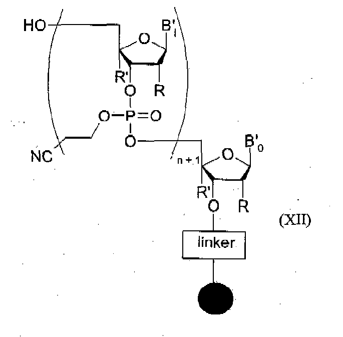
in which:
* n is an integer comprised from 1 to 100, in particular 1 to 50, and in particular 5 to 40,
* i is an integer comprised from 1 to n+1,
* B'₀ and B'ᵢ represent independently of each other a nitrogenous base chosen from adenine, guanine, thymine, cytosine, and uracil,
said nitrogenous base being protected by at least one protective group, in particular chosen from the benzoyl (Bz), acetyl (Ac), isobutyryl (iBu), dimethylformamidine (dmf), phenoxyacetyl (Pac), 4-isopropyl-phenoxyacetyl (iPr-Pac) groups,
* R represents hydrogen or a hydroxyl group,
* R' represents hydrogen
* linker represents a linker in particular chosen from:
- a -CO-Z-CO- group, in which Z represents an alkyl group with 1 to 2 carbon atoms or a -CH₂-O-Ph-O-CH₂- group,
- a -CO-CO- group,
- a group of formula (A) or (B) below: 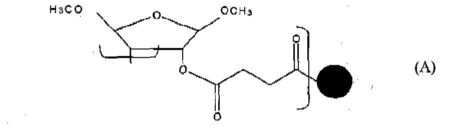 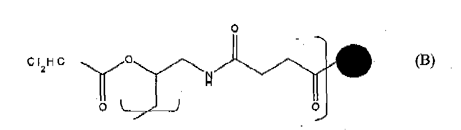
* 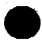 represents a solid support in particular chosen from CPG (Controlled Pore Glass) with long chain aminoalkyl (Icaa) of different porosities and the resins derived from polystyrene,
followed by a reaction in basic medium, in particular with ammonium hydroxide, sodium hydroxide, methylamine, potassium carbonate, or a mixture of these bases,
in order to obtain said compound of formula (II).

14. Process according to any one of claims 12 and 13 for preparing a compound of formula (III) below:
in which: 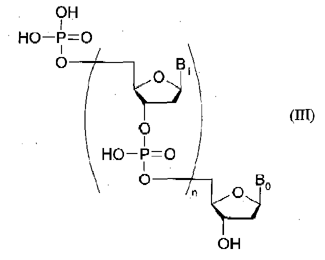
* n is an integer comprised from 1 to 100, in particular 1 to 50, and in particular 5 to 40,
* i is an integer comprised from 1 to n,
* B₀ and Bᵢ represent independently of each other adenine, guanine, thymine, or cytosine,
comprising the stage of:
r eaction of an oxidant chosen from the hypervalent iodine derivatives, in particular 2-iodoxybenzoic acid (IBX) and Dess-Martin periodinane (DMP), and Swern's reagent, on a compound of formula (XIII) below: 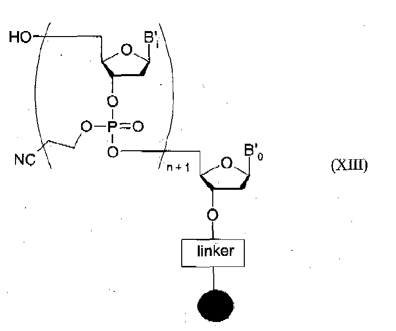
in which:
* n is an integer comprised from 1 to 100, in particular 1 to 50, and in particular 5 to 40,
* i is an integer comprised from 1 to n+1,
* B'₀ and B'ᵢ represent independently of each other a nitrogenous base chosen from adenine, guanine, thymine, and cytosine,
said nitrogenous base being protected by at least one protective group, in particular chosen from the benzoyl (Bz), acetyl (Ac), isobutyryl (iBu), dimethylformamidine (dmf), phenoxyacetyl (Pac), 4-isopropyl-phenoxyacetyl (iPr-Pac) groups,
* linker represents a linker in particular chosen from:
- a -CO-Z-CO- group, in which Z represents an alkyl group with I to 2 carbon atoms or a -CH₂-O-Ph-O-CH₂- group,
- a -CO-CO- group,
- a group of formula (A) or (B) below: 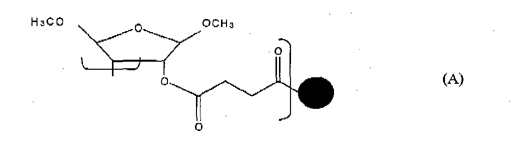 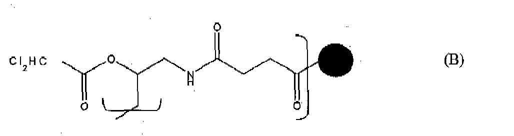
* 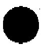 represents a solid support in particular chosen from CPG (Controlled Pore Glass) with long chain aminoalkyl (Icaa) of different porosities and the resins derived from polystyrene,
followed by a reaction in basic medium, in particular with ammonium hydroxide, sodium hydroxide, methylamine, potassium carbonate, or a mixture of these bases,
in order to obtain said compound of formula (III).

15. Process according to any one of claims 12 and 13 for preparing a compound of formula (IV) below: 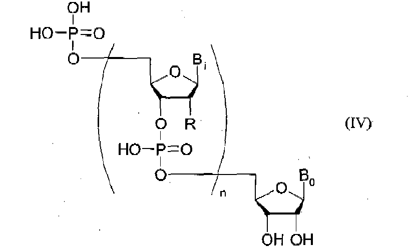 in which:
* n is an integer comprised from 1 to 100, in particular 1 to 50, and in particular 5 to 40
* i is an integer comprised from 1 to n,
* B₀ and Bᵢ represent independently of each other adenine, guanine, cytosine, or uracil,
comprising the stage of:
r eaction of an oxidant chosen from the hypervalent iodine derivatives, in particular 2-iodoxybenzoic acid (IBX) and Dess-Martin periodinane (DMP), and Swern's reagent, on a compound of formula (XIV) below: 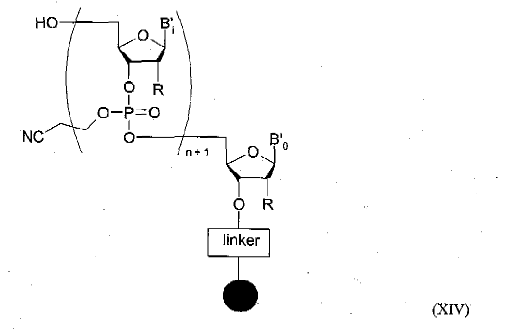 in which:
* n is an integer comprised from 1 to 100, in particular 1 to 50, and in particular 5 to 40
* i is an integer comprised from 1 to n+1,
* B'₀ and B'ᵢ represent independently of each other a nitrogenous base chosen from adenine, guanine, cytosine, and uracil,
said nitrogenous base being protected by at least one protective group, in particular chosen from the benzoyl (Bz), acetyl (Ac), isobutyryl (iBu), dimethylformamidine (dmf), phenoxyacetyl (Pac), 4-isopropyl-phenoxyacetyl (iPr-Pac) groups,
* linker represents a linker in particular chosen from:
- a -CO-Z-CO- group, in which Z represents an alkyl group with 1 to 2 carbon atoms or a -CH₂-O-Ph-O-CH₂- group,
- a -CO-CO- group,
a group of formula (A) or (B) below: 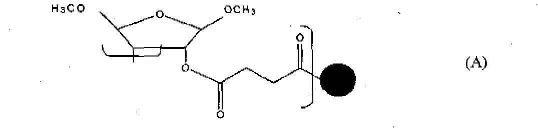 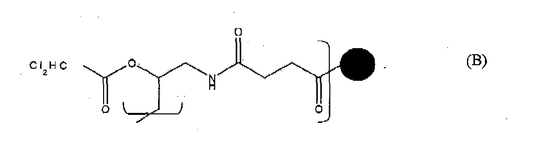
* 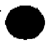 represents a solid support in particular chosen from CPG (Controlled Pore Glass) with long chain aminoalkyl (Icaa) of different porosities and the resins derived from polystyrene,
followed by a reaction in basic medium, in particular with ammonium hydroxide, sodium hydroxide, methylamine, potassium carbonate, or a mixture of these bases,
in order to obtain said compound of formula (IV).

16. Process according to claim 12 for preparing a compound of formula (V') below: 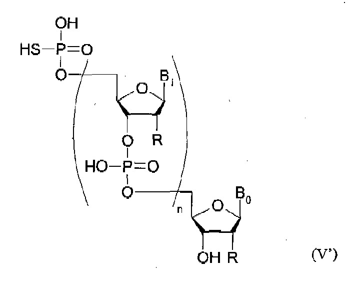 in which:
* n is an integer comprised from 1 to 100, in particular 1 to 50, and in particular 5 to 40,
* i is an integer comprised from 1 to n,
* B₀ and Bᵢ represent independently of each other adenine, guanine, thymine, cytosine, or uracil,
* R represents hydrogen or a hydroxyl group,
comprising the stage of:
r eaction of an oxidant chosen from the hypervalent iodine derivatives, in particular 2-iodoxybenzoic acid (IBX) and Dess-Martin periodinane (DMP), and Swern's reagent, on a compound of formula (XXI) below: 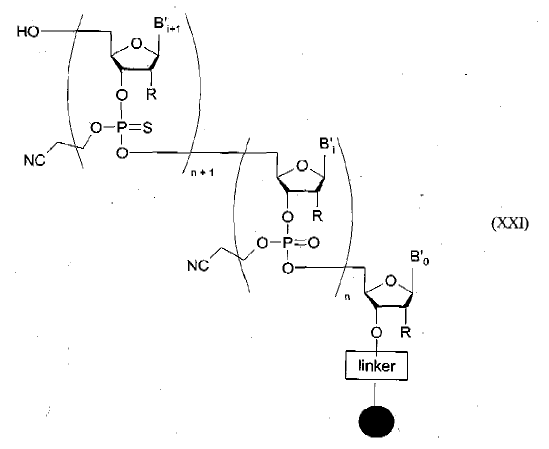
* n is an integer comprised from 1 to 100, in particular 1 to 50, and in particular 5 to 40
* i is an integer comprised from 1 to n+1,
* B'₀, B'ᵢ and B'ᵢ₋₁ represent independently of each other a nitrogenous base chosen from adenine, guanine, cytosine, and uracil,
said nitrogenous base being protected by at least one protective group, in particular chosen from the benzoyl (Bz), acetyl (Ac), isobutyryl (iBu), dimethylfonnamidine (dmf), phenoxyacetyl (Pac), 4-isopropyl-phenoxyacetyl (iPr-Pac) goups,
* R represents a hydroxyl group protected by a protective group, in particular chosen from the acetyl (Ac), silyl such as tertbutyldimethylsilyl (TBDMS) and triisopropylsilyloxymethyl (TOM) groups.
* linker represents a linker in particular chosen from:
- a -CO-Z-CO- group, in which Z represents an alkyl group with 1 to 2 carbon atoms or a -CH₂-O-Ph-O-CH₂- group,
- a -CO-CO- group,
- a group of formula (A) or (B) below: 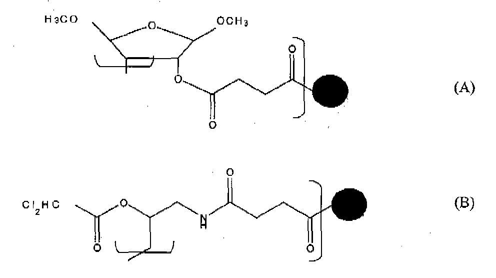
* 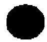 represents a solid support in particular chosen from CPG (Controlled Pore Glass) with long chain aminoalkyl (Icaa) of different porosities and the resins derived from polystyrene,
followed by a reaction in basic medium, in particular with ammonium hydroxide, sodium hydroxide, methylamine, potassium carbonate, or a mixture of these bases, in order to obtain said compound of formula (V').

17. Process according to claim 12 for preparing a compound of formula (V) below: 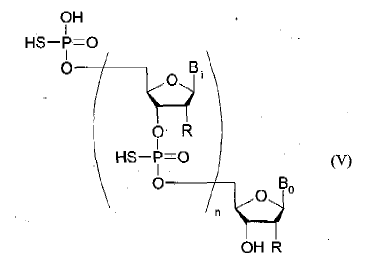 in which:
* n is an integer comprised from 1 to 100, in particular 1 to 50, and in particular 5 to 40
* i is an integer comprised from 1 to n,
* B₀ and Bᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methylcytosine, 5-propynyl-uracil, pseudo-uracil, 4-thio-thymine, 2-thiothymine, 4-thio-uracil, 5-halogeno-uracil, 5-halogeno-cytosine, 5,6-dihydro-thymine, 5-hydroxy-uracil, 5-hydroxy-cytosine, 5,6-dihydro-uracil, 5-hydroxymethyl-uracil, 5,6-dihydroxy-thymine,
* R represents hydrogen or a hydroxyl group,
comprising the stage of:
reaction of an oxidant chosen from the hypervalent iodine derivatives, in particular 2-iodoxybenzoic acid (IBX) and Dess-Martin periodinane (DMP), and Swern's reagent,
on a compound of formula (XV) below: 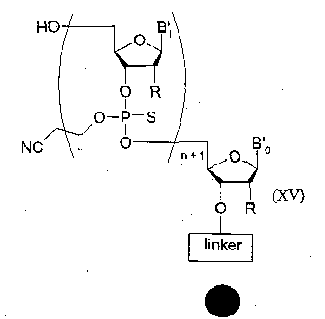
in which:
* n is an integer comprised from 1 to 100, in particular 1 to 50, and in particular 5 to 40
* i is an integer comprised from 1 to n+1,
* B'₀ and B'ᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methylcytosine, 5-propynyl-uracil, pseudo-uracil, 4-thio-thymine, 2-thiothymine, 4-thio-uracil, 5-halogeno-uracil, 5-halogeno-cytosine, 5,6-dihydro-thymine, 5-hydroxy-uracil, 5-hydroxy-cytosine, 5,6-dihydro-uracil, 5-hydroxymethyl-uracil, 5,6-dihydroxy-thymine,
said nitrogenous base being protected by at least one protective group, in particular chosen from the benzoyl (Bz), acetyl (Ac), isobutyryl (iBu), dimethylformamidine (dmf), phenoxyacetyl (Pac), 4-isopropyl-phenoxyacetyl (iPr-Pac) groups,
* R represents hydrogen or a hydroxyl group,
* linker in particular chosen from:
- a -CO-Z-CO- group, in which Z represents an alkyl group with 1 to 2 carbon atoms or a -CH₂-O-Ph-O-CH₂- group,
- a -CO-CO- group,
- a group of formula (A) or (B) below: 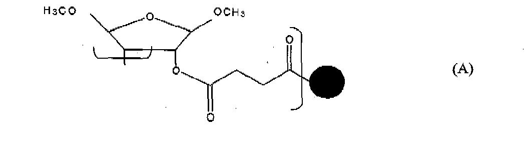 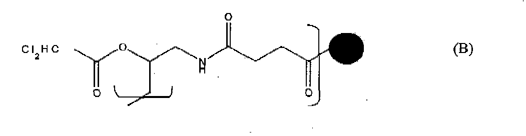
* 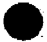 represents a solid support in particular chosen from CPG (Controlled Pore Glass) with long chain aminoalkyl (Icaa) of different porosities and the resins derived from polystyrene,
followed by a reaction in basic medium, in particular with ammonium hydroxide, sodium hydroxide, methylamine, potassium carbonate, or a mixture of these bases,
in order to obtain said compound of formula (V).

18. Process according to claim 12 for preparing a compound of formula (VI) below: 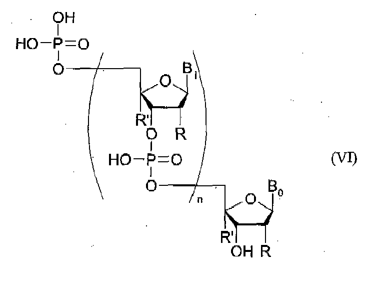 in which:
* n is an integer comprised from 1 to 100, in particular 1 to 50, and in particular 5 to 40
* i is an integer comprised from 1 to n,
* B₀ and Bᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methylcytosine, 5-propynyl-uracil, pseudo-uracil, 4-thio-thymine, 2-thiothymine, 4-thio-uracil, 5-halogeno-uracil, 5-halogeno-cytosine, 5,6-dihydro-thymine, 5-hydroxy-uracil, 5-hydroxy-cytosine, 5,6-dihydro-uracil, 5-hydroxymethyl-uracil, 5,6-dihydroxy-thymine,
* R represents:
- a linear or branched -O-alkyl group comprising 1 to 4 carbon atoms, or
- a -O-(CH₂)ₘ-NH₂ group, in which m represents an integer comprised from 2 to 10, or
- an oxygen atom,
* R' represents:
- hydrogen, or
- a -(CH₂)ₚ- group in which p varies from 1 to 4,
such that when R represents an oxygen atom, R' represents a -(CH₂)ₚ- group, and when R' represents a -(CH₂)ₚ- group, R represents an oxygen atom, the R and R' groups being bridged and then forming an ether ring, and
comprising the stage of:
reaction of an oxidant chosen from the hypervalent iodine derivatives, in particular 2-iodoxybenzoic acid (IBX) and Dess-Martin periodinane (DMP), and Swern's reagent,
on a compound of formula (XVI) below: 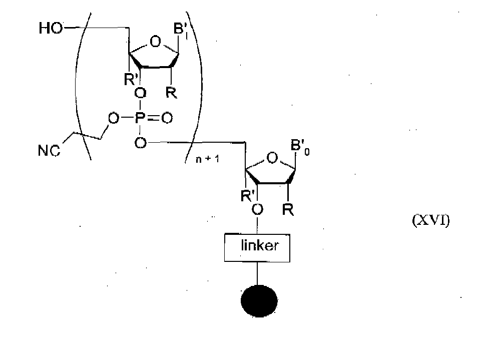
in which:
* n is an integer comprised from 1 to 100, in particular 1 to 50, and in particular 5 to 40,
* i is an integer comprised from 1 to n,
* B'₀ and B'ᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methyloytosine, 5-propynyl-uracil, pseudo-uracil, 4-thio-thymine, 2-thiothymine, 4-thio-uracil, 5-halogeno-uracil, 5-halogeno-cytosine, 5,6-dihydro-thymine, 5-hydroxy-uracil, 5-hydroxy-cytosine, 5,6-dihydro-uracil, 5-hydroxymethyl-uracil, 5,6-dihydroxy-thymine,
said nitrogenous base being protected by at least one protective group, in particular chosen from the benzoyl (Bz), acetyl (Ac), isobutyryl (iBu), dimethylformamidine (dmf), phenoxyacetyl (Pac), 4-isopropyl-phenoxyacetyl (iPr-Pac) groups,
* R represents:
- a linear or branched -O-alkyl group comprising 1 to 4 carbon atoms, or
- an -O-(CH₂)ₘ-NH₂ group, in which m represents an integer comprised from 2 to 10, or
- an oxygen atom,
* R' represents:
- hydrogen, or
- a -(CH₂)ₚ- group in which p varies from 1 to 4,
such that when R represents an oxygen atom, R' represents a -(CH₂)ₚ- group, and when R' represents a group -(CH₂)ₚ- , R represents an oxygen atom, the R and R' groups being bridged and then forming an ether ring, except in the case of the last base, where R' represents hydrogen.
and,
* Rᵢ" represents independently a hydroxyl or thiol group,
* linker in particular chosen from:
- a -CO-Z-CO- group, in which Z represents an alkyl group with 1 to 2 carbon atoms or a -CH₂-O-Ph-O-CH₂- group,
- a -CO-CO- group,
- a group of formula (A) or (B) below: 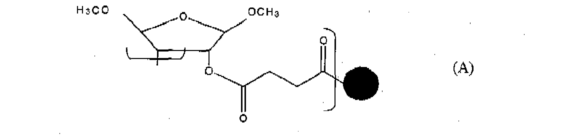 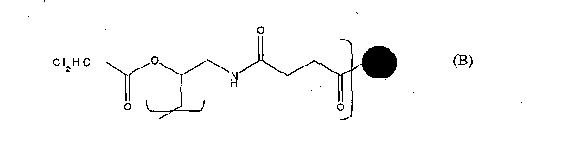
* 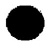 represents a solid support in particular chosen from CPG (Controlled Pore Glass) with long chain aminoalkyl (Icaa) of different porosities and the resins derived from polystyrene,
followed by a reaction in basic medium, in particular with ammonium hydroxide, sodium hydroxide, methylamine, potassium carbonate, or a mixture of these bases, in order to obtain said compound of formula (VI).

19. Process for preparing a compound of formula (XX) below: 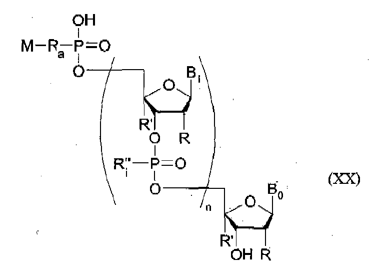 in which:
* n is an integer comprised from 1 to 100, in particular 1 to 50, and in particular 5 to 40,
* i is an integer comprised from 1 to n,
* B₀ and Bᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methylcytosine, 5-propynyl-uracil, pseudo-uracil, 4-thio-thymine, 2-thiothymine, 4-thio-uracil, 5-halogeno-uracil, 5-halogeno-cytosine, 5,6-dihydtothymine, 5-hydroxy-uracil, 5-hydroxy-cytosine, 5,6-dihydro-uracil, 5-hydroxymethyl-uracil, 5,6-dihydroxy-thymine,
* R represents:
- hydrogen, or
- a hydroxyl group, or
- a linear or branched -O-alkyl group comprising 1 to 4 carbon atoms, or
- an oxygen atom,
* R' represents:
- hydrogen, or
- a -(CH₂)ₚ- group in which p varies from 1 to 4,
such that when R represents an oxygen atom, R' represents a -(CH₂)ₚ- group, and when R' represents a -(CH₂)ₚ- group, R represents an oxygen atom, the R and R' groups being bridged and then forming an ether ring, and
* Rᵢ" represents independently a hydroxyl or thiol group,
* Ra represents a sulphur or oxygen atom,
* M represents a fluorescent probe, a peptide, an oligonucleotide or an intercalating agent,
comprising the stage of:
r eaction of an oxidant chosen from the hypervalent iodine derivatives, in particular 2-iodoxybenzoic acid (IBX) and Dess-Martin periodinane (DMP), and Swern's reagent, on a compound of formula (XI) below:
in which: 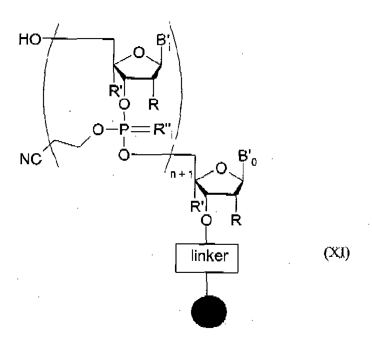
* n is an integer comprised from 1 to 100, in particular 1 to 50, and in particular 5 to 40,
* i is an integer comprised from 1 to n+1,
* B'₀ and B'ᵢ represent independently of each other a nitrogenous base, in particular:
- a purine derivative, in particular adenine, guanine, 8-hydroxy guanine, inosine, or
- a pyrimidine derivative, in particular thymine, cytosine, uracil or 5-methylcytosine, 5-propynyl-uracil, pseudo-uracil, 4-thio-thymine, 2-thiothymine, 4-thio-uracil, 5-halogeno-uracil, 5-halogeno-cytosine, 5,6-dihydro-thymine, 5-hydroxy-uracil, 5-hydroxy-cytosine, 5,6-dihydro-uracil, 5-hydroxymethyl-uracil, 5,6-dihydroxy-thymine,
said nitrogenous base being protected by at least one protective group, in particular chosen from the benzoyl (Bz), acetyl (Ac), isobutyryl (iBu), dimethylformamidine (dmf), phenoxyacetyl (Pac), 4-isopropyl-phenoxyacetyl (iPr-Pac) groups,
* R represents:
- hydrogen,or
- a hydroxyl group, or
- a linear or branched -O-alkyl goup comprising 1 to 4 carbon atoms, or
- an oxygen atom,
* R' represents:
- hydrogen, or
- a -(CH₂)ₚ- group in which p varies from 1 to 4,
such that when R represents an oxygen atoms, R' represents a -(CH₂)ₚ- group, and when R' represents a -(CH₂)ₚ- group, R represents an oxygen atom, the R and R' groups being bridged and then forming an ether ring, except in the case of the last base, where R' represents hydrogen.
and,
* Ri" represents independently a hydroxyl or thiol group,
* linker represents a linker in particular chosen from:
- a -CO-Z-CO- group, in which Z represents an alkyl group with I to 2 carbon atoms or a -CH₂-O-Ph-O-CH₂- group,
- a -CO-CO- group,
- a group of formula (A) or (B) below: 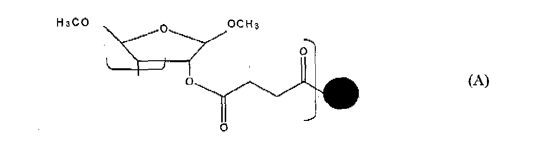 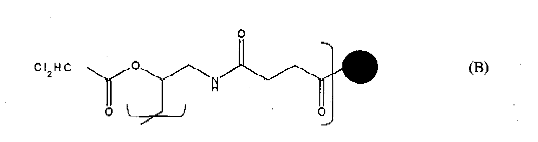
* ● represents a solid support, in particular chosen from CPG (Controlled Pore Glass) with long chain aminoalkyl (1caa) of different porosities and the resins derived from polystyrene,
followed by a reaction with a molecule M-GP, in which M represents a fluorescent probe, a peptide, an oligonucleotide or an intercalating agent, and GP represents a leaving group, in particular chosen from chlorine, bromine, iodine, the tosylate, mesylate groups, when Ra is a sulphur atom,
or with a molecule M-OH or M-NH₂ after activation of the 5'-phosphate when Ra is an oxygen atom.
and by a reaction in basic medium, in particular with ammonium hydroxide, sodium hydroxide, methylamine, potassium carbonate, or a mixture of these bases,
said reactions with a molecule M-GP, M-OH or M-NH₂ and in basic medium being able to be carried out successively or in a single "one-pot" stage,
in order to obtain said compound of formula (XX).

20. Process according to any one of claims 12 to 19, in which the oxidant is IBX.

## Patentansprüche

1. Verwendung mindestens eines Oxidationsmittels, das insbesondere aus den folgenden ausgewählt ist:
- den Derivaten von hypervalentem Iod, insbesondere aus 2-Iodoxybenzoesäure (IBX) und Dess-Martin-Periodinan (DPM), und
- dem Swern-Reagenz,
mit einem modifizierten oder unmodifizierten Oligonukleotid, das ein 5'-OH-Ende aufweist, zur Synthese von modifizierten oder unmodifizierten Oligonukleotiden, insbesondere Oligoribonukleotiden, Oligodesoxyribonukleotiden, wobei die Oligonukleotide ein 5'-Phosphatmonoester- oder 5'-Thiophosphatmonoester-Ende aufweisen.

2. Verwendung nach Anspruch 1, eines modifizierten oder unmodifizierten Oligenukleotids,
- das ein 5'-OH-Ende aufweist, und,
- (n+1) Nukleotide enthält,
um ein modifiziertes beziehungsweise unmodifiziertes Oligonukleotid zu erhalten,
- das ein 5'-Phosphatmonoester- oder 5'-Thiophosphatmonoester-Ende aufweist, und
- n Nukleotide enthält.

3. Verwendung nach einem beliebigen der Ansprüche 1 oder 2, wobei es sich bei der Synthese um eine Synthese auf einem Träger handelt.

4. Verwendung nach einem beliebigen der Ansprüche 1 bis 3, zur Synthese einer Verbindung nach der folgende Formel I): 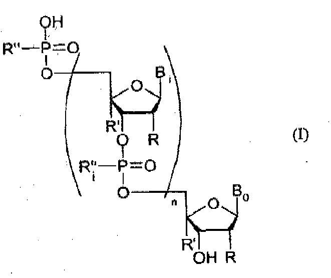 wobei:
* n eine ganze Zahl im Bereich von 1 bis 100, insbesondere von 1 bis 50, und besonders von 5 bis 40 ist,
* eine ganze Zahl von im Bereich von 1 bis n ist,
* B₀ und Bᵢ unabhängig voneinander für eine stickstoffhaltige Base stehen, insbesondere für:
- ein Purinderivat, insbesondere Adenin, Guanin, 8-Hydroxyguanin, 8-Hydroxyadenin, 8-Aminoguanin, 8-Methoxyguanin, 8-Bromguanin, 8-Bromadenin, Inosin, oder.
- ein Pyrimidinderivat, insbesondere Thymin, Cytosin, Uracil oder 5-Methylcytosin, 5-propinyluracil, Pseudouraci, 4-Thiothymin, 2-Thiothymin, 4-Thiouracil, 5-Halogenuracil, 5-Halogencytosin, 5,6-Dihydrothymin, 5-Hydroxyuracil, 5-Hydroxycytosin, 5,6-Dihydrouracil, 5-Hydroxymethyluracil, 5,6-Dihydroxythymin,
* R für folgendes steht:
- Wasserstoff, oder
- eine Hydroxylgruppe, oder
- eine -O-Alkylgruppe, die 1 bis 4 geradkettige oder verzweigte Kohlenstoffatome aufweist, oder
- eine -O-CH₂)ₘ-NH₂-Gruppe, wobei m für eine ganze Zahl im Bereich von 2 bis 10 steht, oder
- ein Sauerstoffatom,
* R' für folgendes steht:
- Wasserstoff, oder
- eine -(CH₂)ₚ-Gruppe, wobei p einen Wert von 1 bis 4 annehmen kann,
derart, dass R' für eine -(CH₂)ₚ-Gruppe steht, wenn R für ein Sauerstoffatom steht, und R für ein Sauerstoffatom steht, wenn R' für eine -(CH₂)ₚ-Gruppe steht, wobei die Gruppen R und R' verbrückt sind und somit einen ringförmigen Ether bilden, und
* R" und Rᵢ" unabhängig voneinander für eine Hydroxy1(OH)- oder Mercapto(SH)-Gruppe stehen.

5. Verwendung nach Anspruch 4, zur Synthese einer Verbindung nach der folgenden Formel (II): 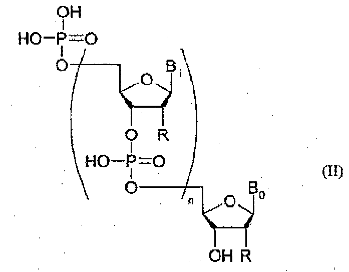 wobei:
* n eine ganze Zahl im Bereich von 1 bis 100, insbesondere von 1 bis 50, und besonders von 5 bis 40 ist,
* i eine ganze Zehl von im Bereich von 1 bis n ist,
* B₀ und Bᵢ unabhängig voneinander für Adenin, Guanin, Thymin, Cytosin oder Uracil stehen,
* R für Wasserstoff oder eine Hydroxylgruppe steht.

6. Verwendung nach einem beliebigen der Ansprüche 4 und 5, zur Synthese einer Verbindung nach der folgenden Formel (III): 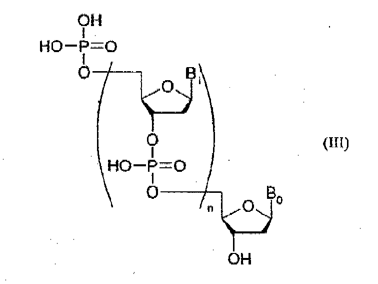 wobei:
* n eine ganze Zahl im Bereich von 1 bis 100, insbesondere von 1 bis 5C, und besonders von 5 bis 40 ist,
* i eine ganze Zahl von im Bereich von 1 bis n ist,
* B₀ und Bᵢ unabhängig voneinander für Adenin, Guanin, Thymin oder Cytosin stehen.

7. Verwendung nach einem beliebigen der Ansprüche 4 bis 5, zur Synthese einer Verbindung nach der folgenden Formel (IV): 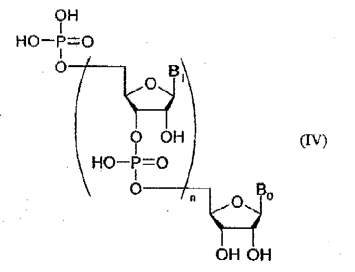 wobei:
* n eine ganze Zahl im Bereich von 1 bis 100, insbesondere von 1 bis 50, und besonders von 5 bis 40 ist,
* i eine ganze Zahl von im Bereich von 1 bis n ist,
* B₀ und Bᵢ unabhängig voneinander für Adenin, Guanin, Cytosin oder Uracil stehen.

8. Verwendung nach Anspruch 4, zur Synthese einer Verbindung nach der folgenden Formel (V'): 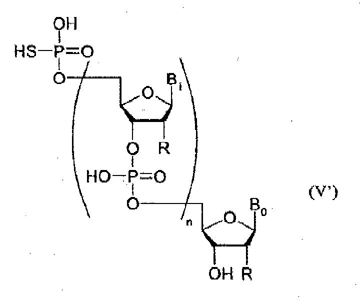 wobei:
* n eine ganze Zahl im Bereich von 1 bis 100, insbesondere von 1 bis 50, und besonders von 5 bis 40 ist,
* i eine ganze Zahl von im Bereich von 1 bis n ist,
* B₀ und Bᵢ unabhängig voneinander für Adenin, Guanin, Cytosin oder Uracil stehen.
* R für Wasserstoff oder eine Hydroxylgruppe steht.

9. Verwendung nach Anspruch 4, zur Synthese einer Verbindung nach der folgenden Formel (V): 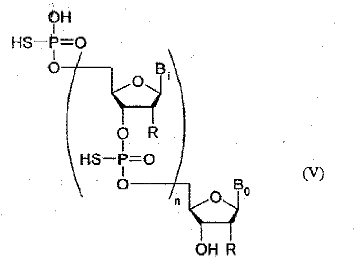 wobei:
n eine ganze Zahl im Bereich von 1 bis 100, insbesondere von 1 bis 50, und besonders von 5 bis 40 ist,
* i eine ganze Zahl von im Bereich von 1 bis n ist,
* B₀ und Bᵢ unabhängig voreinander für eine stickstoffhaltige Base stehen, insbesondere für:
- ein Purinderivat, insbesondere Adenin, Guanin, 8-Hydroxyguanin, Incsin, oder,
- ein Pyrimidinderivat, insbesondere Thymin, Cytosin, Uracil oder 5-Methylcytosin, 5-Propinyluracil, Pseudouracil, 4-Thiothymin, 2-Thiothymin, 4-Thiouracil, 5-Halogenuracil, 5-Halogencytosin, 5,6-Dihydrothymin, 5-Hydroxyuracil, 5-Hydroxycytosin, 5,6-Dihydrouracil, 5-Hydroxymethyluracil, 5,6-Dihydroxythymin,
* R für Wasserstoff oder eine Hydroxylgruppe steht.

10. Verwendung nach Anspruch 4, zur Synthese einer Verbindung nach der folgenden Formel (VI): 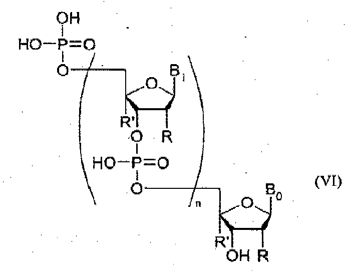 wobei:
* n eine ganze Zahl im Bereich von 1 bis 100, insbesondere von 1 bis 5C, und besonders von 5 bis 40 ist,
* i eine ganze Zahl von im Bereich von 1 bis n ist,
* B₀ und Bᵢ unabhängig voneinander für eine stickstoffhaltige Base stehen, insbesondere für:
- ein Purinderivat, insbesondere Adenin, Guanin, 8-Hydroxyguanin, Inosin, oder,
- ein Pyrimidinderivat, insbesondere Thymin, Cytosin, Uracil oder 5-Methylcytosin, 5-Propinyluraril, Pseudouracil, 4-Thiothymin, 2-Thiothymin, 4-Thiouracil, 5-Halogenuracil, 5-Halogencytosin, 5,6-Dihydrothymin, 5-Hydroxyuracil, 5-Hydroxycytosin, 5,6-Dihydrouracil, 5-Hydroxymethyluracil, 5,6-Dihydroxythymin,
* R für folgendes steht:
- eine -O-Alkylgruppe, die 1 bis 4 geradkettige oder verzweigte Kohlenstoffatome aufweist, oder
- eine -O-(CH₂)ₘ-NH₂-Gruppe, wobei m für eine ganze Zahl im Bereich von 2 bis 10 steht, oder
- ein Sauerstoffatom,
* R' für folgendes steht:
- Wasserstoff, oder
- eine -(CH₂)ₚ-Gruppe, wobei p einen Wert von 1 bis 4 annehmen kann,
derart, dass R' für eine -(CH₂)ₚ-Gruppe steht, wenn R für ein Sauerstoffatom steht, und R für ein Sauerstoffatom steht, wenn R' für eine (CH₂)ₚ-Gruppe steht, wobei die Gruppen R und R' verbrückt sind und somit einen ringförmigen Ether bilden.

11. Verwendung nach einem beliebigen der Ansprüche bis 10, wobei es sich bei dem Oxidationsmittel um IBX handelt.

12. Verfahren zur Herstellung einer Verbindung gemäß der Begriffsbestimmung in Anspruch 1, nach der folgenden Formel (I): 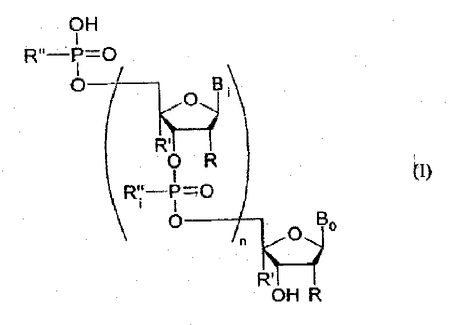 wobei:
* n eine ganze Zahl im Bereich von 1 bis 100, insbesondere von 1 bis 50, und besonders von 5 bis 40 ist,
* i eine ganze Zahl von im Bereich von 1 bis n ist,
* B₀ und Bᵢ unabhängig voneinander für eine stickstoffhaltige Base stehen, insbesondere für:
- ein Purinderivat, insbesondere Adenin, Guanin, 8-Hydroxyguanin, Inosin, oder,
- ein Pyrimidinderivat, insbesondere Thymin, Cytosin, Uracil oder 5-Methylcytosin, 5-Propinyluracil, Pseudouracil, 4-Thiothymin, 2-Thiothymin, 4-Thiouracil, 5-Halogenuracil, 5-Halogencytosin, 5,6-Dihydrothymin, 5-Hydroxyuracil, 5-Hydroxycytosin, 5,6-Dihydrouracil, 5-Hydroxymethyluracil, 5,6-Dihydroxythymin,
* R für folgendes steht:
- Wasserstoff, oder
- eine Hydroxylgruppe, oder
- eine -O-Alkylgruppe, die 1 bis 4 geradkettige oder verzweigte Kohlenstoffatome aufweist, oder
- eine -C-(CH₂)ₘ-NH₂-Gruppe, wobei m für eine ganze Zahl im Bereich von 2 bis 10 steht, oder
- ein Sauerstoffatom,
* R' für folgendes steht:
- Wasserstoff, oder
- eine -(CH₂)ₚ-Gruppe, wobei p einen Wert von 1 bis 4 annehmen kann,
derart, dass R' für eine -(CH₂)ₚ-Gruppe steht, wenn R für ein Sauerstoffatom steht, und R für ein sauerstoffatom steht, wenn R' für eine -(CH₂)ₚ-Gruppe steht, wobei die Gruppen R und R' verbrückt sind und somit einen ringförmigen Ether bilden, und
* R" und Rᵢ" unabhängig voneinander für eine Hydroxyl- oder Thiolgruppe stehen,
den folgenden Schritt umfassend:
Reaktion eines Oxidationsmittels, das aus den Derivaten von hypervalentem Iod, insbesondere aus 2-Iodoxybenzoesäure (IBX) und Dess-Martin-Periodinan (DPM), und aus dem Swern-Reagenz ausgewählt ist, mit einer Verbindung nach der folgenden Formel (XI): 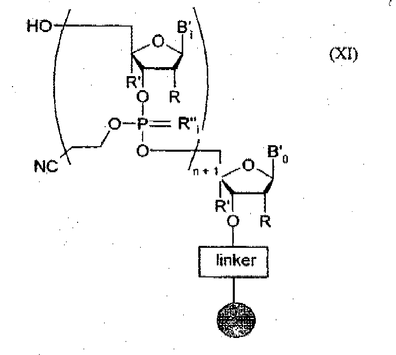 wobei:
* n eine ganze Zahl im Bereich von 1 bis 100, insbesondere von 1 bis 50, und besonders von 5 bis 40 ist,
* i eine ganze Zahl von im Bereich von 1 bis n+1 ist,
* B'₀ und B'ᵢ unabhängig voneinander für eine stickstoffhaltige Base stehen, insbesondere für:
- ein Purinderivat, insbesondere Adenin, Guanin, 8-Hydroxyguanin, Inosin, oder,
- ein Pyrimidinderivat, insbesondere Thymin, Cytosin, Uracil oder 5-Methylcytosin, 5-Propinyluracil, Pseudouracil, 4-Thiothymin, 2-Thiothymin, 4-Thiouracil, 5-Halogenuracil, 5-Halogencytosin, 5,6-Dihydrothymin, 5-Hydroxyuracil, 5-Hydroxycytosin, 5,6-Dihydrouracil, 5-Hydroxymethyluracil, 5,6-Dihydroxythymin,
wobei die stickstoffhaltige Base mit mindestens einer Schutzgruppe versehen ist, die insbesondere aus den Gruppen Benzoyl (Bz), Acetyl (Ac), Isobutyryl (iBu), Dimethylformamidin (dmf), Phenoxyacetyl (Pac), 4-Isopropylphenoxyacetyl (iPr-Pac) ausgewählt ist,
* R für folgendes steht:
- Wasserstoff, oder
- eine Hydroxylgruppe, die mit einer Schutzgruppe versehen ist, die insbesondere aus den Gruppen Acetyl (Ac), Silyl wie etwa tert.-Butyldimethylsilyl (TBDMS), Triisopropylsilylmethyl (TOM) ausgewählt ist,
- eine -O-Alkylgruppe, die 1 bis 4 geradkettige oder verzweigte Kohlenstoffatome aufweist, oder
- eine -O-(CH₂)ₘ-NH₂-Gruppe, wobei m für eine ganze Zahl im Bereich von 2 bis 10 sieht, und wobei die Aminfunktion mit einer Schutzgruppe versehen ist, die insbesondere aus den Carbamaten wie etwa der Fluorenylmethylcarbonylgruppe (Fmoc), den Amiden wie etwa Trifluoracetamid (CO-CF3) und Phthalimid ausgewählt ist,
oder
- ein Sauerstoffatom,
* R' für folgendes steht:
- Wasserstoff, oder,
- eine (CH₂)ₚ-Gruppe, wobei p einen Wert von 1 bis 4 annehmen kann,
außer im Falle der letzten Base, bei welcher R' für Wasserstoff steht.
derart, dass R' für eine -(CH₂)ₚ-Gruppe steht, wenn R für ein Sauerstoffatom steht, und R für ein Sauerstoffatom steht, wenn R' für eine -(CH₂)ₚ-Gruppe steht, wobei die Gruppen R und R' verbrückt sind und somit einen ringförmigen Ether bildern, und
* Rᵢ" urabhängig davon für ein Sauerstoff-oder Schwefelatom stehen,
* linker für ein Bindeglied steht, das insbesondere für ein Bindeglied steht, das insbesondere aus den folgenden ausgewählt ist:
- einer -CO-Z-CO-Gruppe, wobei Z für eine Alkylgruppe mit 1 bis 2 Kohlenetoffatomen steht, oder einer -CH₂-O-Ph-O-CH₂-Gruppe,
- einer -CO-CO-Gruppe,
- einer Gruppe nach den folgenden Formeln (A) oder (B): 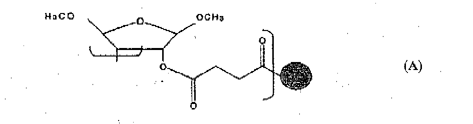 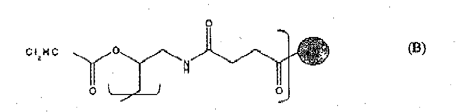
* wobei ● für einen feststofflichen Träger steht, der insbesondere aus CPG (Controlled Pore Glass) mit langer Aminoalkylkette (Icca) und verschiedenartigen Porenbescharfenheiten sowie aus Harzen, die sich von Polystyrol ableiten, ausgewählt ist,
woraufhin eine Reaktion in basischem Milieu erfolgt, insbesondere mit Ammoniumhydroxid, Natriumhydroxid, Methylamin, Kaliumcarbonat oder einer Mischung dieser Basen,
um die Verbindung nach der Formel (I) zu erhalten.

13. Herstellungsverfahren nach Anspruch 12, für eine Verbindung nach der folgenden Formel (II): 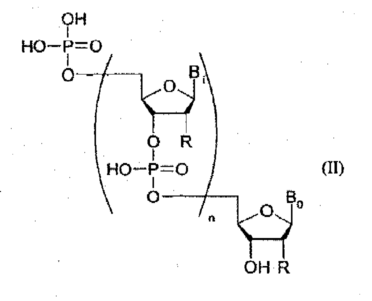 wobei:
* n eine ganze Zahl im Bereich von 1 bis 100, insbesondere von 1 bis 50, und besonders von 5 bis 40 ist,
* i eine ganze Zahl von im Bereich von 1 bis n ist,
* B₀ und Bᵢ unabhängig voneinander für Adenin, Guanin, Thymin, Cytosin oder Uracil stehen,
* R für Wasserstoff oder eine Hydroxylgruppe steht, den folgenden Schritt umfassend:
Reaktion eines Oxidationsmittels, das aus den Derivaten von hypervalentem Iod, insbesondere aus 2-Iodoxybenzoesäure (IBX) und Dess-Martin-Periodinan (DPM), und aus dem Swern-Reagenz ausgewählt ist,
mit einer Verbindung nach der folgenden Formel (XII): 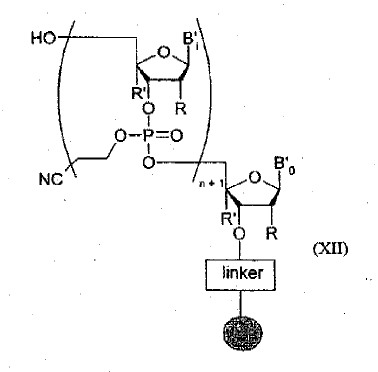 wobei:
* n eine ganze Zahl im Bereich von 1 bis 100, insbesondere von 1 bis 50, und besonders von 5 bis 40 ist,
* i eine ganze Zahl von im Bereich von 1 bis n+1 ist,
* B'₀ und B'ᵢ unabhängig voneinander für eine stickstoffhaltige Base stehen, die aus Adenin, Guanin, Thymin, Cytosin und Uracil ausgewählt ist, wobei die stickstoffhaltige Base mit mindestens einer Schutzgruppe versehen ist, welche insbesondere aus den Gruppen Benzoyl (Bz), Acetyl (Ac), Isobutyryl (iBu), Dimethylformamidin (dmf), Phenoxyacetyl (Pac), 4-Isopropylphenoxyacetyl (iPr-Pac) ausgewählt ist,
* R für Wasserstoff oder eine Hydroxylgruppe steht,
* R' für wasserstoff steht linker
* für ein Bindeglied steht, das insbesondere aus den folgenden ausgewählt ist:
- einer -CO-Z-CO-Gruppe, wobei Z für eine Alkylgruppe mit 1 bis 2 Kohlenstoffatomen steht, oder einer -CH₂-O-Ph-O-CH₂-Gruppe,
- einer -CO-CO-Gruppe,
- einer Gruppen nach den folgenden Formeln (A) oder (B): 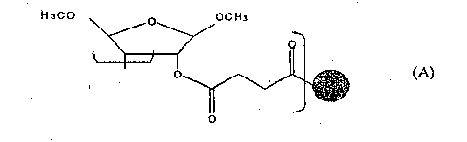 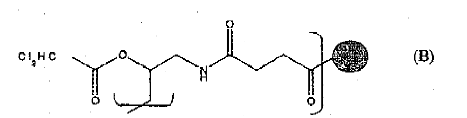
* wobei ● für einen feststofflichen Träger steht, der insbesondere aus CPG (Controlled Pore Class) mit langer Aminoalkylkette (lcca) und verschiedenartiger Porenbeschaffenheiten sowie aus Harzen, die sich von Polystyrol ableiten, ausgewählt ist,
woraufhin eine Reaktion in basischem Milieu erfolgt, insbesondere mit Ammoniumhydroxid, Natriumhydroxid, Methylamin, Kaliumcarbonat oder einer Mischung dieser Basen,
um die Verbindung nach der Formel (II) zu erhalten.

14. Herstellungsverfahren nach einem beliebigen der Ansprüche 12 und 13, für eine Verbindung nach der folgenden Formel (III): 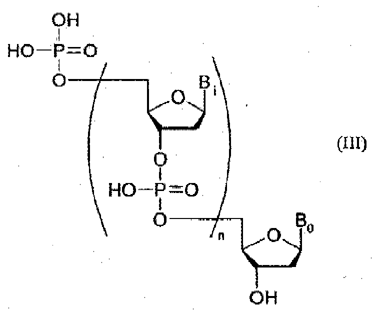 wobei:
* n eine ganze Zahl im Bereich von 1 bis 100, insbesondere von 1, bis 50, und besonders von 5 bis 40 ist,
* i eine ganze Zahl von im Bereich von 1 bis n ist,
* B₀ und Bᵢ unabhängig voneinander für Adenin, Guanin, Thymin oder Cytosin stehen,
den folgenden Schritt umfassend:
Reaktion eines Oxidationsmittels, das aus den Derivaten von hypervalentem Iod, insbesondere aus 2-Iodoxybenzoesäure (IBX) und Dess-Martin-Periodinan (DPM), und aus dem Swern-Reagenz ausgewählt ist,
mit einer Verbindung nach der folgenden Formel (XIII): 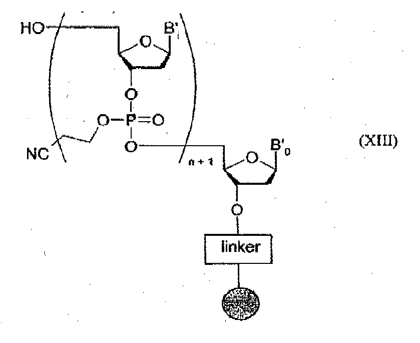 wobei:
* n eine ganze Zahl im Bereich von 1 bis 100, insbesondere von 1 bis 50, und besonders von 5 bis 40 ist,
* i eine ganze Zahl von im Bereich von 1 bis n+1 ist,
* B'₀ und B'ᵢ unabhängig voneinander für eine stickstoffhaltige Base stehen, die aus Adenin, Guanin, Thymin und Cytosin ausgewählt ist,
wobei die stickstoffhaltige Base mit mindestens einer Schutzgruppe versehen ist, die insbesondere aus den Gruppen Benzoyl (Bz), Acetyl (Ac), Isobutyryl (iBu), Dimethylformamidin (dmf), Phencxyacetyl (Pac), 4-Isopropylphenoxyacetyl (iPr-Pac) ausgewählt ist,
* linker für ein Bindeglied steht, das insbesondere aus den folgenden ausgewählt ist:
- einer -CO-Z-CO-Gruppe, wobei Z für eine Alkylgruppe mit 1 bis 2 Kohlenstoffatomen steht, oder einer -CH₂-O-Ph-O-CH₂-Gruppe,
- einer -CO-CO-Gruppe,
- einer Gruppe nach den folgenden Formeln (A) oder (B): 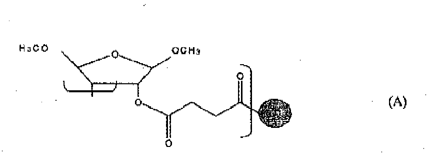 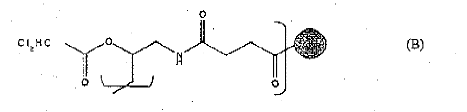
* wobei ● für einen feststofflichen Träger steht, der insbesondere aus CPG (Controlled Pore Glass) mit langer Aminoalkylkette (Icca) und verschiedenartigen Porenbeschaffenheiten sowie aus Harzen, die sich von Polystyrol ableiten, ausgewählt ist,
woraufhin eine Reaktion in basischem Milieu erfolgt, insbesondere mit Ammoniumhydroxid, Natriumhydroxid, Methylamin, Kaliumcarbonat oder einer Mischung dieser Basen,
um die Verbindung nach der Formel (III) zu erhalten.

15. Herstellungsverfahren nach einem beliebigen der Ansprüche 12 und 13, für eine Verbindung nach der folgenden Formel (IV): 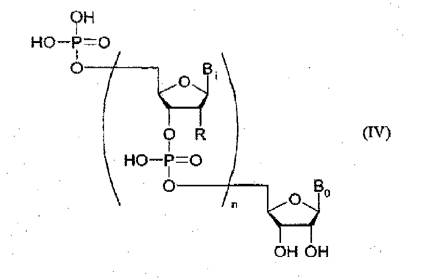 wobei:
* n eine ganze zahl im Bereich von 1 bis 100, insbesondere von 1 bis 50, und besonders von 5 bis 40 ist,
* i eine ganze Zahl von im Bereich von 1 bis n ist,
* B₀ une Bᵢ unabhängig voneinander für Adenin, Guanin, Cytosin oder Uracil stehen,
den folgenden Schritt umfassend:
Reaktion eines Oxidationsmittels, das aus den Derivaten von hypervalentem Iod, insbesondere aus 2-Iodoxybenzoesäure (IBX) und Dess-Martin-Periodinan (DPM), und aus dem Swern-Reagenz ausgewählt ist,
mit einer Verbindung nach der folgenden Formel (XIV): 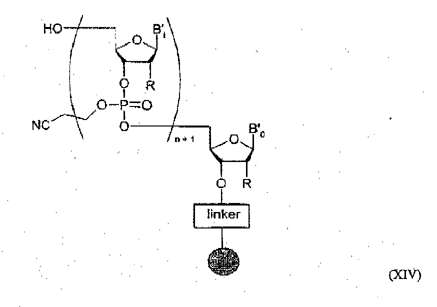
wobei:
* n eine ganze Zahl im Bereich von 1 bis 10C, insbesondere von 1 bis 50, und besonders von 5 bis 40 ist,
* i eine ganze Zahl von im Bereich von 1 bis n+1 ist,
* B'₀ und B'₁ unabhängig voneinander für eine stickstoffhaltige Base stehen, die aus Adenin, Guanin, Cytosin und Uracil ausgewählt ist,
wobei die stickstoffhaltige Base mit mindestens einer Schutzgruppe versehen ist, die insbesondere aus den Gruppen Benzoyl (Bz), Acetyl (Ac), Isobutyryl (iBu), Dimethylformamidin (dmf), Phenoxyacetyl (Pac), 4-Isopropylphenoxyacetyl (iPr-Pac) ausgewählt ist,
* **linker** für ein Bindeglied steht, das
insbesondere aus den folgenden ausgewählt ist:
- einer -CO-Z-CC-Gruppe, wobei Z für eine Alkylgruppe mit 1 bis 2 Kohlenstoffatomen steht, oder einer -cH₂-O-Ph-O-CH₂-Gruppe,
- einer -CO-CO-Gruppe,
- einer Gruppe nach den folgenden formeln (A) oder (B): 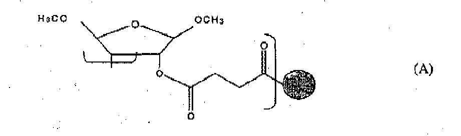 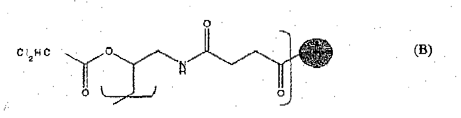
* wobei 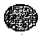 für einen feststofflichen Träger steht, der insbesondere aus CPG (Controlled Pore Glass) mit langer Aminoalkylkette (Icca) und verschiedenartigen Porenbeschaffenheiten sowie aus Harzen, die sich von Polystyrol ableiten, ausgewählt ist,
woraufhin eine Reaktion in basischem Milieu erfolgt, insbesondere mit Ammoniumhydroxid, Natriumhydroxid, Methylamin, Kaliumcarbonat oder einer Mischung dieser Basen,
um die Verbindung nach der Formel (IV) zu erhalten.

16. Herstellungsverfahren nach Anspruch 12, für eine Verbindung nach der folgenden Formel (V'): 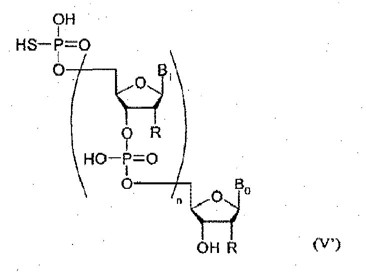 wobei:
* n eine ganze Zahl im Bereich von 1 bis 100, insbesondere von 1 bis 50, und besonders von 5 bis 40 ist,
* i eine ganze Zahl von im Bereich von 1 bis n ist,
* B₀ und Bᵢ unabhängig voneinander für Adenin, Guanin, Thymin, Cytosin oder Uracil stehen,
* R für Kassarstoff oder eine Hydroxylgruppe steht, den folgenden Schritt umfassend:
Reaktion eines Oxidationsmittels, das aus den Derivaten von hypervalentem Iod, insbesondere aus 2-Iodoxybenzoesäure (IBX) und Dess-Martin-Periodinan (DPM), und aus dem Swern-Reagenz ausgewählt ist,
mit einer Verbindung nach der folgenden Formel (XXI): 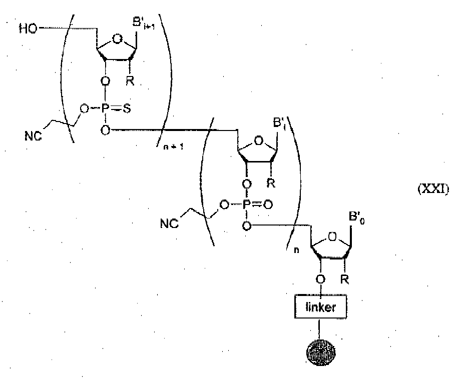
* n eine ganze Zahl im Bereich von 1 bis 100, insbesondere von 1 bis 50, und besonders von 5 bis 40 ist,
* i eine ganze Zahl von im Bereich von 1 bis n-1 ist,
* B'₀, B'ᵢ und B'ᵢ₊₁ unabhängig voneinander für eine stickstoffhaltige Base stehen, die aus Adenin, Guanin, Cytosin und Uracil ausgewählt ist,
wobei die stickstoffhaltige Base mit mindestens einer Schutzgruppe versehen ist, die insbesondere aus den Gruppen Benzoyl (Bz), Acetyl (Ac), Isobutyryl (iBu), Dimethylformamidin (dmf), Phenoxyacetyl (Pac), 4-Isopropylphenoxyacetyl (iPr-Pac) auscewählt ist,
* R für eine Hydroxylgruppe steht, die mit einer Schutzgruppe versehen ist, die insbesondere aus den Gruppen Acetyl (Ac), Silyl wie etwa tert.-Butyldimethylsilyl (TBDMS) und Triisopropylsilylmethyl (TOM) ausgewählt ist.
* linker für ein Bindeglied steht, das insbesondere aus den folgenden ausgewählt ist:
- einer -CC-Z-CC-Gruppe, wobei Z für eine Alkylgruppe mit 1 bis 2 Kohlenstoffatomen steht, oder einer -CH₂-O-Ph-O-CH₂-Gruppe,
- einer -CO-CO-Gruppe,
- einer Gruppe nach den folgenden Formeln (A) oder (B): 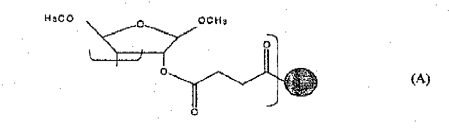 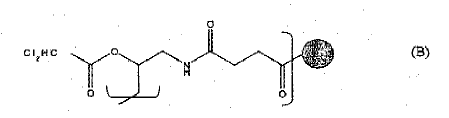
* wobei 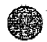 für einen feststoftlichen Träger steht, der insbesondere aus CPG (Controlled Pore Glass) mit langer Aminoalkylkette (Icca) und verschiedenartigen Porenbeschaffenheiten sewie aus Harzen, die sich von Polystyrol ableiten, ausgewählt ist,
woraufhin eine Reaktion in basischem Milieu erfolgt, insbesondere mit Ammoniumhydroxid, Natriumhydroxid, Methylamin, Kaliumcarbonat oder einer Mischung dieser Basen,
um die Verbindung nach der Formel (V') zu erhalten.

17. Herstellungsverfahren nach Anspruch 12, für eine Verbindung nach der folgenden Formel (V): 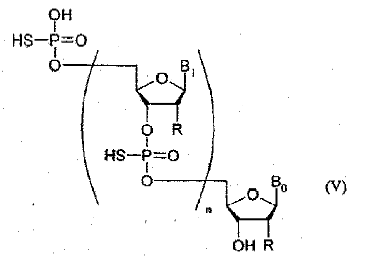 wobei:
* n eine ganze Zahl im Bereich von 1 bis 100, insbesondere von 1 bis 50, und besonders von 5 bis 40 ist,
* i'eine ganze Zahl von im Bereich von 1 bis n ist,
* B₀ und Bᵢ unabhängig voneinarder für eine stickstoffhaltige Base stehen, insbesondere für:
- ein Purinderivat, insbesondere Adenin, Guanin, 8-Hydroxyguanin, Inosin, oder,
- ein Pyrimidinderivat, insbesondere Thymin, Cytosin, Uracil oder 5-Methylcytosin, 5-Propiryluracil, Pseudouracil, 4-Thiothymin, 2-Thiothymin, 4-Thiouracil, 5-Halogenuracil, 5-Halogancytosin, 5,6-Dihydrothymin, 5-Hydroxyuracil, 5-Hydroxycytosin, 5,6-Dihydrouracil, 5-Hydroxymethyluracil, 5,6-Dihydroxythymin,
* R für Wassersteff oder eine Hydroxylgruppe steht, den folgenden Schritt umfassend:
Reaktion eines Oxidationsmittels, das aus den Derivaten von hypervalentem Iod, insbesondere aus 2-Iodoxybenzoesäure (IBX) und Dess-Martin-Periodinan (DPM), und aus dem Swern-Reagenz ausgewählt ist,
mit einer Verbindung nach der folgenden Formel (XV): 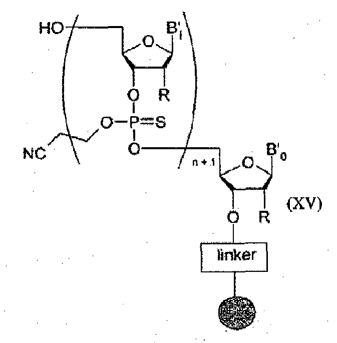 wobei:
* n eine ganze Zahl im Bereich von 1 bis 100, insbesondere von 1 bis 50, und besonders von 5 bis 40 ist,
* i eine ganze Zahl von im Bereich von 1 bis n+1 ist,
* B'₀ und B'ᵢ unabhängig voneinander für eine stickstoffhaltige Base stehen, insbesondere für:
- ein Purinderivat, insbesondere Adenin, Guanin, 3-Hydroxyguanin, Inosin, oder,
- ein Pyrimidinderivat, insbesondere Thymin, Cytosin, Uracil oder 5-Methylcytosin, 5-Propinyluracil, Pseudouracil, 4-Thiothymin, 2-Thiothymin, 4-Thiouracil, 5-Halogeruracil, 5-Halogencytosin, 5,6-Dihydrothymin, 5-Hydroxyuracil, 5-Hydroxycytosin, 5,6-Dihydrouracil, 5-Hydroxymethyluracil, 5,6-Dihydroxythymin,
wobei die stickstoffhaltige Rase mit mindestens einer Schutzgruppe versehen ist, die insbesondere aus den Gruppen Benzoyl (Bz), Acetyl (Ac), Isobutyryl (iBu), Dimethylformamidin (dmf), Phenoxyacetyl (Pac), 4-Isopropylphenoxyacetyl (iPr-Fac) ausgewählt ist,
* R für Wasserstoff oder eine Hydroxylgruppe steht,
* wobei **linker** insbesondere aus Folgendem ausgewählt ist:
- einer -CO-Z-CO-Gruppe, wobei Z für eine Alkylgruppe mit 1 bis 2 Kohlenstoffatomen steht, oder einer -CH₂-O-Ph-O-CH₂-Gruppe,
- einer -CO-CO-Gruppe,
- einer Gruppe nach den folgenden Formeln (A) oder (B): 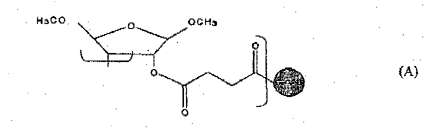 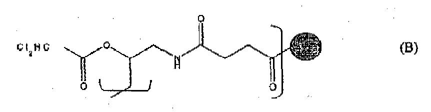
* wobei 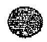 für einen feststofflichen Träger steht, der insbesondere aus CPG (Controlled Pore Glass) mit langer Aminoalkylkette (lcca) und verschiedenartigen Porenbeschaffenheiten sowie aus Harzen, die sich von Polystyrol ableiten, ausgewählt ist,
woraufhin eine Reaktion in basischem Milieu erfolgt, insbesondere mit Ammoniumhydroxid, Natriumhydroxid, Methylamin, Kaliumcarbonat oder einer Mischung dieser Basen,
um die Verbindung nach der Formel (V) zu erhalten.

18. Herstellungsverfahren nach Anspruch 12, für eine Verbindung nach der folgenden Formel (VI): 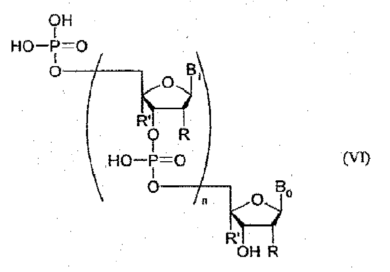 wobei:
* n eine ganze Zahl im Bereich von 1 bis 100, insbesondere vcn 1 bis 50, und besonders von 5 bis 40 ist,
* i eine ganze Zahl von im Bereich von 1 bis n ist,
* B₀ und Bᵢ unabhängig voneinander für eine stickstoffhaltige Base stehen, insbesondere für:
- ein Purinderivat, insbesondere Adenin, Guanin, 8-Hydroxyguanin, Inosin, oder,
- ein Pyrimidinderivat, insbesondere Thymin, Cytosin, Uracil oder 5-Methylcytosin, 5-Prepinyluracil, Pseudouracil, 4-Thiothymin, 2-Thiothymin, 4-Thiouracil, 5-Halogenuracil, 5-Halogencytosin, 5,6-Dihydrothymin, 5-Hydroxyuracil, 5-Hydroxycytosin, 5,6-Dyhydrouracil, 5-Hydroxymethyluracil, 5,6-Dihydroxythymin,
* R für folgendes steht:
- eine -O-Alkylgruppe, die 1 bis 4 geradkettige oder verzweigte Kohlenstoffatome aufweist, oder
- eine -O-(CH₂)ₘ-NH₂-Gruppe, wobei m für eine ganze Zahl im Bereich von 2 bis 10 steht, oder,
- ein Sauerstoffatom,
* R' für folgendes steht:
- Wasserstoff, oder
- eine -(CH₂)ₚ-Gruppe, wobei p einen Wert von 1
bis 4 annehmen kann,
derart, dass R' für eine -(CH₂)ₚ-Gruppe steht, wenn R für ein Sauerstoffatom steht, und R für ein Sauerstoffatom steht, wenn R' für eine -(CH₂)ₚ-Gruppe steht, wobei die Gruppen R und R' verbrückt sind und somit einen ringförmigen Ether bilden, und,
den folgenden Schritt umfassend:
Reaktion eines Oxidationsmittels, das aus den Derivaten von hypervalentem Iod, insbesondere aus 2-Iodoxybenzoesäure (IBX) und Dess-Martin-Periodinan (DPM), und aus dem Swern-Reagenz ausgewählt ist,
mit einer Verbindung nach der folgenden Formel (XVI): 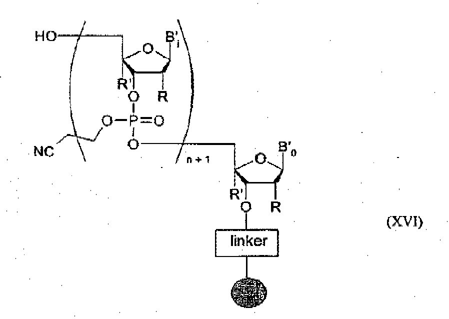
wobei:
* n eine ganze Zahl im Bereich von 1 bis 100, insbesondere von 1 bis 50, und besonders von 5 bis 40 ist,
* i eine ganze Zahl von im Bereich von 1 bis n ist,
* B'₀ und B'ᵢ unabhängig voneinander für eine stickstoffhaltige Base stehen, insbesondere für:
- ein Purinderivat, insbesondere Adenin, Guanin, 8-Hydroxyguanin, Inosin, oder,
- ein Pyrimidinderivat, insbesondere Thymin, Cytosin, Uracil oder 5-Methylcytosin, 5-Propinyluracil, Pseudouracil, 4-Thiothymin, 2-Thiothymin, 4-Thiouracil, 5-Halogenuracil, 5-Halogencytosin, 5,6-Dibydrothymin, 5-Hydroxyuracil, 5-Hydroxycytosin, 5,6-Dihydrouracil, 5-Hydroxymethyluracil, 5,6-Dihydroxythymin,
wobei die stickstoffhaltige Base mit mindestens einer Schutzgruppe versehen ist, die insbesondere aus den Gruppen Benzoyl (Bz), Acetyl (Ac), Isobutyryl (iBu), Dimethylformamidin (dmf), Phenoxyacetyl (Pac), 4-Isopropylphenoxyacetyl (iPr-Pac) ausgewählt ist,
* R für folgendes steht:
- eine -O-Alkylgruppe, die 1 bis 4 geradkettige oder verzweigte Kohlenstcffatome aufweist, oder
- eine -O- (CH₂)ₘ-NH₂-Gruppe, wobei m für eine ganze Zahl im Bereich von 2 bis 10 steht, oder
- ein Sauerstoffatom,
* R' für folgendes steht:
- Wasserstoff, oder
- eine -(CH₂)ₚ-Gruppe, wobei p einen Wert von 1 bis 4 annehmen kann,
derart, dass R' für eine -(CH₂)ₚ-Gruppe steht, wenn 3 für ein Sauerstoffatom steht, und R für ein Sauerstoffatom steht, wenn R' für eine -(CH₂)ₚ-Gruppe steht, wobei die Gruppen R und R' verbrückt sind und somit einen ringförmigen Ether bilden, außer im Falle der letzten Base, bei welcher R' für Wasserstoff steht.
und
* Rⱼ" unabhängig davon für eine Hydroxyl- oder Thiolgruppe stehen,
* wobei **linker** insbesondere aus Folgendem
ausgewählt ist:
- einer -CO-Z-CO-Gruppe, wobei Z für eine Alkylgruppe mit 1 bis 2 Kohlenstoffatomen steht, oder einer -CH₂-O-Ph-O-CH₂-Gruppe,
- einer -CO-CO-Gruppe,
- einer Gruppe nach den folgenden Formeln (A) oder (B): 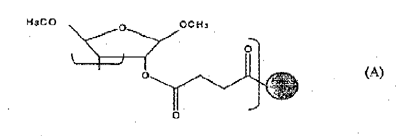 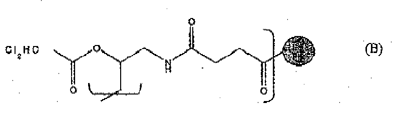
* wobei 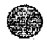 für einen feststofflichen Träger steht, der insbesondere aus CPG (Controlled Pore Glass) mit langer Amincalkylkette (leca) und verschiedenartigen Porenbeschaffenheiten sowie aus Harzen, die sich von Polystyrol ableiten, ausgewählt ist,
woraufhin eine Reaktion in basischem Milieu erfolgt, insbesondere mit Ammoniumhydroxid, Natriumhydroxid, Methylamin, Kaliumcarbonat oder einer Mischung dieser Basen,
um die Verbindung nach der Formel (VI) zu erhalten.

19. Verfahren zur Herstellung einer verbindung nach Formel (XX): 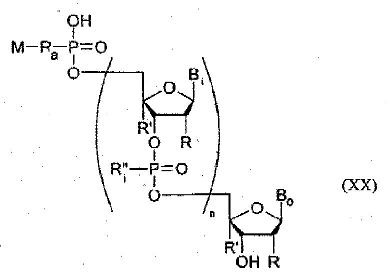 wobei:
* n eine ganze Zahl im Bereich von 1 bis 100, insbesondere von 1, bis 50, und besonders von 5 bis 40 ist,
* i eine ganze Zahl von im Bereich von 1 bis n ist,
* B_{c} und Bᵢ unabhängig voneinander für eine stickstoffhaltige Base stehen, insbesondere für:
- ein Purinderivat, insbesondere Adenin, Guanin, 8-Hydroxyguanin, Inosin, oder,
- ein Pyrimidinderivat, insbesondere Thymin, Cytosin, Uracil oder 5-Methylcytosin, 5-Propinyluracil, Pseudouracil, 4-Thiothymin, 2-Thiothyrcin, 4-Thiouracil, 5-Halogenuracil, 5-Halogencytosin, 5, 6-Eihydrothymin, 5-Hydroxyuracil, 5-Ilydroxycytosin, 5,6-Dihydrouracil, 5-Hydroxymethyluracil, 5,6-Dihydroxythymin,
* R für folgendes steht:
- Wasserstoff, oder
- eine Hydroxylgruppe, oder
- eine -O-Alkylgruppe, die 1 bis 4 geradkettige oder verzweigte Kohlenstoffatome aufweist, oder
- ein Sauerstoffatom,
* R' für folgendes steht:
- Wasserstoff, oder
- eine -(CH₂)ₚ-Gruppe, wobei p einen Wert von 1 bis 4 annehmen kann,
derart, dass R' für eine -(CH₂)ₚ-Gruppe steht, wenn R für ein Sauerstoffatom steht, und R für ein Sauerstoffatom steht, wenn R' für eine -(CH₂)ₚ-Gruppe steht, wobei die Gruppen R und R' verbrückt sind und somit einen ringförmigen Ether bilden, und,
* Rᵢ" unabhängig davon für eine Hydrexyl- oder Thiolgruppe stehen,
* Ra für ein Schwefel- oder Sauerstoffatom steht,
* M für einen Fluoreszenzmarker, ein Peptid, ein Oligonukleotid oder ein Interkalationsmittel steht, den folgenden Schritt umfassend:
Reaktion eines Oxidationsmittels, das aus den Derivaten von hypervalentem Iod, insbesondere aus 2-Iodoxybenzoesäure (IBX) und Dess-Martin-Periodinan (DPM), und aus dem Swern-Reagenz ausgewählt ist, mit einer Verbindung nach der folgenden Formel (XI): 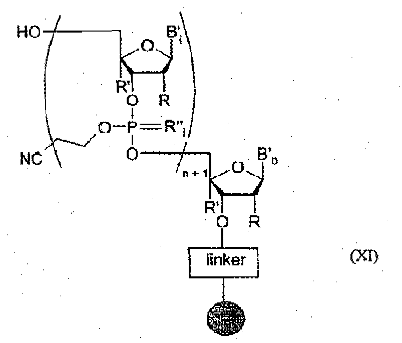 wobei:
* n eine ganze Zahl im Bereich von 1 bis 100, insbesondere von 1 bis 50, und besonders von 5 bis 40 ist,
* i eine ganze Zahl von im Bereich von 1 bis n-1 ist,
* B'_{c} und B'ᵢ unabhängig voneinander für eine stickstoffhaltige Base stechen, insbesondere für:
- ein Purinderivat, insbesondere Adenin, Guanin, 8-Hydroxyguanin, Inosin, oder,
- ein Pyrimidinderivat, insbesondere Thymin, Cytosin, Uracil oder 5-Methylcytosin, 5-Propinyluracil, Pseudouracil, 4-Thiothymin, 2-Thiothymin, 4-Thiouracil, 5-Halogenuracil, 5-Halogencytosin, 5,6-Dihydrothymin, 5-Hydroxyuracil, 5-Hydroxycytosin, 5,6-Dihydrouracil, 5-Hydroxymethyluracil, 5,6-Dihydroxythymin,
wobei die stickstoffhaltige Base mit mindestens einer Schutzgruppe versehen ist, die insbesondere aus den Gruppen Benzoyl (Bz), Acetyl (Ac), Isobutyryl (iBu), Dimethylformamidin (dmf), Phenoxyacetyl (Pac), 4-Isopropylphenoxyacetyl (iPr-Pac) ausgewählt ist,
* R für folgendes steht:
- Wasserstoff, oder
- eine Hydroxylgruppe, oder
- eine -O-Alkylgruppe, die 1 bis 4 geradkettige oder verzweigte Kohlenstoffatome aufweist, oder
- ein Sauerstoffatom,
* R' für folgendes steht:
- Wasserstoff, oder
- eine -(CH₂)ₚ-Gruppe, wobei p einen Wert von 1
bis 4 annehmen kann,
derart, dass R' für eine -(CH₂)ₚ-Gruppe steht, wenn R für ein Sauerstoffatom steht, und R für ein Sauerstoffatom steht, wenn R' für eine -(CH₂)ₚ-Gruppe steht, wobei die Gruppen R und R' verbrückt sind und somit einen ringförmigen Ether bilden, außer im Falle der letzten Base, bei welcher R' für Wasserstoff steht.
und
* Rᵢ" unabhängig davon für eine Hydroxyl- oder Thiolgruppe stehen,
* **linker** für ein Bindeglied steht, das insbesondere aus den folgenden ausgewählt ist:
- einer -CO-Z-CO-Gruppe, wobei Z für eine Alkylgruppe mit 1 bis 2 Kohlenstoffatomen steht, oder einer -CH₂-O-Ph-C-CH₂-Gruppe,
- einer -CO-CO-Gruppe,
- einer Gruppe nach den folgenden Formeln (A) oder (B): 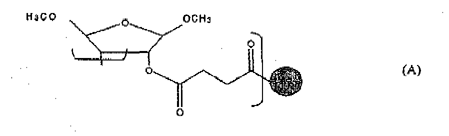 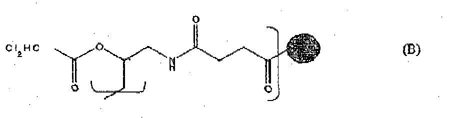
* wobei 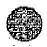 für einen feststofflichen Träger steht, der insbesondere aus CPG (Controlled Pore Glass) mit langer Aminoalkylkette (Icca) und verschiedenartigen Porenbesahaffenheiren sowie aus Harzen, die sich von Polystyrol ableiten, ausgewählt ist,
woraufhin eine Reaktion mit einem Molekül M-GP erfolgt, wobei M für einen Fluoreszenzmarker, ein Peptid, ein Oligonukleotid oder ein Interkalationsmittel steht und GP für eine Abgangsgruppe steht, die insbesondere aus Chlor, Brom, Iod, den Gruppen Tosylat, Mesylat ausgewählt ist, wenn Ra ein Schwefelatom ist,
oder mit einem Molekül M-OH oder M-NH₂ nach Aktivierung des Phosphats in 5'-Stellung, wenn Ra ein Sauerstoffatom ist.
und eine Reaktion in basischem Milieu, insbesondere mit Ammoniumhydroxid, Natriumhydroxid, Methylamin, Kaliumcarbonat oder einer Mischung dieser Basen,
wobei die Reaktionen mit einem Molekül M-GP, M-OH oder M-NH₂ sowie in basischem Milieu nacheinander oder in einem einzigen "Eintopf"-Schritt durchgeführt werden können, um die Verbindung nach der Formel (XX) zu erhalten.

20. Verfahren nach einem beliebigen der Ansprüche 12 bis 19, wobei es sich bei dem Oxidationsmittel um IBX handelt.
